(19) Europäisches Patentamt
European Patent Office
Office européen des brevets

(11) EP 4 286 416 A1

(12) **EUROPEAN PATENT APPLICATION**
published in accordance with Art. 153(4) EPC

(43) Date of publication:
**06.12.2023 Bulletin 2023/49**

(21) Application number: **22745948.4**

(22) Date of filing: **26.01.2022**

(51) International Patent Classification (IPC):
***C08B 37/16*** *(2006.01)* ***A61K 9/16*** *(2006.01)*
***A61K 38/46*** *(2006.01)* ***A61K 47/34*** *(2017.01)*
***A61K 47/40*** *(2006.01)* ***A61K 48/00*** *(2006.01)*
***A61P 43/00*** *(2006.01)* ***C12N 15/09*** *(2006.01)*
***C12Q 1/68*** *(2018.01)*

(52) Cooperative Patent Classification (CPC):
**A61K 9/16; A61K 38/46; A61K 47/34; A61K 47/40; A61K 48/00; A61P 43/00; C08B 37/0012; C12N 15/09; C12Q 1/68**

(86) International application number:
**PCT/JP2022/002968**

(87) International publication number:
**WO 2022/163729 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.01.2021 JP 2021010606**

(71) Applicant: **National University Corporation Kumamoto University**
**Kumamoto 860-8555 (JP)**

(72) Inventors:
- **HIGASHI, Taishi**
  **Kumamoto-shi, Kumamoto 860-8555 (JP)**
- **MOTOYAMA, Keiichi**
  **Kumamoto-shi, Kumamoto 860-8555 (JP)**
- **ONODERA, Risako**
  **Kumamoto-shi, Kumamoto 860-8555 (JP)**
- **TAHARABARU, Tooru**
  **Kumamoto-shi, Kumamoto 860-8555 (JP)**
- **KIHARA, Takuya**
  **Kumamoto-shi, Kumamoto 860-8555 (JP)**

(74) Representative: **Abel & Imray LLP**
**Westpoint Building**
**James Street West**
**Bath BA1 2DA (GB)**

Remarks:
The complete document including Reference Table(s) and the Sequence Listing(s) can be downloaded from the EPO website

# (54) CARRIER FOR FUNCTIONAL NUCLEIC ACID AND PROTEIN INTRODUCTION

(57) The present invention provides an aminated polyrotaxane (PRX) (amino-PRX) carrier as a nucleic acid/protein carrier. Amino-PRX freely provides an amino group to the sgRNA and acidic amino acids of Cas9. A polyplex with Cas9 RNP (automatic molecular imprinting) was efficiently and easily formed by rotating and moving CD merely by mixing amino-PRX and Cas9 RNP, resulting in efficient intracellular delivery of Cas9 RNP. Furthermore, structural optimization of the axis/end cap linker and the amino group/CD linker made it possible to realize strict control of the intracellular dynamics of Cas9 RNP and high efficiency in genome editing.

EP 4 286 416 A1

**Description**

CROSS-REFERENCE TO RELATED APPLICATION

The present international application claims priority based on Japanese Patent Application No. 2021-010606 filed with the Japanese Patent Office on January 26, 2021, and the entire contents of Japanese Patent Application No. 2021-010606 are incorporated herein by reference.

TECHNICAL FIELD

The present disclosure relates to a carrier for assisting in stable nucleic acid and protein delivery into cells.

BACKGROUND ART

In recent years, it has been sought to develop a technology for safely and highly efficiently introducing, into a cell(s), a protein and/or a nucleic acid polymer including a protein/nucleic acid complex such as Cas9 RNP used for inducing genome editing or a nucleic acid molecule such as siRNA.

CRISPR-Cas9 (clustered regularly interspaced short palindromic repeats-CRISPR associated proteins 9) is an innovative genome editing technology capable of cleaving a DNA double strand and deleting, substituting, and inserting a nucleotide(s) at a given position(s) in a genome sequence. In order to induce genome editing by CRISPR-Cas9, it is necessary to deliver a Cas9 protein and a guide RNA (nucleic acid) into a cell. Examples of the delivery method that has been primarily used include: a method of introducing a plasmid DNA encoding a Cas9 protein and a guide RNA; a method of introducing an mRNA encoding Cas9 and a guide RNA; and a method of introducing a complex of a Cas9 protein and a guide RNA (Cas9 RNP). Among them, a method of directly introducing a preassembled Cas9 RNP has attracted the most attention because of excellent genome editing efficiency, high safety, and high convenience (Non-Patent Documents 1 and 2). Since Cas9 RNP is membrane-impermeable, a carrier is required for delivery into cells. Examples of such a carrier used include a non-viral carrier such as a cationic material (lipid/liposome) (Non-Patent Documents 3 to 7), nanoparticles (Non-Patent Documents 8 to 10), or a hetero-interacting cationic polymer (Non-Patent documents 11 and 12). However, Cas9 RNP has zwitterion derived from Cas9 (protein) and a complex surface structure. This has caused a problem that the interaction with the cationic material is low. The multilayered nanoparticles or the hetero-interacting cationic polymer could interact with the basic amino acids of Cas9, but could not fit for the conformation and charge distribution of Cas9 RNP. On the other hand, it has been reported that an in situ polymerized nanocapsule (NC) can present appropriate monomers based on the surface structure of Cas9 RNP (molecular imprinting), so that more efficient loading of Cas9 RNP can be performed (Non-Patent Document 13). Since the crosslinker between the monomers of NC is biodegradable, Cas9 RNP enables efficient genome editing by controlling intracellular dynamics. However, the preparation of NC requires not only the addition of interactable monomers but also many steps (e.g., nitrogen substitution/reflux, dialysis, lyophilization). There has been a challenge that the use of NC and gene retargeting are very difficult.

Polyrotaxane (PRX) is a polymer compound having a threaded structure in which a plurality of macrocyclic molecules such as cyclodextrins (CDs) are threaded using an axial molecule such as PEG, and having bulky substituents (caps) at both ends of the axial molecule in order to prevent dissociation of the macrocyclic molecules (Non-Patent Documents 14 to 19). It has been reported that CDs of PRX are movable on the axial molecule (Non-Patent Document 20). Therefore, the modified cationic charge on CD of PRX is flexibly movable, and strongly interacts with a gene (Non-Patent Documents 21 and 22), a nucleic acid (Non-Patent Documents 23), and an acidic protein (Non-Patent Documents 24 and 25). For example, it has been reported that a compound (DMAE-PRX) obtained by introducing a cationic N,N-dimethylaminoethyl group (DMAE) into a CD of PRX forms and stabilizes a polyion complex with an anionic plasmid DNA, and contributes to gene introduction. In addition, a cell detachment agent in which an amino group is introduced into a macrocyclic molecule of PRX has been reported (Patent Document 1).

Further, a molecule has been designed such that both ends of the axial molecule of DMAE-PRX are each bonded to a cap via a structure cleavable under a reducing environment; and after gene introduction, the cap is removed and the CDs are released, so that intracellular nucleic acid release is promoted. Use of this molecule reportedly improves the functionality of siRNA or protein after introduction when compared with non-releasing-type DMAE-PRX (Non-Patent Documents 26 and 27). Also, use of multi-arm PRX reportedly improves retention in blood (Non-Patent Document 28). Further, a multi-arm PRX may be used in which DMAE is bonded, via a disulfide linker, to $\alpha$-cyclodextrin, thereby improving the release of plasmid DNA under an intracellular reducing environment; and a functional peptide-linked multi-arm PRX has been reported (Non-Patent Document 29).

PRIOR ART DOCUMENTS

PATENT DOCUMENTS

Patent Document 1: International Publication WO 2015/025815

NON-PATENT DOCUMENTS

Non-patent document 1: S. Kim et al., Genome Res., 24: 112-1019 (2014).
Non-patent document 2: M. wang et al., Proc. Natl. Acad. Sci., 113: 2868-2873 (2015).
Non-patent document 3: Gao, X. et al., Nature 553, 217-221 (2018).
Non-patent document 4: Wei, T. et al., Nat Commun 11, 3232 (2020).
Non-patent document 5: Lee, K. et al., Elife, 6 (2017).
Non-patent document 6: Wang, Y. et al., ACS Appl Mater Interfaces 10, 31915-31927 (2018).
Non-patent document 7: Taharabaru, T. et al., ACS Appl Mater Interfaces; 12, 21386-21397 (2020).
Non-patent document 8: Lee, K. et al., Nat Biomed Eng 1,889-901 (2017).
Non-patent document 9: Lee, B. et al., Nat Biomed Eng 2,497-507 (2018).
Non-patent document 10: Cho, E. Y. et al., J Nanobiotechnology 17, 19 (2019).
Non-patent document 11: Liu, C. et al., Sci Adv 5, eaaw8922 (2019).
Non-patent document 12: Rui, Y. et al., Sci Adv 5, eaay3255 (2019).
Non-patent document 13: Chen, G. et al., Nat Nanotechnol 14, 974-980 (2019).
Non-patent document 14: Akira Harada et al., Nature, 356: 325-327 (1992).
Non-patent document 15: Akira Harada et al., Chem. Rev., 109: 5974-6023 (2009).
Non-patent document 16: Jun Araki et al., Macromolecules, 38: 7524-7527 (2005).
Non-patent document 17: Wenz, G. et al., Chemical Reviews 106, 782-817 (2006).
Non-patent document 18: Tamura, A. et al., Chem Commun (Camb) 50, 13433-13446 (2014).
Non-patent document 19: Higashi, T. et al., Chem Pharm Bull (Tokyo) 67, 289-298 (2019).
Non-patent document 20: Yasuda, Y. et al., J Am Chem Soc 141, 9655-9663 (2019).
Non-patent document 21: Ooya, T. et al., Journal of the American Chemical Society 128, 3852-3853 (2006).
Non-patent document 22: Emami, M. R. et al., Advanced Therapeutics 2, 1900061 (2019).
Non-patent document 23: Tamura, A. et al., Biomaterials 34, 2480-2491 (2013).
Non-patent document 24: Tamura, A. et al., Sci Rep 3, 2252 (2013).
Non-patent document 25: Tamura, A. et al., Macromol Biosci 15, 1134-1145 (2015).
Non-patent document 26: Atsushi Tamura et al., Biomaterials; 34: 2480-2491(2013)
Non-patent document 27: Atsushi Tamura et al., Sci Rep; 3: 2252 (2013).
Non-patent document 28: Ying Ji et al., Biomaterials; 192: 416-428 (2019).
Non-patent document 29: Michael R. Enami et al., Adv. Therap.; 2: 1900061 (2019).


SUMMARY OF THE INVENTION

PROBLEMS TO BE SOLVED BY THE INVENTION

In order to highly efficiently introduce a nucleic acid/protein complex such as Cas9 RNP or a biopolymer such as a protein or a nucleic acid into a cell by using a non-viral carrier, it is necessary to, for example, introduce the biopolymer into a cell and to make the biopolymer quickly escape from endosomes and avoid degradation, then to be released in the cytoplasm, followed by translocation into a nucleus. In this way, a multistep barrier is present. However, for example, a complex such as Cas9 RNP is an amphoteric material composed of a positively charged Cas9 protein and a negatively charged guide RNA. The three-dimensional structure is also complicated. Thus, it is difficult to form a complex with a non-viral carrier such as a cationic liposome or a cationic polymer. This causes low intracellular introduction efficiency. This is a problem (Tao Wan et al., Journal of Controlled Release; 322: 236-247 (2020)).

MEANS FOR SOLVING THE PROBLEMS

As a result of studying better carriers, the present inventors have found that a carrier obtained by introducing an amino group into a modifying moiety on a macrocyclic molecule of a polyrotaxane (PRX) can be simply mixed and flexibly complexed with a protein and/or a nucleic acid, and then stabilized and efficiently taken up by a cell. It has also

been found that a PRX carrier having an amino group-modified macrocyclic molecule can achieve an excellent gene editing effect upon Cas9/sgRNA delivery. As a result of repeatedly devising the modifying group, it has been further found that by modifying the macrocyclic molecule of PRX with a modifying moiety (e.g., a diethylenetriamine (DET) group having both a secondary amine and an amino group) having a monovalent proton at neutral pH and a divalent proton at acidic pH, the complex is efficiently taken up by a cell and then released from the endosome. Thus, the DET group-modified PRX has achieved an excellent gene editing effect and an siRNA-mediated silencing effect.

Furthermore, the present inventors have found that the dissociation of the carrier from the nucleic acid/protein in the cytoplasm is promoted by introducing an intracellular degradable bond between the endcap and the axial molecule and/or between the modifying amino group on the macrocyclic molecule and the macrocyclic molecule. As a result, the present inventors have succeeded in the development of a carrier excellent not only in more efficient complexation with a protein/nucleic acid and intracellular uptake than the conventional carrier, but also efficient release from the endosome after the intracellular uptake, followed by dissociation from the carrier in the cell, which have not been achieved by the conventional carrier. Use of the carrier of the present disclosure enables a nucleic acid/protein to be delivered into a cell very efficiently and simply.

## EFFECTS OF THE INVENTION

The modified PRX of the present disclosure can be used as an excellent carrier for delivering a nucleic acid/protein because the modified PRX can be simply mixed and complexed with a protein/nucleic acid, and just its contact with a cell can cause efficient intracellular uptake, so that escape from the endosome and dissociation from the carrier automatically proceed in the cell.

## BRIEF DESCRIPTION OF THE DRAWINGS

FIG. 1A is a diagram showing the structure of Cas9 RNP and amino acid and phosphate charges. FIG. 1B is a schematic diagram illustrating the structure of BAEE-PRX (Amino-PRX (1G)). FIG. 1C is a diagram showing the structure (automatic molecular imprinting) of BAEE-PRX/Cas9 RNP polyplex.

FIG. 2 is a graph indicating the ζ potential of Cas9 RNP polyplex using BAEE-PRX or BAEE-DEX. The ordinate represents the ζ potential (mV), and the abscissa represents the amino group imprinting rate (%). Each value represents the mean ± S.E. of 3 to 4 experiments. "*" denotes $p < 0.05$ when compared to BAEE-DEX polyplex.

FIG. 3 is a graph showing cellular uptake of a complex of BAEE-PRX and Cas9 RNP at various mixing ratios in HeLa GFP cells. Each value represents the mean ± S.E. of 5 to 6 experiments.

FIG. 4A is a TEM image of Cas9 RNP alone. FIG. 4B is a TEM image of BAEE-PRX/Cas9 RNP polyplex. FIG. 4C is a TEM image of BAEE-DEX/Cas9 RNP polyplex. In FIGS. 4A to 4C, the scale bar = 50 nm.

FIG. 5A is a photograph showing the stability of Cas9 RNP polyplex using BAEE-PRX or BAEE-DEX in the absence or presence of heparin. This photograph was obtained by agarose S gel electrophoresis of Cas9 RNP polyplexes incubated with heparin and then by staining with ethidium bromide. Each numerical value indicates the ratio (w/w) of heparin/sgRNA. FIG. 5B is a graph showing the stability of Cas9 RNP polyplex using BAEE-PRX or BAEE-DEX in the absence or presence of heparin. This graph indicates the percentage (%) of residual polyplex. +BAEE-PRX denotes BAEE-PRX polyplex, and +BAEE-DEX denotes BAEE-DEX polyplex. The ordinate represents the percentage (%) of residual polyplex, and the abscissa represents the heparin/sgRNA ratio (w/w). The percentage of residual polyplex was quantified by ImageJ software based on the band intensity ratio between the polyplex and released Cas9 RNP. Each value represents the mean ± S.E. of 3 to 4 experiments. "*" denotes $p < 0.05$ when compared to BAEE-DEX polyplex.

FIG. 6A is a graph showing uptake of Cas9 RNP polyplex with each cationic polymer into HeLa cells. The horizontal axis represents the kind of cationic polymer. The results include, from the left, the cases of no cationic polymer (Cas9 RNP alone), BAEE-PRX (Amino-PRX (1G)), BAEE-DEX, Linear-polyethyleneimine (L-PEI) (2.5 kDa), L-PEI (25 kDa), branched-polyethyleneimine (B-PEI) (2 kDa), polyamidoamine dendrimer (Dendrimer) (G2), Dendrimer (G3), Dendrimer (G4), and CRISPRMAX. The concentration of Cas9 RNP was 14.6 nM. Each value represents the mean ± S.E. of 4 experiments. FIG. 6B is a diagram showing the chemical structures of the control cationic polymers. L-PEI represents linear polyethyleneimine, B-PEI represents branched PEI, PLL represents poly-L-lysine hydrobromide, and Dendrimer (G2-4) represents polyamidoamine dendrimer (G2-4) (df). Incidentally, the dotted line in the chemical structural formula of B-PEI denotes a C9-10 alkyl group. FIG. 6C is a table showing the number of amino groups of each control cationic polymer and their molecular weight.

FIG. 7 is a graph showing cell viability of HeLa cells after treatment with Cas9 RNP polyplex using PRX modified with each amino group at pH 7.4 or pH 5.5. The concentration of Cas9 RNP was 29.2 nM. The amount of mixed

polymer was set such that 50% amino groups were used for Cas9 RNPs. Each value represents the mean ± S.E. of 3 to 4 experiments. "*" denotes $p < 0.05$ when compared to the polyplex at pH 7.4.

FIG. 8 is a graph showing uptake of Cas9 RNP polyplex using PRX modified with each amino group into HeLa cells. The concentration of Cas9 RNP was 14.6 nM. The amount of mixed polymer was set such that 50% amino groups were used for Cas9 RNPs. Each value represents the mean ± S.E. of 9 experiments.

FIG. 9A is a graph showing the genome editing activity of Cas9 RNP polyplex using PRX modified with each amino group (BAEE-PRX, DET-PRX, or DMAE-PRX) in HeLa/GFP cells. The ordinate represents the percentage of GFP knockout. In each group, the results include, from the left, the cases where the concentration of Cas9/sgGFP was 29.2 nM, 58.4 nM, or 116.8 nM, and the concentration of sgCont was 116.8 nM. Each value represents the mean ± S.E. of 3 to 4 experiments. FIG. 9B is a graph showing the genome editing activity of Cas9 RNP polyplex using PRX modified with each amino group (BAEE-PRX, DET-PRX, or DMAE-PRX) in HeLa/GFP cells. The ordinate represents the percentage of GFP knockout. In each group, the results include, from the left, the cases of 20% imprinting, 50% imprinting, or 100% imprinting, and the case of 100% imprinting for sgCont. The concentration of Cas9 RNP was 29.2 nM. Each value represents the mean ± S.E. of 6 experiments.

FIG. 10 is a graph showing cell viability of HeLa cells after treatment with Cas9 RNP polyplex with DET-PRX or DET-DEX at pH 7.4 or pH 5.5. The concentration of Cas9 RNP was 29.2 nM. The amount of mixed polymer was set such that 50% amino groups were used for Cas9 RNPs. Each value represents the mean ± S.E. of 3 to 4 experiments. "*" denotes $p < 0.05$ when compared to the polyplex at pH 7.4.

FIG. 11 is a schematic diagram illustrating the efficient endosomal disruption effect of DET-PRX polyplex as exerted by the effect of rotation of DET-modified macrocyclic molecules.

FIG. 12A is a schematic diagram illustrating Cas9 RNP release from the endcap-degradable PRX. FIG. 12B is a diagram illustrating the chemical formulas of candidate endcap linkers. R1 represents the endcap side, and R2 represents the axial molecule side.

FIG. 13 is a graph showing the genome editing activity of Cas9 RNP polyplex using DET-PRX with each endcap-degradable linker (amide, carbamate, disulfide, or ketal). The ordinate represents the percentage of GPF knockout. The concentration of Cas9 RNP was 29.2 nM. Each value represents the mean ± S.E. of 3 experiments.

FIG. 14A is a schematic diagram illustrating Cas9 RNP release from the brush-degradable PRX. FIG. 14B is a diagram showing the structure of each amino group that is added to a hydroxyl group of α-CD and can disrupt the endosome and can be cleaved by GSH.

FIG. 15A is a graph showing the genome editing activity of Cas9 RNP polyplex using a mixture of DET-PRX and Cys-PRX. The molar ratio of DET unit : Cys unit was set to 1 : 1. The concentration of Cas9 RNP was 29.2 nM. Each value represents the mean ± S.E. of 9 experiments. "*" denotes $p < 0.05$ when compared to Cas9 RNP alone. FIG. 15B is a graph showing the genome editing activity of Cas9 RNP polyplex using Cys-DET-PRX. The concentration of Cas9 RNP was 29.2 nM. Each value represents the mean ± S.E. of 6 experiments.

FIG. 16A is a scheme for measuring how the concentration of GSH affected the genome editing activity of Cys-DET-PRX. FIG. 16B is a graph showing genome editing activity at each GSH concentration. The ordinate represents a relative value (efficiency of GFP knockout in cells stably expressing GFP) when the genome editing effect at the time of treatment using Cys-DET-PRX/Cas9 RNP polyplex preincubated with 0 mM GSH is set to 1, and the abscissa represents the concentration (mM) of GSH. The concentration of Cas9 RNP was 29.2 nM. Each value represents the mean ± S.E. of 6 experiments.

FIG. 17 is a graph showing a comparison between genome editing activities of CRISPRMAX/Cas9 RNP and Cys-DET-PRX/Cas9 RNP. Each value represents the mean ± S.E. of 6 experiments.

FIG. 18 is a schematic diagram of a process until the efficient genome editing by Cys-DET-PRX is achieved, the process including Cas9 RNP complexation by automatic molecular imprinting, membrane disruption by endosomal protonation and α-CD rotation, degradation of brush-linker and release of Cas9 RNP in the cytosol, and nuclear translocation of Cas9 RNP.

FIG. 19A is a graph showing the particle size of Cys-DET-PRX/Cas9 RNP. Each value represents the mean ± S.E. of 3 experiments. FIG. 19B is a graph showing the ζ potential of Cys-DET-PRX/Cas9 RNP. Each value represents the mean ± S.E. of 3 experiments. FIG. 19C is a graph showing the percentage of DET-PRX degraded after treatment with carboxylesterase. Each value represents the mean ± S.E. of 4 experiments.

FIG. 20 is a graph showing RNAi activities of siRNA complexes with each amino group-modified PRX in HeLa/GFP cells. The volume to amount ratio of Lipofectamine™ 2000 to siRNA was set to 3.75. Each value represents the mean ± S.E. of 6 experiments.

FIG. 21 is a graph showing a comparison between in vivo genome editing activities of CRISPRMAX/Cas9 RNP and Cys-DET-PRX/Cas9 RNP. The ordinate represents the relative value of the fluorescence intensity in the tumor when the sample was directly administered into the tumor of each tumor-bearing mouse transplanted with HeLa/GFP cells. Each value represents the mean ± S.E. of experiments using 3 to 5 mice.

FIG. 22 is a graph showing RNAi activities of siRNA complexes with each amino group-modified PRX in HeLa/GFP

cells. The charge ratio between Amino-PRX and siRNA was set to 10. Each value represents the mean ± S.E. of 6 experiments. "*" denotes $p < 0.05$ when compared to siRNA alone, "†" denotes $p < 0.05$ when compared to +DMAE-PRX, and "‡" denotes $p < 0.05$ when compared to +BAEE-PRX. Note that +BAEE-PRX indicates BAEE-PRX/siRNA, +DET-PRX indicates DET-PRX/siRNA, and +DMAE-PRX indicates DMAE-PRX/siRNA.

FIG. 23 is a graph showing RNAi activities of siRNA complexes with each Amino-PRX (3G) in HeLa/GFP cells. The charge ratio between Amino-PRX (3G) and siRNA was set to 10. "*" denotes $p < 0.05$ when compared to siRNA alone and "†" denotes $p < 0.05$ when compared to amide-DET-PRX. Note that siGFP alone represents siRNA alone, +Amide-DET-PRX represents amide-DET-PRX/siRNA, +Carbamate-DET-PRX represents carbamate-DET-PRX/siRNA, +Disulphide-DET-PRX represents disulfide-DET-PRX/siRNA, and +Ketal-DET-PRX represents ketal-DET-PRX/siRNA.

FIG. 24 is a graph showing cell viability of HeLa cells after treatment with a complex of siRNA/Amino-PRX (5G) or Lipofectamine™ 2000. The volume to amount ratio of Lipofectamine™ 2000 to siRNA was set to 3.75. In addition, the charge ratio between Amino-PRX (5G) and siRNA was set to 10. Each value represents the mean ± S.E. of 7 to 8 experiments. "*" denotes $p < 0.05$ when compared to Lipofectamine™ 2000/siRNA.

FIG. 25 is a graph showing gene knockdown activity of a complex of ASO/Amino-PRX (5G) in HeLa/GFP cells. Each value represents the mean ± S.E. of 6 experiments. "*" denotes $p < 0.05$ when compared to ASO alone and "†" denotes $p < 0.05$ when compared to +Lipofectamine™ 2000.

FIG. 26 is a graph showing cell viability of HeLa cells after treatment with a complex of Amino-PRX (5G)/ASO or Lipofectamine™ 2000/ASO. The volume to amount ratio of Lipofectamine™ 2000 to ASO was set to 3.75. In addition, the charge ratio between Amino-PRX (5G) and ASO was set to 10. Each value represents the mean ± S.E. of 7 to 8 experiments. "*" denotes $p < 0.05$ when compared to Lipofectamine™ 2000/ASO.

FIG. 27 is a graph showing gene knockdown activity of a complex of gapmer-type ASO/Amino-PRX (5G) in HeLa/GFP cells. Each value represents the mean ± S.E. of 3 experiments. Note that GFP gapmer indicates gapmer type ASO alone, +5G NP2 indicates Amino-PRX (5G) NP2/gapmer-type ASO, +5G NP5 indicates Amino-PRX (5G) NP5/gapmer-type ASO, and +5G NP10 indicates Amino-PRX (5G) NP10/gapmer-type ASO.

FIG. 28 is fluorescence images showing intracellular β-gal enzyme activity in HeLa cells after treatment with Amino-PRX (5G)/β-Gal complex. Regarding the images, a column of SPiDER-β-Gal shows fluorescence (green) derived from SPiDER-β-Gal, a column of Hoechst 33342 shows fluorescence (blue) of each stained nucleus, a column of Merge shows each image where both fluorescence images are merged, and a column of FC shows each phase contrast image. Note that +Amino-PRX (5G) NC2 represents Amino-PRX (5G) NC2/β-gal, +Amino-PRX (5G) NC5 represents Amino-PRX (5G) NC5/β-gal, and +Amino-PRX (5G) NC10 represents Amino-PRX (5G) NC10/β-gal. NC indicates the ratio of amino groups of Amino-PRX (5G) to acidic amino acid residues (COOH) of β-gal.

FIG. 29 is a graph showing the level of mCherry expression in HeLa cells after treatment with a complex of Amino-PRX (5G)/mCherry mRNA. Each value represents the mean ± S.E. of 3 experiments. Note that in the graph, +Lipo2000 represents Lipofectamine™ 2000/mCherry mRNA, 5G NP0.5 represents Amino-PRX (5G) NP0.5/mCherry mRNA, +5G NP0.75 represents Amino-PRX (5G) NP0.75/mCherry mRNA, +5G NP1 represents Amino-PRX (5G) NP1/mCherry mRNA, and +5G NP2 represents Amino-PRX (5G) NP2/mCherry mRNA.

FIG. 30 is a graph showing genome editing activity of a complex of each Amino-PRX/Cas9 RNP in HeLa/GFP cells. Each value represents the mean ± S.E. of 3 experiments. The concentration of Cas9 RNP was 29.2 nM. "*" denotes $p < 0.05$ when compared to Control, "†" denotes $p < 0.05$ when compared to Cas9 RNP, and "‡" denotes $p < 0.05$ when compared to CRISPRMAX. Note that in the graph, Cas9 RNP indicates Cas9/sgGFP RNP alone, CRISPRMAX (cont) indicates CRISPRMAX/Cas9 RNP (sgCont), CRISPRMAX indicates CRISPRMAX/Cas9 RNP (sgGFP), less substituted amino-PRX (5G) (cont) indicates Amino-PRX (5G)/Cas9 RNP (sgCont), and less substituted amino-PRX (5G) indicates Amino-PRX (5G)/Cas9 RNP (sgGFP).

## MODE FOR CARRYING OUT THE INVENTION

### (Polyrotaxane)

The "polyrotaxane" includes a plurality of macrocyclic molecules and an axial molecule penetrating the ring of each macrocyclic molecule and having caps at both ends. A schematic diagram of the chemical structure of the polyrotaxane is shown below. The poly-pseudorotaxane has a plurality of macrocyclic molecules and an axial molecule penetrating the ring of each macrocyclic molecule, but does not have any cap structure.

[Chemical Formula 1]

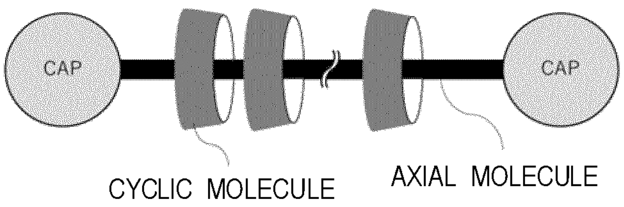


The "axial molecule" is usually a polymer, and is a polymer of single monomers (homopolymer), a copolymer composed of two or more different monomers, or the like. The "polymer" refers to a polymer in which many of one or more monomers are repeatedly bonded. The monomer is usually a molecule having one carbon-carbon double bond or a molecule having at least two functional groups per molecule. The copolymer may be, for example, a random copolymer, an alternating copolymer, and/or a block copolymer. When the axial molecule is a homopolymer, examples of the polymer include polyalkylene oxide, polyethylene glycol, polypropylene glycol, polyvinyl ether, polymethyl vinyl ether, polyethyleneimine, poly(trimethylene oxide), poly(ε-caprolactone), polylactic acid, polyamino acid, poly(ε-lysine), polyamide, poly(iminooligomethylene), ionene, poly(vinyldiene chloride), polypropylene, oligopropylene, polyethylene, oligoethylene, poly(alkylenebenzimidazole), polyurethane, poly(viologen), poly(N-dimethyldecamethylene ammonium), poly(dimethylsiloxane), polyaniline, polycarbonate, poly(methyl methacrylate), poly(N-acylethyleneimine), a poly(4-vinylpyridin)-dodecylbenzene sulfonate complex, a fullerene-polyethylene glycol conjugate, or a hydrophobized polysaccharide. When the axial molecule is a copolymer, examples thereof include a copolymer of polyethylene glycol and polypropylene glycol, a copolymer of poly(ε-caprolactone) and polylactic acid, a copolymer of polylactic acid and polyethylene glycol, a polyethylene glycol-polysaccharide graft copolymer, or a polypropylene glycol-polysaccharide graft copolymer. In addition, the axial molecule may be a branched multi-arm polymer, and may be, for example, star-shaped polyethylene glycol, highly branched polyether, highly branched oligoethylene glycol, or highly branched oligopropylene glycol. These polymers may be optionally modified or substituted with substituents. The degree of polymerization and the molecular weight of the axial molecule depend on the target nucleic acid/protein to be introduced into a cell, and any length is acceptable if the axial molecule can penetrate through the required number of macrocyclic molecules. For example, it is possible to use a polymer having a number average molecular weight (Mn) of about 200 to 1,000,000 Da, about 400 to 50,000 Da, about 500 to 40,000 Da, or about 1,000 to 30,000 Da.

As used herein, the term "macrocyclic molecule" means a cyclic molecule having an internal opening (cavity) sufficiently large for the axial molecule to penetrate and having a group (e.g., a hydroxyl group) that can be modified. Examples of the macrocyclic molecule include cyclic polyether, cyclic polyester, cyclic polyetheramine, cyclic polyamine, crown ether, cucurbit[n]uril, calixarene, cyclic amide, a transition metal complex, cyclodextrin, a cyclodextrin derivative, or any combination thereof. The "cyclodextrin" is a cyclic oligosaccharide compound, and examples include α-cyclodextrin (hexasaccharide), β-cyclodextrin (heptasaccharide), or γ-cyclodextrin (octasaccharide). The macrocyclic molecule may be substituted with a substituent other than the "modifying moiety" described later, or may be substituted with a methyl group, a hydroxyethyl group, a hydroxypropyl group, an acetyl group, a carboxymethyl group, a succinyl group, a glucosyl group, a carboxyethyl group, a sulfobutyl group, an amino group, a halogen atom, or any combination thereof.

In the polyrotaxane or the poly-pseudorotaxane, the axial molecule penetrates through each macrocyclic molecule by penetrating the opening of the macrocyclic molecule. The polyrotaxane or the poly-pseudorotaxane has a plurality of macrocyclic molecules threaded along the axial molecule as described above. The number of macrocyclic molecules per polyrotaxane molecule can be set according to the purpose, and may be, for example, about 5 to about 200 or about 10 to about 100 as an average value. Each macrocyclic molecule is not covalently bonded to the axial molecule, and can thus move in the rotational direction and the axial direction with respect to the axial molecule.

In the polyrotaxane of the present disclosure, a "modifying moiety" (hereinafter, simply referred to as a "modifying moiety") having a monovalent proton at neutral pH or a divalent proton at acidic pH (preferably, weakly acidic pH (e.g., pH 5.5)) is bonded to at least one macrocyclic molecule. The modifying moiety is preferably a group having a secondary amine and an amino group, and is typically a group represented by $-L^1-NH-L^2-NH_2$ (where $L^1$ and $L^2$ are the same or different and each represent a linear or branched C1-6 alkylene group), and is, for example, diethylenetriamine. The modifying moiety can be safe for in vivo components because of having a monovalent proton at neutral pH. On the other hand, the endosomal membrane can be disrupted by having a divalent proton at an acidic pH (preferably, weakly acidic pH (e.g., pH 5.5)), namely an endosomal environment. As a result, the polyion complex intracellularly up-taken into the endosome can disrupt the endosome and be then released into the cytoplasm.

Examples of the "linear or branched C1-6 alkylene group" herein include $-CH_2-$, $-(CH_2)_2-$, $-(CH_2)_3-$, $-(CH_2)_4-$, $-(CH_2)_5-$, $-(CH_2)_6-$, $-CH(CH_3)CH_2-$, $-CH_2CH(CH_3)-$, $-CH(CH_2CH_3)CH_2-$, $-CH_2CH(CH_2CH_3)-$, $-CH(CH_3)CH_2CH_2-$, $-CH_2CH(CH_3)CH_2-$, or $-CH_2CH_2CH(CH_3)-$.

In another aspect, in the polyrotaxane of the present disclosure, a group having, via an intracellularly degradable bond, an amino group (hereinafter, sometimes referred to as an "intracellularly degradable amino group") is bonded to at least one macrocyclic molecule. The group having, via an intracellularly degradable bond, an amino group is typically a group represented by $-L^3-X-L^4-NH_2$ (where $L^3$ and $L^4$ are the same or different and are each a linear or branched C1-6 alkylene group or not present, and X is an intracellularly degradable bond). Examples of the group having an amino group (in the above formula, a group corresponding to $-L^4-NH_2$) include $CH_2-NH_2$, $-(CH_2)_2-NH_2$, $-(CH_2)_3-NH_2$, $-(CH_2)_4-NH_2$, $-(CH_2)_5-NH_2$, $-(CH_2)_6-NH_2$, or $-CH(CH_3)CH_2-NH_2$.

The "intracellularly degradable bond" means a bond that is easily decomposed under conditions such as an intracellular environment different from an extracellular environment, for example, physicochemical conditions such as pH or biological conditions such as intracellular enzymes. Preferably, the intracellularly degradable bond is a bond that is hardly degraded extracellularly. Here, the terms "degradable" and "hardly degraded" do not have absolute meanings, and do not mean that the bond is completely degradable and not at all degraded, respectively. The terms "degradable" and "hardly degraded" mean that the bond is relatively easily degradable and relatively hardly degraded, respectively, when extracellular and intracellular environments are compared. For example, the intracellular environment condition different from the extracellular environment condition may be a GSH concentration. In this case, the intracellularly degradable bond is hardly degraded at a GSH concentration of 0.2 mM or lower (extracellular concentration), and the bond may be degradable at a GSH concentration of 2 to 10 mM (intracellular concentration). The intracellularly degradable bond may be, for example, a carbamate (-NH(C=O)O-), ketal ($-OC(CH_3)_2O-$), amide (-NHCO-), disulfide (-S-S-), acetal (-C(OH)O-), orthoester, vinyl ether ($-CH_2=CH-O-$), hydrazide, or ester (-COO-) bond.

The "group having, via an intracellularly degradable bond, an amino group" is preferably $-(CH_2)_2-S-S-(CH_2)_2-NH_2$ (cystamine).

In the polyrotaxane of the present disclosure, when an intracellularly degradable amino group, in addition to the modifying moiety, is bonded, the modifying moiety and the intracellularly degradable amino group are usually different groups. In this case, preferably, the modifying moiety is bonded to the macrocyclic molecule through a bond that is not degraded or hardly degraded in the cell. Here, until the intracellular release of the polyion complex, some of the intracellularly degradable amino group bonds may be degraded, so that the amino group is dissociated from the macrocyclic molecule. Even in this case, the amino acid of the modifying moiety allows the bond between the polyrotaxane and the biological material to be maintained. Also, after the intracellular release, almost all the intracellularly degradable amino group bonds are cleaved. This can weaken the binding between the biological material and the polyrotaxane, thereby promoting the dissociation of the biological material from the polyrotaxane.

For example, the macrocyclic molecule may have a hydroxyl group like in the case of cyclodextrin. In this case, the hydroxyl group may be replaced by the modifying moiety and/or a group having an amino group bonded via an intracellularly degradable bond (hereinafter, in this paragraph, collectively referred to as a "substituent of the macrocyclic molecule"). The hydroxyl group of the cyclodextrin may be a hydroxyl group of glucose constituting the cyclodextrin. In this case, the substituent of the macrocyclic molecule may be bonded to an oxygen atom constituting the hydroxyl group via -O-CO-NH-, -O-COO-, -O-OC-, -O-, -O-C(OH)-, or -O-C(=S)NH- (in any case, the leftmost -O-represents the oxygen atom derived from the hydroxyl group). The following structural formula can represent, for example, the case where the macrocyclic molecule is α-cyclodextrin and an oxygen atom constituting a hydroxyl group of the α-cyclodextrin is bonded via -O-CO-NH- to a substituent represented by $-L^1-NH-L^2-NH_2$.

[Chemical Formula 2]

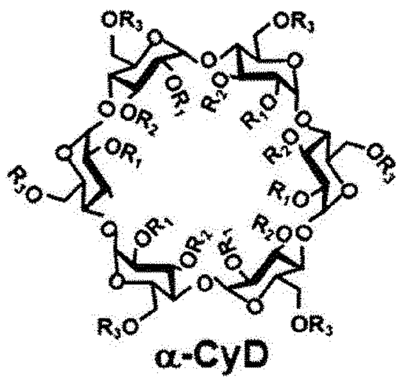

α-CyD

**$R = -CO-NH-L^1-NH-L^2-NH_2$**

The "cap" (CAP) is a bulky substituent bonded to each end of the polyrotaxane, and prevents the macrocyclic

molecule(s) from being detached from the axial molecule. Examples of the cap include dinitrophenyl groups, cyclodextrins, adamantane groups, trityl groups, fluoresceins, silsesquioxanes, pyrenes, substituted benzenes (optionally substituted with a substituent(s) which may be one or more alkyl, alkyloxy, hydroxy, halogen, cyano, sulfonyl, carboxyl, amino, and/or phenyl groups), steroids, amino acids, oligopeptides, oligosaccharides, sugar derivatives, groups having one or more benzene rings (e.g., a benzyloxycarbonyl (Z) group, 9-fluoroolenylmethyloxycarbonyl (Fmoc) group, benzyl ester (OBz) group), or groups having one or more tert-butyl groups (e.g., a tert-butylcarbonyl (Boc) group, amino acid tert-butyl ester (OBu) group). Preferred are dinitrophenyl groups, cyclodextrins, adamantane groups, trityl groups, fluoresceins, silsesquioxanes, or pyrenes, and more preferred are adamantane groups or cyclodextrins. The cap is bonded to each end of the poly-pseudorotaxane, thereby preventing the macrocyclic molecules threaded along the axial molecule from being detached from the axial molecule. Thus, the cap has sufficient steric bulkiness to block the macrocyclic molecules from escaping the axial molecule.

Preferably, the cap is bonded, via an intracellularly degradable bond, to the axial molecule. Here, the "intracellularly degradable bond" is in accordance with the above definition. Preferably, the cap is bonded, via a carbamate bond, to the axial molecule. The cap can be bonded via the intracellularly degradable bond to the axial molecule. One can refer to, for example, the procedures described below: Journal of Controlled Release 76 (2001) 11 -25; Biomacromolecules 2003, 4, 1426-1432; Langmuir 2011, 27 (2), 612-617; Angew. Chem. Int. Ed. 2013, 52, 7300-7305; J. Mater. Chem. B, 2013, 1, 3535-3544; Biomaterials, 34, 2480-2491 (2013); or Scientific Reports, 3, 2252 (2013).

The polyrotaxane of the present disclosure can be produced by a method well-known to those skilled in the art in accordance with the methods of Production Examples 1 to 10 described later.

Another aspect of the present disclosure provides a polyrotaxane composition including a polyrotaxane having at least one macrocyclic molecule bonded to an amine-containing modifying moiety, and a polyrotaxane having at least one macrocyclic molecule bonded to a group having, via an intracellularly degradable bond, an amino group.

Further, another aspect of the present disclosure provides a polyrotaxane including a plurality of macrocyclic molecules, an axial molecule penetrating a ring of each macrocyclic molecule, and caps bonded to ends of the axial molecule, wherein an amine-containing modifying moiety is bonded to at least one of the macrocyclic molecules, and a group having, via an intracellularly degradable bond, an amino group is bonded to at least one of the macrocyclic molecules.

(Polyion Complex)

Another aspect of the present disclosure relates to a polyion complex of a biological material and the polyrotaxane, and a method of producing the polyion complex. The "biological material" means a substance composed of a natural or synthetic amino acid(s) or nucleic acid to be introduced into a cell, and may be, for example, a nucleic acid molecule, a vector, a protein, or a peptide, or a fusion thereof, or a complex thereof. Examples of the nucleic acid molecule include guide RNAs (including derivatives such as single-guide RNAs (sgRNAs) or prime-editing guide RNAs (pegRNAs) (Chow, R.D. et al. Nat Biomed Eng (2020))), CRISPR RNAs (crRNAs), trans-activating crRNAs (tracrRNAs), siRNAs (including derivatives such as shRNAs), decoys, CpG oligos, miRNAs, antisense DNAs, antisense RNAs, aptamers, mRNAs, or nucleic acid vaccines (e.g., mRNA vaccines, DNA vaccines). Examples of the vector include viral vectors or plasmid vectors. Examples of the protein/peptide include functional proteins/peptides such as nucleases (e.g., Cas proteins such as Cas9 nuclease, Cas9 nickase, Cas 12a, Cas13 a), deaminases (e.g., cytidine deaminase, adenosine deaminase), or reverse transcriptases, or labeled proteins/peptides such as luciferase or fluorescent/radiolabeled proteins. The protein/peptide may be, for example, a nucleic acid binding protein/peptide having a DNA binding domain (e.g., a zinc finger, helix-turn-helix, helix-loop-helix, wing helix, leucine zipper) or an RNA binding domain (e.g., a zinc finger, KH, S1, PAZ, PUF, PIWI, or RRM (RNA recognition motif) domain). Examples of the fusion can include zinc finger nuclease or Transcription Activator-Like Effector Nuclease (TALEN). The complex includes a protein-DNA complex or a protein-RNA complex. Examples thereof include a complex of a Cas protein and a guide RNA (typically, a complex of Cas9 and sgRNA (Cas9 RNP)).

The imprinting rate in the polyion complex can be, for example, 20% or more, and may be from 20 to 100%, from 20 to 90%, from 20 to 80%, or from 20 to 50%. The imprinting rate may be, for example, 50% or more, or may be from 50 to 100%, from 50 to 90%, or from 50 to 80%. As used herein, the "imprinting rate" means the percentage of the total number of amines (primary, secondary, or tertiary amine) present at the end (including the vicinity of the end) of the group (modifying moiety and/or intracellularly degradable amino group) bonded to a macrocyclic molecule of the polyrotaxane to the maximum number of cationic monomers that can bind to the biological material contained in the polyion complex. For example, when the group bonded to the macrocyclic molecule is DET, only the amino group at the end of DET is counted as the "amine present at the end of the group bonded to the macrocyclic molecule", and the internal secondary amine is not counted. In other words, one DET group provides one amine to a macrocyclic molecule on the polyrotaxane.

The polyion complex in the present disclosure can be prepared by mixing the biological material with the

polyrotaxane of the present disclosure. The mixing can be performed by stirring at room temperature in a buffer or a culture medium. The mixing time can be set, if appropriate, according to, for instance, the polyrotaxane and the biological material to be used and the concentrations thereof, but may be usually from 5 minutes to overnight, from 5 minutes to 6 hours, from 5 to 180 minutes, from 5 to 120 minutes, from 5 to 60 minutes, or from 5 to 30 minutes.

(Method of delivering biological material into cell)

Another aspect of the present disclosure relates to a method of delivering a biological material into a cell, the method including bringing a polyion complex in the present disclosure into contact with a cell to incorporate the polyion complex into the cell. This method may include, if necessary, mixing a biological material with a polyrotaxane of the present disclosure to form a polyion complex, and bringing the polyion complex into contact with a cell to incorporate the polyion complex into the cell.

In addition, in this method, a complex of a Cas9 protein and a guide RNA may be used as a biological material. Thus, this method can be used as a method for genome editing. Hence, one aspect of the present disclosure relates to a method for genome editing, the method including bringing a polyion complex between a complex of a Cas9 protein and a guide RNA (Cas9 RNP) and a polyrotaxane of the present disclosure into contact with a cell to incorporate the polyion complex into the cell. This method may include, if necessary, producing a polyion complex by mixing a complex of a Cas9 protein and a guide RNA (Cas9 RNP) with a polyrotaxane of the present disclosure, and bringing the polyion complex into contact with a cell to incorporate the polyion complex into the cell.

The contact between the polyion complex and the cell can be performed by adding the polyion complex to a cell culture medium and culturing the cell. The culture medium and culture conditions to be used can be set, if appropriate, according to the cells to be used, but are usually under the conditions at 37°C and 5% $CO_2$. The culture time can be from 1 hour to overnight, from 1 to 12 hours, from 2 to 6 hours, or from 3 to 5 hours. If necessary, the cells may be washed to remove the polyion complex, and may then be further cultured for a period of 1 to 4 days or longer or 6 to 10 days or shorter.

(Cell Delivery Agent)

The polyrotaxane of the present disclosure allows for efficient uptake by the endosome, intracellular release from the endosome, and dissociation from the biological material in the cytoplasm after the release. This enables efficient delivery of the biological material into, in particular, the cytoplasm or nucleus. Therefore, the polyrotaxane of the present disclosure can be used as an agent for delivering a biological material into a cell, in particular, a cytoplasmic delivery agent or a nuclear delivery agent. The cell delivery agent may optionally include, in addition to the polyrotaxane, for example, a buffer and/or a stabilizer.

(Pharmaceutical Composition)

Another aspect of the present disclosure relates to a pharmaceutical composition including the polyrotaxane or the polyion complex. The biological material in the pharmaceutical composition of the present disclosure is a substance (e.g., a Cas protein/sgRNA complex, a nucleic acid medicine, a vaccine) that is introduced into a cell or a nucleus and then exhibits a therapeutic effect.

One aspect of the present disclosure relates to a method of treating or ameliorating a disease or condition in a patient, including administering an effective amount of the polyion complex to a patient in need thereof. The "treatment" is an approach for obtaining beneficial or desired clinical outcomes. For purposes of the present disclosure, the beneficial or desired clinical outcomes include, but are not limited to, alleviation of one or more symptoms, diminishment of region of disease, stabilized (i.e., not exacerbated) disease condition, delay or slowing of disease progression, recovery from or alleviation of and remission (partial or total) of disease condition, whether detectable or undetectable. The "treatment" may also mean prolonging life expectancy relative to expected life expectancy if not receiving treatment. The "effective amount" is an amount sufficient to achieve beneficial or desired clinical outcomes, including clinical results. The effective amount can be administered in one or more doses. The therapeutic target includes mammals such as humans, cows, horses, dogs, cats, or sheep, and is preferably a human.

The pharmaceutical composition may contain a pharmacologically acceptable carrier (additive for preparation). A person skilled in the art can appropriately select, according to the form of the composition, the type of the additive for preparation used in the production of the pharmaceutical composition, the ratio of the additive for preparation to the active ingredient, or the method of producing the pharmaceutical composition. As the additive for preparation, an inorganic or organic substance, or a solid or liquid substance (e.g., physiological saline) can be used. Generally, the additive may be blended in an amount from 1 wt% to 99 wt% based on the weight of the active ingredient.

The pharmaceutical composition of the present disclosure can be an oral or parenteral pharmaceutical composition, and may be, for example, an injectable preparation such as an injectable preparation for intravenous injection, an injectable preparation for subcutaneous administration, an injectable preparation for intramuscular injection, or a drip infusion. Alternatively, the pharmaceutical composition of the present disclosure may be a cell treatment agent in cell therapy. When administered to a subject such as a mammal (including a model animal (e.g., a mouse) or a human), the above-described preparation may be orally administered, or an injection or a drip infusion may be administered in the blood (intravenously or intraarterially). The dose and the administration frequency vary depending on, for example, the target mammal, symptom, disease or disorder, age, sex, body weight, and dosage form. For example, in the case of administration into human blood, the daily dose can be from 0.001 to 100 g or from 0.01 to 1000 mg/kg. In addition, the daily dose may be divided and administered in several portions. The administration frequency may be daily, weekly, every two weeks, monthly, or once every several months. The administration period can be appropriately determined in view of improvement of symptoms, and may be 1 month, several months, half a year, 1 year, several years, 5 years, or 10 years.

Hereinafter, the present disclosure will be described in more detail using Examples. However, this does not limit the scope of the present disclosure. Note that documents cited throughout this specification are incorporated herein by reference in their entirety.

EXAMPLES

(Materials)

α-CD was donated by NIHON SHOKUHIN KAKO CO., LTD. (Tokyo, Japan). N,N-carbonyldiimidazole (CDI), ethylenediamine, BOP reagent, 1-hydroxybenzotriazole (HOBt), N-ethyldiisopropylamine (EDIPA), triethylamine (TEA), cystamine dihydrochloride, and 1-adamantanecarboxaldehyde were purchased from FUJIFILM Wako Pure Chemical Corporation (Osaka, Japan). Then, 1-adamantaneacetic acid, 1-adamantanamine, diethylenetriamine (DET), DMAE, and dextran 70 were purchased from Tokyo Chemical Industry Co., Ltd. (Tokyo, Japan). PEG (20 kDa), BAEE, 2,2-bis(aminoethoxy)propane, and dendrimers (G2-4) were purchased from Sigma-Aldrich Japan (Tokyo, Japan). PEI and PLL were purchased from Polysciences Asia Pacific, Inc. (Taipei, Taiwan). Recombinant Streptococcus pyogenes Cas9 protein (with the N-terminus modified with NLS) was purchased from Takara Bio Inc. (Shiga, Japan). Here, sgRNA, crRNA, and ATTO-tracrRNA were purchased from Integrated DNA Technologies Japan (Tokyo, Japan). Table 1 shows the sequences of the sgRNA target and the PCR primers. CRISPRMAX and Opti-MEM were purchased from Thermo Fisher Scientific K.K. (Tokyo, Japan). All other chemicals and solvents used were analytical reagent grade and deionized water.

[Table 1]

| **Sequences of sgRNA target and PCR primers for T7E1 assay** | | |
|---|---|---|
| Name | Sequence | SEQ ID NO |
| GFP targeted sgRNA (sgGFP) | 5'-GGGCGAGGAGCUGUUCACCG-3' | 1 |
| Non-targeted sgRNA (sgCont) | 5'-AAAUGUGAGAUCAGAGUAAU-3' | 2 |
| crRNA (non-target) | 5'-AAAUGUGAGAUCAGAGUAAU-3' | 3 |
| GFP forward primer | 5'-ATGGTGAGCAAGGGCGAG-3' | 4 |
| GFP reverse primer | 5'-CCGGTGGTGCAGATGAACTT-3' | 5 |
| The sequences of sgRNAs show only DNA targeted regions. | | |

(Cell Culture)

HeLa cells, a human neuroblastoma cell line and human cervical epithelial cancer, were obtained from RIKEN BioResource Center (TSUKUBA, JAPAN) and maintained in DMEM (high glucose) supplemented with 10% fetal bovine serum (FBS) and containing glutamine (2 mM), penicillin (100 U/mL), and streptomycin (100 mg/L) at 37°C under humidified 5% $CO_2$ atmosphere. HeLa GFP cells, namely HeLa cells stably expressing GFP, were purchased from Cell Biolabs, Inc. (San Diego, USA) and maintained in the same manner as for HeLa cells except that the medium contained 10 μg/mL blasticidin.

(Data Analysis)

Data is provided as mean ± S.E., and statistical significance was determined by Scheffe's test. The case where the p value was $< 0.05$ was considered to be statistically significant.

(Production Example 1) Preparation of Carbamate-PRX

Carbamate-PRX was prepared according to the method reported by Araki et al. (Araki, J., et al., Macromolecules 38, 7524-7527 (2005)) with slight modifications. To obtain diamino-terminated PEG (20 kDa) (PEG-DAT), PEG (20 kDa) (56 g, 2.8 mmol) and CDI (2.0 g, 12.4 mmol) were dissolved in tetrahydrofuran (THF) (200 mL) and stirred at 50°C for 18 hours under nitrogen purge. The reactant was added dropwise to ethylenediamine (6.0 mL, 88.0 mmol), and the mixture was stirred at 50°C for 2 hours. Ethanol (200 mL) was added, and the mixture was allowed to stand at -20°C for 2 hours. The precipitate was collected by centrifugation, washed several times with cold ethanol, and dried under reduced pressure. Yield: 52.7 g, 94%.

To obtain α-CD/PEG-DAT poly-pseudorotaxane, PEG-DAT (3.0 g) was added to 12% (w/v) α-CD aqueous solution (100 mL). After stirring at 4°C overnight, the precipitate was collected by centrifugation and dried by lyophilization.

To obtain carbamate-PRX, 1-adamantaneacetic acid (2.45 g, 12.6 mmol), BOP reagent (5.25 g, 11.8 mmol), HOBt (1.75 g, 11.4 mmol), and EDIPA (2.28 mL, 13.2 mmol) were dissolved in dimethylformamide (DMF) (100 mL), and α-CD/PEG-DAT poly-pseudorotaxane (14 g) was added. After stirring at 4°C for 48 hours under nitrogen purge, the precipitate was collected by centrifugation and washed twice with methanol/DMF (1 : 1 v/v) and methanol, respectively. The resulting product was dissolved in dimethyl sulfoxide (DMSO) and precipitated by adding excess water. The same procedure was repeated three times and the resulting precipitate was dried by lyophilization. Yield: 10.23 g, 82% (based on PEG).

(Production Example 2) Preparation of Amide-PRX

Amide-PRX was prepared according to the method reported by Araki et al. (Araki, J., et al., Macromolecules 38, 7524-7527 (2005)) with slight modifications. To obtain HOOC-PEG-COOH (20 kDa) (PEG-COOH), PEG (20 kDa) (10 g, 0.5 mmol), 2,2,6,6-tetramethylpiperidine 1-oxyl (100 mg, 0.64 mmol), NaBr (100 mg, 0.97 mmol), and NaClO (effective chlorine concentration > 5%; 10 mL) in water were stirred at room temperature for 15 minutes. To quench the reaction, ethanol (10 mL) was added to the reactant and deionized by adding 1 N HCl to the reactant to adjust the pH < 2. After dialysis against water by using Spectra/Por™ membrane MWCO: 10 kDa, the sample was concentrated with an evaporator and dried by lyophilization. Yield: 9.53 g, 95%.

The α-CD/PEG-COOH poly-pseudorotaxane was prepared according to the method described above using PEG-COOH.

To obtain amide-PRX, 1-adamantanamine (72.5 mg, 0.48 mmol), BOP reagent (212.5 g, 0.48 mmol), and EDIPA (90.25 μL, 0.52 mmol) were dissolved in DMF (25 mL), and α-CD/PEG-COOH poly-pseudorotaxane (3.5 g) was added. After stirring at 4°C for 48 hours under nitrogen purge, the precipitate was collected by centrifugation and washed twice with methanol/DMF (1:1 v/v) and methanol, respectively. The resulting product was dissolved in DMSO and precipitated by adding excess water. The same procedure was repeated three times and the resulting precipitate was dried by lyophilization. Yield: 3.12 g, 92.4% (based on PEG).

(Production Example 3) Preparation of Ester-PRX

Ester-PRX was prepared according to Production Example 2 using PEG-bissuccinic acid (20 kDa) (PEG-bis SA). To obtain PEG-bis SA, PEG (20 kDa) (5.8 g, 0.29 mmol), 1,8-diazabicyclo[5.4.0]undeca-7-ene (85.5 μL, 0.57 mmol), and succinic anhydride (1.7 g, 17 mmol) were dissolved in pyridine (26 mL), and the mixture was stirred at 55°C for 18 hours under nitrogen purge. Ethanol (25 mL) was added, and the mixture was allowed to stand at -20°C for 2 hours. Thereafter, the precipitate was collected by centrifugation, washed several times with cold ethanol, and dried under reduced pressure. Yield: 4.68 g, 81%.

The α-CD/PEG-bis SA poly-pseudorotaxane was prepared according to the method described above using PEG-bis SA.

To obtain ester-PRX, 1-adamantanamine (72.5 mg, 0.48 mmol), BOP reagent (212.5 mg, 0.48 mmol) and EDIPA (90.3 μL, 0.52 mmol) were dissolved in DMF (25 mL) and α-CD/PEG-bis SA poly-pseudorotaxane (3.5 g) was added. After stirring at 4°C for 48 hours under nitrogen purge, the precipitate was collected by centrifugation and washed twice with methanol/DMF (1:1 v/v) and methanol, respectively. The resulting product was dissolved in DMSO and precipitated by adding excess water. The above procedure was repeated three times and the resulting precipitate was dried by lyophilization. Yield: 2.71 g, 87% (based on PEG).

(Production Example 4) Preparation of Disulfide-PRX

Disulfide-PRX was prepared according to Production Example 1 using PEG-biscystamine (20 kDa) (PEG-bis Cys). To obtain PEG-bis Cys, PEG (20 kDa) (14.5 g, 0.73 mmol), CDI (3.3 g, 20.0 mmol), and TEA (208 μL, 1.5 mmol) were dissolved in DMSO (500 mL), and the mixture was stirred at room temperature for 24 hours under nitrogen purge. Cystamine dihydrochloride (3.5 g, 15.5 mmol) was dissolved in DMSO (50 mL), and the mixture was stirred for 30 minutes in the presence of TEA (2.3 mL, 31.0 mmol) for desalting. The reactant was added dropwise to desalted cystamine, and the mixture was stirred at room temperature for 48 hours. Ethanol (550 mL) was added, and the mixture was allowed to stand at -20°C for 2 hours. The precipitate was collected by centrifugation, washed several times with cold ethanol, and dried under reduced pressure. Yield: 14.2 g, 98%.

The α-CD/PEG-bis Cys poly-pseudorotaxane was prepared according to the method described above using PEG-bis Cys.

To obtain disulfide-PRX, 1-adamantaneacetic acid (0.61 g, 3.2 mmol), BOP reagent (1.31 g, 3.0 mmol), HOBt (0.44 g, 2.9 mmol), and EDIPA (0.57 mL, 3.3 mol) were dissolved in DMF (22 mL), and α-CD/PEG-bis Cys poly-pseudorotaxane (3.5 g) was added. After stirring at 4°C for 48 hours under nitrogen purge, the precipitate was collected by centrifugation and washed twice with methanol/DMF (1 : 1 v/v) and methanol, respectively. The resulting product was dissolved in DMSO and precipitated by adding excess acetone. The precipitate was washed three times with water, and the resulting precipitate was dried by lyophilization. Yield: 1.41 g, 51% (based on PEG).

(Production Example 5) Preparation of Ketal-PRX (Method A) Ketal-PRX was prepared according to Production Example 1 using PEG-bis ketal (20 kDa) (PEG-bis ket). To obtain PEG-bis ket, PEG (20 kDa) (2 g, 0.1 mmol) and CDI (72 mg, 0.44 mmol) were dissolved in THF (7.15 mL), and the mixture was stirred at 50°C for 18 hours under nitrogen purge. The reactant was added dropwise to ethylenediamine (0.5 mL, 3.14 mmol), and the mixture was stirred at 50°C for 2 hours. Ethanol (14.3 mL) was added, and the mixture was allowed to stand at -20°C for 2 hours. Thereafter, the precipitate was collected by centrifugation, washed several times with cold ethanol, and dried under reduced pressure. Yield: 1.7 g, 83.6%.

The α-CD/PEG-bis ket poly-pseudorotaxane was prepared according to the method described above using PEG-bis ket. The yield of the poly-pseudorotaxane was low.

(Production Example 6) Preparation of Ketal-PRX (Method B) Ketal-PRX was prepared according to Production Example 2 using 1-adamantane ketal (Ad-ket). To obtain Ad-ket, 1-adamantaneacetic acid (194 mg, 1.0 mmol), N-hydroxysuccinimide (230 mg, 2 mmol), and 1-(3-dimethylaminopropyl)-3-ethylcarbodiimide hydrochloride (382 mg, 2 mmol) were dissolved in dichloromethane (DCM) (10 mL), and the mixture was stirred at room temperature for 5 hours. The reactant was added dropwise to a solution containing 2,2-bis (aminoethoxy) propane (796.8 μL, 5 mmol) in DCM (10 mL), and the mixture was stirred overnight at room temperature. After addition of saturated aqueous NaCl, the organic layer was recovered. This extraction was repeated three times, and the resulting material was dried under reduced pressure. Yield: 308 mg, 91%.

To obtain ketal-PRX, Ad-ket (300 mg, 0.89 mmol), BOP reagent (393.1 mg, 0.89 mmol), and EDIPA (167 μL, 0.96 mmol) were dissolved in DMF (40.7 mL), and α-CD/PEG-COOH poly-pseudorotaxane (6.475 g) was added. After stirring at room temperature for 48 hours under nitrogen purge, the precipitate was collected by centrifugation and washed twice with methanol/DMF (1:1 v/v) and methanol, respectively. The resulting product was dissolved in DMSO and precipitated in excess water. The above procedure was repeated three times and the resulting precipitate was dried by lyophilization. Yield: 4.69 g, 89% (based on PEG).

(Production Example 7) Preparation of Hydrazone-PRX

The α-CD/PEG-bishydrazide (20 kDa) (PEG-bishydrazide) poly-pseudorotaxane was prepared according to the above using PEG-bishydrazide (Creative PEGWorks, Chapel Hill, USA). To obtain hydrazone-PRX, 1-adamantane carboxaldehyde (59.1 mg, 0.36 mmol) was dispersed in DMF (2.86 mL) and α-CD/PEG-bishydrazide poly-pseudorotaxane (400 mg) was added. After stirring at room temperature for 48 hours under nitrogen purge, the precipitate was collected by centrifugation and washed twice with methanol/DMF (1 : 1 v/v) and methanol, respectively. The resulting product was dissolved in DMSO and precipitated by adding excess acetone. The precipitate was washed twice with water, and the resulting precipitate was dried by lyophilization. Yield: < 5 mg, < 2% (based on PEG).

(Production Example 8) BAEE, DET, or DMAE Modification at Hydroxyl Group of α-CD in PRX

BAEE-PRX (Amino-PRX (1G)), DET-PRX (Amino-PRX (2G)), or DMAE-PRX was prepared by using CDI to activate the hydroxyl group of α-CD in each PRX prepared according to the above and adding the reactant to a solution of bisamine or amine at one end.

As a typical protocol, for the preparation of carbamate-DET-PRX, carbamate-PRX (100 mg, 1.25 μmol; α-CD:

76.8 μmol) and CDI (114 mg, 0.7 mmol) were dissolved in DMSO (3 mL), and the mixture was stirred overnight at room temperature under nitrogen purge. The reactant was added dropwise to DET (758.6 μL, 7.0 mmol), and the mixture was stirred overnight at room temperature under nitrogen purge.

For purification of carbamate-BAEE-PRX, carbamate-DET-PRX, carbamate-DMAE-PRX, or amide-DET-PRX, samples were each dialyzed against water (Spectra/Por™ membrane MWCO: 10 kDa) and dried by lyophilization. To purify the disulfide-DET-PRX, a sample was dialyzed against methanol (Spectra/Por™ membrane MWCO: 10 kDa), evaporated, dissolved in water, and dried by lyophilization. To purify ketal-DET-PRX, a sample was precipitated with excess cold ethanol, washed five times with ethanol, evaporated, dissolved in water, and dried by lyophilization. The yields of carbamate-BAEE-PRX, carbamate-DET-PRX, carbamate-DMAE-PRX, amide-DET-PRX, disulfide-DET-PRX, and ketal-DET-PRX were 133.0 mg (80%), 116.2 mg (84%), 92.5 mg (69%), 574 mg (85%), 110.6 mg (73%), and 27.7 mg (42%), respectively.

(Production Example 9) Preparation of Cys-PRX (Amino-PRX (4G))

Carbamate-PRX (100 mg, 1.25 μmol; α-CD: 76.8 μmol) and CDI (114 mg, 0.7 mmol) were dissolved in DMSO (3 mL), and the mixture was stirred for 24 hours at room temperature under nitrogen purge. Cystamine dihydrochloride (3.16 g, 14.0 mmol) was dissolved in DMSO (45 mL), and the mixture was stirred for 30 minutes in the presence of TEA (3.9 mL, 28.1 mol) for desalting. The reactant was added dropwise to desalted cystamine, and the mixture was stirred overnight at room temperature under a nitrogen atmosphere. For purification, a sample was precipitated with excess cold ethanol, washed five times with ethanol, evaporated, dissolved in water, and dried by lyophilization. Yield: 82.1 mg, 66%.

(Production Example 10) Preparation of Cys-DET-PRX (Amino-PRX (5G))

Carbamate-PRX (100 mg, 1.25 μmol; α-CD: 76.8 μmol) and CDI (114 mg, 0.7 mmol) were dissolved in DMSO (3 mL), and the mixture was stirred for 24 hours at room temperature under a nitrogen atmosphere. Cystamine dihydrochloride (1.58 g, 7.0 mmol) was dissolved in DMSO (22.5 mL), and the mixture was stirred for 30 minutes in the presence of TEA (2.0 mL, 14.0 mol) for desalting. Next, DET (758.0 μL, 7.0 mol) was added to the desalted cystamine solution. The reactant was added dropwise to a DET/desalted cystamine mixture, and the mixture was stirred overnight at room temperature under a nitrogen atmosphere. The sample was purified using the same protocol as for Cys-PRX described above. Yield: 98.2 mg, 76%.

(Production Example 11) Preparation of BAEE-DEX

Dextran 70 (7.56 g, 11 mmol) and CDI (10.07 g, 62 mmol) were dissolved in DMSO (250 mL), and the mixture was stirred overnight at room temperature under a nitrogen atmosphere. The reactant was added dropwise to 1,2-bis(2-aminoethoxy)ethane (92.1 mL, 630 mmol), and the mixture was stirred overnight at room temperature under a nitrogen atmosphere. After dialysis against water (Spectra/Por™ membrane MWCO: 10 kDa), the sample was dried by lyophilization.

(Production Example 12) Preparation of DET-DEX

In the preparation of DET-DEX, dextran 70 (0.5 g, 7.14 μmol) and CDI (1.33 g, 8.2 mmol) were dissolved in DMSO (15 mL), and the mixture was stirred overnight at room temperature under a nitrogen atmosphere. The reactant was added dropwise to DET (9.0 mL, 83.4 mmol), and the mixture was stirred overnight at room temperature under a nitrogen atmosphere. After dialysis against water (Spectra/Por™ membrane MWCO: 10 kDa), the sample was dried by lyophilization. Yield: 433.6 mg, 65%.

(Production Example 13) Preparation of Cas9 RNP Complex Using Each Cationic Material

Recombinant Cas9 protein (with the N-terminus modified by NLS) and sgRNA were mixed in a 1 : 1 molar ratio. The mixture in nuclease-free water (for evaluation of physical properties) or Opti-MEM (for cell experiments) (25 μL for 0.24 μg sgRNA) was incubated for 10 minutes at room temperature. Subsequently, the Cas9 RNP solution was added to the same volume of Hank's Balanced Salt Solution (HBSS) or Opti-MEM (containing various amounts of Amino-PRX, cationic polymer, or CRISPRMAX), and the mixture was stirred gently and incubated for 15 minutes at room temperature. The polymer content was set based on % of imprinting (927 amino groups/Cas9 RNP = 100%). Basically, each of the primary, secondary, or tertiary amino group was counted as 1 amine. However, a DET unit was recognized as 1 amine, and only the surface amine of the dendrimer was used for calculation. The CRISPRMAX/Cas9 RNP complex was

prepared according to the manufacturer's protocol strictly for mixing ratio, dilution time, incubation time, and others. Intracellular uptake and intracellular distribution tests were performed using a crRNA/ATTO-tracrRNA complex instead of sgRNA in accordance with exactly the same procedure.

(Test Example 1) How Protonation of DET-PRX Affects pH

The pD of DET-PRX dissolved in $D_2O$ was adjusted to neutral or acidic by adding DCI. The downshift of the proton of the methylene unit of DET was then observed by $^1$NMR. The pD was calculated by applying the value displayed on the pH meter to the following formula based on the past report (Liu, F. et al. Nat Cell Biol. 19, 1358-1370 (2017)). pD=pH+0.41

(Test Example 2) To Measure Size and ζ Potential of Cas9 RNP Polyplex

Cas9 RNP polyplex (sgRNA: 0.96 μg; total 200 μL) was diluted with 800 μL HBSS (pH 7.4). The size and ζ potential of the polyplex were determined by dynamic light scattering while using a Zetasizer Nano ZS instrument (Malvern Instruments, Worcestershire, UK).

(Test Example 3) Cryo-TEM Observation

The cryo-TEM measurement was performed with a JEM-2100F field emission TEM apparatus (JEOL Ltd.) at an acceleration voltage of 120 kV. For cryo-TEM, 2 μL of suspension prepared by including a highly concentrated Cas9 RNP complex (sgRNA: 2.85 μg; total 10 μL) was placed on a 200-mesh copper grid (Nisshin EM Co., Ltd., Tokyo, Japan) covered with a perforated carbon film. A thin water film (thickness about 100 nm) of the suspension was prepared by sucking up excess liquid. In addition, the film was rapidly vitrified by immersion in liquid ethane while using a Leica CPSC low-temperature preparation chamber (Leica Microsystems, Wetzlar, Germany). A grid with the vitrified thin film was set in a sample holder, which was transferred to a microscope chamber. Liquid nitrogen was used to maintain the sample temperature at less than -170°C.

(Test Example 4) Heparin Competition Assay

Cas9 RNP polyplex (sgRNA: 0.2 μg; total 7 μL) was mixed with 1 μL of heparin sodium salt (Nacalai Tesque, Kyoto, Japan) solution at each concentration (0, 0.25, 0.5, 0.75, or 1 mg/mL), and the mixture was incubated for 10 minutes at room temperature. After 2 μL of 6 × loading buffer (Takara Bio, Shiga, Japan) was added to the mixture, gel electrophoresis was performed at room temperature at 100 V for 40 minutes on 2% (w/v) agarose S gel in Tris-borate EDTA buffer (45 mM Tris-borate, 1 mM EDTA, pH 8.0 (0.5 × TBE)). The gel was stained with ethidium bromide. The sgRNA and Cas9 RNP bands were visualized using an Amersham Typhoon scanner (FLA-9000, Fujifilm, Tokyo, Japan). The percentage (%) of residual polyplex was quantified by ImageJ software (National Institutes of Health, Bethesda, MD, USA) based on the relative band intensities of the polyplex and the released Cas9 RNP.

(Test Example 5) Intracellular Uptake of Cas9 RNP Complexed with Each Cationic Material

HeLa cells ($3.75 \times 10^4$ cells/well) were seeded on a 24-well plate 24 hours prior to transfection and washed twice with serum-free culture medium. Next, 500 μL of Cas9 RNP complex (crRNA/ATTO tracrRNA, non-targeting) diluted to a final concentration of 14.6 nM in DMEM was added to each well, and the mixture was incubated at 37°C for 4 hours. The cells were washed twice with HBSS, detached by a trypsin-EDTA method, recovered by centrifugation, dispersed in 500 μL of HBSS containing 10% FBS, and filtered through a nylon mesh. Data from $1 \times 10^4$ cells was analyzed using a BD Accuri C6 flow cytometer (BD Bioscience Japan, Tokyo, Japan) and BD Accuri C6 software (BD Bioscience Japan, Tokyo, Japan).

(Test Example 6) Cell Viability

HeLa cells ($1.5 \times 10^4$ cells/well) were seeded on a 96-well plate 24 hours prior to transfection and washed twice with serum-free culture medium. Next, 200 μL of Cas9 RNP complex (sgCont) diluted to a final concentration of from 14.6 nM to 58.4 nM in DMEM was added to each well, and the mixture was incubated at 37°C for 4 hours. The cells were washed twice with DMEM, 200 μL of DMEM (10% FBS) was added to each well, and the mixture was incubated at 37°C for 20 hours. After washing twice with HBSS, 100 μL of fresh HBSS and 10 μL of Cell Counting Kit-8 (Dojindo, Kumamoto, Japan) were added to each well and the mixture was incubated at 37°C for 1 hour. The absorbance (450 nm (sample) and 655 nm (reference)) of the solution was measured with an Epoch microplate reader (Bio Tek Instruments,

Winooski, VT, USA).

For determination of cell viability at pH 5.5 to study endosomal membrane disruption, Cas9 RNP polyplex diluted in normal DMEM (pH 7.4) or acidic DMEM (pH 5.5) adjusted with HCl was used and other procedures were performed in the same way described above.

(Test Example 7) Genome Editing Activity of Each Cas9 RNP Complex in HeLa/GFP Cells

HeLa/GFP cells (3.7.5 $\times$ $10^4$ cells/well) were seeded on a 24-well plate 24 hours prior to transfection and washed twice with serum-free culture medium. Next, 500 μL of Cas9 RNP complex (sgCont (sgRNA not targeting GFP) or sgGFP) diluted to a final concentration of from 29.2 to 116.8 nM in DMEM was added to each well, and the mixture was incubated at 37°C for 4 hours. The cells were washed twice with DMEM, 500 μL of DMEM (10% FBS) was added to each well, and the mixture was cultured for 5 days. The cells were washed twice with HBSS, detached by pipetting in HBSS, recovered by centrifugation, dispersed in 1 mL of 10% FBS-containing HBSS, and filtered through a nylon mesh. Flow cytometry analysis was performed by the procedure described above. GFP knockout (%) was calculated by the following equation:

$$\text{GFP knockout (\%)} = 100 - 100 \times (\text{GFP positive cells in the sample/GFP positive cells in the untreated control group}).$$

(Test Example 8) Genome Editing Activity of Amino-PRX (5G)/Cas9 RNP Polyplex in Presence of GSH

Amino-PRX (5G)/Cas9 RNP polyplex was diluted in DMEM with a varied concentration of GSH (0 to 10 mM) and genome editing activity was measured according to the procedure described above.

(Test Example 9) T7E1 Assay

Genomic DNA of cells treated with Cas9 RNP complex was extracted using a MightyPrep reagent for DNA (Takera Bio, Shiga, Japan). The GFP target sequence was amplified using MightyAmp DNA polymerase Ver. 3 (Takara Bio, Shiga, Japan). The resulting PCR amplicon was purified using NucleoSpin gel and PCR Clean-up (Takara Bio, Shiga, Japan). The purified amplicon was denatured, re-annealed, and digested with T7E1 by using a T7 endonuclease I assay kit (GeneCopoeia, Inc., Rockville, MD, USA.). Gel electrophoresis was performed at room temperature and using 0.5 $\times$ TBE and 4% (w/v) agarose 21 gel under conditions of 100 V for 40 minutes. The bands were stained and visualized in the same manner as previously described.

(Test Example 10) Particle Response of Amino-PRX/Cas9 RNP Polyplex to Various Stimuli

Cas9 RNP polyplex (sgRNA: 0.96 g; total 200 μL) was diluted in 800 μL of HBSS (pH 7.4: normal), acetate buffer (pH 5.5: endosomal environment), or HBSS (GSH: 2 mM: reducing environment in the cytosol). The size and ζ potential of the polyplex were determined according to the procedure described above.

(Test Example 11) Cellular Distribution of Each Amino-PRX/Cas9 RNP Polyplex

HeLa cells (1.0 $\times$ $10^4$ cells/dish) were seeded on a 35-mm glass bottom dish 24 hours prior to transfection and washed twice with serum-free culture medium. Next, 200 μL of Cas9 RNP complex (crRNA/ATTO tracrRNA, non-targeting) diluted to a final concentration of 14.6 nM in DMEM was added to each dish, and the mixture was incubated at 37°C for 4 hours (4-h sample). For some samples, the cells were then washed twice with DMEM and 2 mL of DMEM was added to each dish, followed by additional 4-h (8-h sample) or 8-h (12-h sample) incubation. The cells were then washed twice with HBSS, fixed with 4% paraformaldehyde solution for 10 minutes at room temperature, and stained with 2 μg/mL Hoechst 33342 (Thermo Fisher Scientific K.K., Tokyo, Japan) for 10 minutes at room temperature. Confocal laser scanning microscopy was performed using Leica TCS SP5 (Leica Japan, Tokyo, Japan). The fluorescence intensity/nucleus area of each obtained image was quantified by ImageJ software.

(Example 1) Automatic Molecular Imprinting of Cas9 RNP by Using Amino-PRX (BAEE-PRX)

Since Cas9 RNP has zwitterions and a three-dimensional charge distribution (FIG. 1A), each conventional cationic polymer cannot form a Cas9 RNP polyplex. It was examined whether BAEE-PRX efficiently formed a polyplex

with Cas9 RNP through the movement and rotation effect of amino group-modified α-CDs, like molecular imprinting, just by mixing, that is, whether BAEE-PRX acted as an automatic molecular imprinting agent for Cas9 RNP.

PRX having a PEG (20 kDa) backbone threaded with α-CDs and capped with adamantane (Ad) was synthesized. Subsequently, the hydroxyl group of α-CD of PRX was modified with 1,2-bis(2-aminoethoxy)ethane (BAEE) to obtain primary amino group-modified PRX (Amino-PRX) (FIG. 1C). As a control polymer, BAEE-dextran (BAEE-DEX), a glucose-based polymer, was prepared with almost the same BAEE modification rate and molecular weight.

The efficiency of complexing BAEE-PRX or BAEE-DEX with Cas9 RNP was evaluated based on their ζ potential (FIG. 2). According to previously reported NC, 927 cationic monomers were required for molecular imprinting of one Cas9 RNP molecule (Chen, G. et al. Nat Nanotechnol 14,974-980 (2019)). Therefore, as an index of complexation, this amino group mixing ratio was set as an amount that gave 100% imprinting. When BAEE-PRX was added, the ζ potential of Cas9 RNP was immediately reversed and reached a plateau at the amino group amount corresponding to 10% imprinting. On the other hand, the reversal of the ζ potential when BAEE-DEX was added was slower than that of BAEE-PRX, and sufficient complexation was obtained only when Amino-DEX was added in an amino group amount corresponding to 100% imprinting.

Next, the proportion of amino groups at the maximum intracellular uptake of the BAEE-PRX/Cas9 RNP polyplex was examined. It has been demonstrated that 50% imprinting resulted in maximum intracellular uptake (FIG. 3).

Further, cryo-TEM was used to observe a BAEE-PRX/Cas9 RNP polyplex prepared using the mixing ratio at which the maximum cellular uptake (50% imprinting) was achieved (FIGS. 4A to 4C). The Cas9 RNP exhibited a crystalline form like aggregated small crystallites (FIG. 4A), whereas the BAEE-PRX/Cas9 RNP polyplex was low contrast particles with a size of around 100 to 200 nm unlike Cas9 RNP crystals (FIG. 4B). The low contrast of the BAEE-PRX/Cas9 RNP particles indicates that the degree of crystallization of the Cas9 RNP was low. This has suggested that there is a relatively strong molecular level interaction between Cas9 RNP and BAEE-PRX. The image of the BAEE-DEX/Cas9 RNP polyplex (FIG. 4C) had little change when compared with the case of the Cas9 RNP alone, suggesting that non-interacting Cas9 RNP might remain as it was.

These results have strongly supported that because of movable macrocyclic molecules, BAEE-PRX can present interacting groups to Cas9 RNP more efficiently than conventional less-movable polymers, so that a polyplex is formed more efficiently.

Further, the stability of BAEE-PRX/Cas9 RNP and the stability of BAEE-DEX/Cas9 RNP in the presence of heparin (negatively charged substance), which electrostatically breaks the polyplex, were compared. Thus, which carrier more strongly interacts with Cas9 RNP and stabilizes the polyplex was examined by heparin competition assay. The results of the heparin competition assay have revealed that the stability of the Cas9 RNP polyplex was higher in the polyplex with BAEE-PRX than in the polyplex with BAEE-DEX (FIG. 5A and FIG. 5B). This has suggested that the formation of Cas9 RNP polyplex by BAEE-PRX was not only more efficient but also more potent than the case of conventional polymers.

Furthermore, the uptake of Cas9 RNP polyplex into HeLa cells by BAEE-PRX was higher than that by BAEE-DEX, a total of eight different cationic polymers exhibiting various structures/molecular weights/charge distributions, or Lipofectamine™ or CRISPRMAX™, each of which is widely used as a reagent for delivering Cas9 RNP, under conditions where the safety can be guaranteed (FIGS. 6A to 6C).

These results have suggested that BAEE-PRX causes highly efficient and potent complexation by simply mixing with Cas9 RNP due to supramolecular conversion, and is thus useful as a carrier having better intracellular introduction efficiency than conventional polymers. In particular, utility as PRX-based automatic molecular imprinting for Cas9 RNP was also demonstrated.

(Example 2) Collaborative Endosomal Disruption Effect of Cas9 RNP Polyplex as Exerted by Dynamic Properties of Diethylenetriamine (DET) and PRX

Cas9 RNP should be intracellularly taken up and then released from the endosome. This is important so as to avoid digestion by lysosomes and achieve efficient genome editing in the case of Cas9 RNP as well as in the case of gene/nucleic acid. Hence, the modifying amino group of PRX that improves the endosome escape potential of Amino-PRX was examined. Until now, studies on optimization of the amino group of PRX have not been conducted not only for Cas9 RNP introduction but also for gene/nucleic acid introduction. In addition to BAEE-PRX having a primary amino group and used as BAEE-PRX, PRX modified with DET (Miyata, K. et al., Journal of the American Chemical Society 130, 16287-16294 (2008)), which has been reported as an endosome-disrupting amino unit, or 2-(dimethylamino)ethylamine (DMAE) (Ooya, T. et al. (2006), supra; Emami, M.R. et al. (2019), supra; Tamura, A. et al., Biomaterials 34, 2480-2491 (2013); Tamura, A. et al., Sci Rep 3, 2252 (2013); Tamura, A. et al. (2015), supra), which is a tertiary amine and often reported as a PRX-modifying amine, at the same modification ratio were produced as DET-PRX or DMAE-PRX, respectively (Table 2). In Table 2, "Number (CyD)" indicates the number of amino groups modified per PRX molecule. In particular, the DET unit has two cation charge states: mono-protonated (the safe form) at neutral pH and

di-protonated (the membrane disrupting form) under acidic conditions. Thus, it has been reported that as a result, membrane (cell/organelle) damage does not occur when the DET unit is present extracellularly or in the cytosol, but membrane damage occurs in an acidic environment (such as late endosome), thereby facilitating the release of contents from the membrane (Miyata, K. et al. (2008), supra). NMR spectra in the acidic, neutral, or basic solution were measured. Then, the two-step protonation of DET-PRX was found (not shown).

[Table 2]

| Name | BAEE-PRX | DET-PRX | DMAE-PRX |
|---|---|---|---|
| Modified amine | 1,2-Bis(2-aminothoxy)ethane | Diethylenetriamine | 2-(Dimethylamino) ethylamine |
| R = | 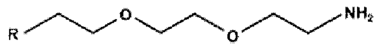 | 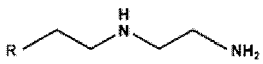 | 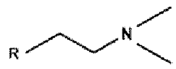 |
| Number (CyD) | 208 (3.5) | 235 (3.8) | 235 (3.8) |
| Mw (kDa) | 114 | 110 | 107 |

To evaluate the endosomal membrane disruption potential of Cas9 RNP polyplex with each amino-modified PRX, cytotoxicity assays were performed assuming the cell membrane at pH 5.5 as an endosomal membrane (FIG. 7). At neutral pH, DET-PRX polyplex and DMAE-PRX polyplex showed lower cytotoxicity than BAEE-PRX polyplex. In the endosomal environment (pH 5.5), DET-PRX polyplex exhibited a stronger cell membrane disruption potential than BAEE-PRX polyplex or DMAE-PRX polyplex due to enhanced $\zeta$ potential. It was suggested that DET-PRX induced endosomal escape of Cas9 RNP most efficiently among the three Amino-PRXs.

The efficiency of cellular uptake of polyplex was the best for DMAE-PRX/Cas9 RNP polyplex and the lowest for DET-PRX/Cas9 RNP polyplex (FIG. 8). Nevertheless, the genome editing activity of DET-PRX/Cas9 RNP polyplex was higher than that of BAEE-PRX/Cas9 RNP polyplex or DMAE-PRX/Cas9 RNP polyplex (FIGS. 9A and 9B). This has strongly suggested the importance of intracellular dynamics (in particular, endosome escape) along with the improved effects of DET-PRX.

Further, under endosomal conditions, the membrane disruption activity of Cas9 RNP was compared using DET-PRX or DET-DEX prepared to have almost the same DET modification rate and molecular weight (FIG. 10). The endosomal disruption activity of DET-PRX polyplex was slightly higher than that of DET-DEX polyplex, suggesting a coordinated effect of dynamic properties of DET and PRX on endosomes and escape of Cas9 RNP, and the importance of the PRX backbone. In this way, in DET-PRX, CDs rotate in the endosomal environment. This can eliminate a three-dimensional structural mismatch between the endosomal membrane and the DET units. Thus, it has been found that through efficient interaction, a higher membrane disruption effect is obtained than in the case of a typical polymer modified with DET. DET-PRX has been successfully developed that promotes escape from the endosome due to the second stage of molecular structure conversion in addition to automatic molecular imprinting (FIG. 11).

(Example 3) Imparting Intracellular Degradability of Amino-PRX for Efficient Release of Cas9 RNP

When Cas9 RNP is released from Amino-PRX, nuclear translocation of Cas9 RNP is promoted by a nuclear localization signal. Genome editing efficiency is then enhanced. So far, it has been reported that PRX, in which an intracellularly cleavable linker (endcap linker) is introduced into the bond between the endcap and the axial molecule, intracellularly releases the loaded component when the linker is cleaved in the cell (Ooya, T. et al. (2006), supra; Tamura, A. et al. (2013), supra; Tamura, A. et al. (2013), supra; Tamura, A. et al. (2015), supra). However, optimization studies of endcap linker fit for each molecule have not been performed so far, and there has been no report on Cas9 RNP. Thus, an attempt was made to prepare DET-PRX containing one of six types of endcap linker: amide, carbamate (DET-PRX in Example 2), disulfide, ketal, ester, or hydrazone by changing the functional group at the PEG terminal (FIGS. 12A and 12B). The 2-methyl group at the PEG terminus inhibits the threading of $\alpha$-CDs due to steric hindrance, resulting in a low yield of poly-pseudorotaxane. Therefore, exceptionally, ketal-PRX was produced by introducing a ketal linker on the adamantane (endcap) side. The hydrazone-PRX yield was low because adamantane aldehyde was hard to dissolve in DMF, and modification of ester-PRX with DET was impossible due to unavoidable degradation in the reaction with slightly hygroscopic and basic DET.

The genome editing efficiency of each of the finally obtained Cas9 RNP polyplexes with 4 different Amino-PRXs (carbamate-DET-PRX, ketal-DET-PRX, amide-DET-PRX, or disulfide-DET-PEX) at low concentrations was evaluated (FIG. 13). All the Amino-PRXs exerted a genome editing effect, while in particular, carbamate-DET-PEX, ketal-DET-PRX, and amide-DET-PRX exhibited comparable genome editing efficiency. Biocompatibility was considered to select, as the optimal endcap linker for Amino-PRX, a carbamate linker that could be hydrolyzed in the long term.

As another strategy to release the molecule loaded on PRX, an intracellularly cleavable linker (brush linker) was introduced into the bond between CD and the amino group. As a result, intracellular stimulation should cleave the linker to decrease the number of amino groups bonded to CD and weaken the interaction between CD and Cas RNP. The drug release function of biodegradable PRX was considered to be more dramatic in the endcap type than in the brush type. The disulfide bond was thus selected as a brush linker. Cys-PRX containing a primary amine via a disulfide bond was newly synthesized by modifying the hydroxyl group of α-CD with cystamine (Cys) (FIGS. 14A and FIG. 14B). Similar to Example 2, Cys-PRX, a primary amine, had a low capacity to release Cas9 RNP from the endosome and exhibited no genome editing at low concentrations (not shown). Therefore, a strategy of mixing Cys and DET was attempted.

Genome editing activity of Cas9 RNP polyplex was examined using a 1 : 1 mixture of Cys-PRX and DET-PRX (DET-PRX/Cys-PRX) or Amino-PRX (Cys-DET-PRX) with Cys and DET modifications on the same PRX (FIGS. 15A and 15B). Any of them exerted a genome editing effect, but significant improvement was found in Cys-DET-PRX. This effect has suggested that both the release from the endosome by DET and the release of Cas9 RNP from the carrier by the intracellular degradation of Cys to halve the interacting functional groups were achieved.

The above experimental results have demonstrated that not only intracellular uptake but also control of intracellular dynamics after intracellular uptake is a factor that determines the genome editing efficiency. In addition, the genome editing activity of Cys-DET-PRX pre-incubated with GSH was not lowered by GSH at an extracellular concentration (0.02 mM or lower), but was lowered by GSH at an intracellular concentration (2 to 10 mM) (FIGS. 16A and 16B). This has revealed that Cys-DET-PRX has a good balance between stability and stimulation responsiveness, and causes polyplex disruption only in an intracellular reducing environment.

Also, the Cys-DET-PRX/Cas9 RNP polyplex was able to induce efficient genome editing equivalent to that of the CRISPRMAX/Cas9 RNP complex (FIG. 17). Further, Cys-DET-PRX is safer than CRISPRMAX, and can thus be processed at a higher concentration. Therefore, it has become clear that more efficient gene delivery can be achieved by adopting a high concentration at which CRISPRMAX cannot be used for induction (FIG. 17).

(Example 4) Efficient Nuclear Translocation of Cas9 RNP and Genome Editing through Five Different Form Changes in Cys-DET-PRX

It was considered that efficient genome editing by Cys-DET-PRX was caused by complexation with Cas9 RNP by automatic imprinting, membrane disruption by endosomal protonation and α-CD rotation, degradation of brush linker, release of Cas9 RNP in the cytosol, and nuclear translocation of Cas9 RNP (FIG. 18). This should be proved by evaluating the physical properties of Cys-DET-PRX/Cas9 RNP polyplex under various stimulation environments (FIGS. 19A to 19C). Cys-DET-PRX polyplex had increased ζ potential in the endosomal environment and their particles were disrupted under treatment of GSH at an intracellular concentration. Further, a carbamate bond was used as an endcap linker. This allows long-term degradation, and enables excellent biocompatibility. Cys-DET-PRX can deliver Cas9 RNP very efficiently, conveniently, and safely due to five different form changes: (1) pre-complexation, (2) polyplex (extracellular/cytosol), (3) highly charged polyplex (endosome), (4) destabilized polyplex (GSH stimulation), and (5) monomeric PRX of Cys-DET-PRX (long term degradation). Indeed, the nuclear translocation potential of Cas9 RNP polyplex introduced using Cys-DET-PRX was very high due to endosome escape capability and releasability of Cas9 RNP in the cytosol. These results suggest that Cys-DET-PRX achieves highly efficient Cas9 RNP delivery through 5-step changes.

(Example 5) RNAi effect of Cys-DET-PRX/siRNA

HeLa/GFP cells were seeded on a 24-well plate and incubated at 37°C for 24 hours. Next, 500 μl of serum-free culture medium containing one of the following samples was added to the cells after washed twice with serum-free culture medium.

(Sample)

siRNA alone (25 nM, 50 nM, 100 nM)
Lipofectamine™2000 / siRNA (25 nM, 50 nM, 100 nM)
Amino-PRX (2G) / siRNA (25 nM, 50 nM, 100 nM)
Amino-PRX (5G) / siRNA (25 nM, 50 nM, 100 nM)

After incubation at 37°C for 4 hours in the presence of each sample, the cells were washed twice with serum-free culture medium, 500 μl of DMEM containing 10% FBS was added, and the cells were then further cultured for 68 hours to evaluate RNAi.

The results are shown in FIG. 20. Amino-PRX (2G)/siRNA polyplex showed RNAi effects comparable to Lipofectamine™ 2000/siRNA, whereas Amino-PRX (5G)/siRNA (25 nM, 50 nM, or 100 nM) polyplex exhibited higher RNAi effects than those. This has suggested that Amino-PRX (2G) and Amino-PRX (5G) are excellent siRNA carriers, and particularly Amino-PRX (5G) is an innovative carrier having a higher siRNA introduction effect than Lipofectamine™ 2000, which is a commercially available reagent for nucleic acid introduction.

(Example 6) Genome Editing by Cys-DET-PRX/Cas9 RNP in vivo HeLa/GFP cells ($1 \times 10^6$ cells, 100 mL) were transplanted into the left hind paw of each Balb/c nu/nu (♂, 4-w old) mouse. The following samples were each directly administered (250 pmol, 200 mL) to the tumor of each mouse (about 5 days after transplantation) having a tumor major diameter of 5 mm.

(Sample)

HBSS (Control)
Cas9 / sgGFP
Amino-PRX (5G) / Cas9 RNP (sgCont)
Amino-PRX (5G) / Cas9 RNP (sgGFP)
CRISPRMAX / Cas9 RNP (sgCont)
CRISPRMAX / Cas9 RNP (sgGFP)

After 5 days of rearing, each mouse was refluxed with PBS, and the tumor was collected. Here, 1 mL of RIPA buffer was added to 100 mg of tumor for homogenization. After centrifugation (r.t., 5000 rpm, 5 min), the supernatant was recovered. This operation (centrifugation and recovery) was repeated twice, and the fluorescence intensity of the obtained supernatant was measured with a fluorescence plate reader.

The results are shown in FIG. 21. CRISPRMAX/Cas9 RNP was unable to induce a genome editing effect in vivo, whereas Amino-PRX (5G)/Cas9 RNP was able to induce genome editing. This has suggested that Amino-PRX (5G) may provide an excellent Cas9 RNP capable of inducing in vivo genome editing that cannot be induced by a commercially available delivery reagent.

(Example 7) To Optimize Modifying Amino Group in Nucleic Acid Delivery

In order to optimize the modifying amino group of PRX in nucleic acid delivery, the RNAi effect was examined when a complex of siRNA and one of three types of Amino-PRX was introduced into cells while using siRNA as a model nucleic acid. The three types of Amino-PRX used included BAEE-PRX, DET-PRX, and DMAE-PRX prepared by substantially the same method as in Example 2. Meanwhile, the following siGFP was used as the siRNA. (siGFP) sence: 5'- GCAAGCUGACCCUGAAGUUCAU dTdT -3' (SEQ ID NO: 6) antisence: 5'- AUGAACUUCAGGGUCAGCUUGCCG -3' (SEQ ID NO: 7)

HeLa/GFP cells were seeded on a 24-well plate ($3.75 \times 10^4$ cells/well) and incubated at 37°C for 24 hours. Next, 300 μL of serum-free culture medium containing one of the following samples was added to the cells after washed twice with serum-free culture medium.

(Sample)

siRNA alone (0 nM, 25 nM, 50 nM, 100 nM)
BAEE-PRX / siRNA (0 nM, 25 nM, 50 nM,100 nM)
DET-PRX / siRNA (0 nM, 25 nM, 50 nM, 100 nM)
DMAE-PRX / siRNA (0nM, 25nM, 50nM, 100nM)

After incubation at 37°C for 4 hours in the presence of the sample, the cells were washed twice with serum-free culture medium. After 500 μL of DMEM containing 10% FBS was added, the cells were incubated at 37°C for 68 hours. Thereafter, the RNAi effect was evaluated by flow cytometry.

The results are shown in FIG. 22. Among BAEE-PRX/siRNA, DET-PRX/siRNA, and DMAE-PRX/siRNA, DET-PRX/siRNA showed the highest RNAi effect. This has suggested that DET, which exerts endosome escape capability, is an optimal modifying amino group even in conventional nucleic acid delivery, like in the case of Cas9 RNP.

(Example 8) To Optimize Endcap-Axial Molecule Bond in Nucleic Acid Delivery

The endcap-axial molecule bond was optimized in nucleic acid delivery.

For this purpose, the RNAi effect was examined using siRNA as a model nucleic acid when a complex of siRNA and DET-PRX having one of four types of endcap-axial molecule bond (amide-DET-PRX, carbamate-DET-PRX, disulfide-DET-PRX, or ketal-DET-PRX) was introduced into cells.

HeLa/GFP cells were seeded on a 24-well plate (3.75 × $10^4$ cells/well) and incubated at 37°C for 24 hours. Next, 300 μL of serum-free culture medium containing one of the following samples was added to the cells after washed twice with serum-free culture medium.

(Sample)

siRNA alone (0 nM, 25 nM, 50 nM, 100 nM)
Amide -DET-PRX / siRNA (0 nM, 25 nM, 50 nM, 100 nM)
Carbamate -DET-PRX / siRNA (0 nM, 25 nM, 50 nM, 100 nM)
Disulfide -DET-PRX / siRNA (0 nM, 25 nM, 50 nM, 100 nM)
Ketal -DET-PRX / siRNA (0 nM, 25 nM, 50 nM, 100 nM)

After incubation at 37°C for 4 hours in the presence of the sample, the cells were washed twice with serum-free culture medium. After 500 μL of DMEM containing 10% FBS was added, the cells were incubated at 37°C for 68 hours. Flow cytometry was used to evaluate the RNAi.

The results are shown in FIG. 23. Among four different complexes of siRNA and DET-PRX having an endcap-axial molecule bond, Carbamate-DET-PRX/siRNA showed the highest RNAi effect. It has been suggested that in delivery of a conventional nucleic acid, DET-PRX (Carbamate-DET-PRX) having a carbamate bond, which may be hydrolyzed long-term, between the endcap and the axial molecule has better nucleic acid delivery potential than DET-PRX (Amide-DET-PRX) having an amide bond. This has suggested that the intracellular enzymatic degradation of carbamate bond may be important not only from the viewpoint of biocompatibility but also from the viewpoint of release of the loaded molecule.

(Example 9) Cytotoxicity of Amino-PRX (5G)/siRNA

In order to compare the cytotoxicity of Amino-PRX (5G)/siRNA with that of Lipofectamine™ 2000/siRNA, the following test was performed.

HeLa cells were seeded on a 96-well plate (1.5 × $10^4$ cells/well), and incubated for 24 hours under conditions at 37°C and 5% $CO_2$. Next, 200 μL of serum-free culture medium containing one of the following samples was added to the cells after washed twice with serum-free culture medium.

(Sample)

Amino-PRX (5G) / siRNA (0 nM, 25 nM, 50 nM, 100 nM, 150 nM, 200 nM, 300 nM, 400 nM)
Lipofectamine™2000 / siRNA (0 nM, 25 nM, 50 nM, 100 nM, 150 nM, 200 nM, 300 nM, 400 nM)

After incubation under conditions at 37°C and 5% $CO_2$ for 4 hours in the presence of the sample, the cells were washed twice with serum-free culture medium. Next, 200 μL of 10% FBS-containing culture medium was added, and the mixture was then incubated for 20 hours under conditions at 37°C and 5% $CO_2$. After washing with HBSS twice, 100 μL of HBSS and 10 μL of WST-8 (Dojindo, Kumamoto, Japan) were mixed. Subsequently, 110 μL of the HBSS/WST-8 mixture was added to each well, and the resulting mixture was then incubated at 37°C for 1 hour. Absorbance was measured with a Micro plate reader (measurement wavelength: 450 nm; reference wavelength: 655 nm).

The results are shown in FIG. 24. Amino-PRX (5G)/siRNA showed significantly lower cytotoxicity than Lipofectamine™ 2000/siRNA. This has suggested that Amino-PRX (5G) is safer than Lipofectamine™ 2000, which is a commercially available reagent for nucleic acid transfection, at the time of siRNA delivery.

(Example 10) GFP Knockdown Effect of Amino-PRX (5G)/ASO

In order to evaluate the nucleic acid delivery potential of Amino-PRX (5G) by using an antisense oligonucleotide (ASO) as a model nucleic acid, the following test was performed.

HeLa/GFP cells were seeded on a 24-well plate (3.75 × $10^4$ cells/well) and incubated at 37°C for 24 hours. Next, 300 μL of serum-free culture medium containing one of the following samples was added to the cells after washed twice with serum-free culture medium. As ASO for GFP knockdown, GFP ASO (TTGCCGGTGGTGCAGATAAA (SEQ ID NO: 8)) was used.

(Sample)

ASO alone (50 nM, 100 nM, 200 nM)
Lipofectamine™2000 / ASO (50 nM, 100 nM, 200 nM)
Amino-PRX (5G) / ASO (50 nM, 100 nM, 200 nM)

After incubation at 37°C for 4 hours in the presence of the sample, the cells were washed twice with serum-free culture medium. After 500 μL of DMEM containing 10% FBS was added, the cells were incubated at 37°C for 68 hours. Flow cytometry was used to evaluate the gene knockdown effect.

The results are shown in FIG. 25. Amino-PRX (5G)/ASO had a significantly higher GFP knockdown effect than Lipofectamine™ 2000/ASO. This has suggested that Amino-PRX (5G) is also useful in ASO delivery.

(Example 11) Cytotoxicity of Amino-PRX (5G)/ASO

In order to compare the cytotoxicity of Amino-PRX (5G)/ASO with that of Lipofectamine™ 2000/siRNA, the following test was performed.

HeLa cells were seeded on a 96-well plate (1.5 × $10^4$ cells/well), and incubated for 24 hours under conditions at 37°C and 5% $CO_2$. Next, 200 μL of serum-free culture medium containing one of the following samples was added to the cells after washed twice with serum-free culture medium.

(Sample)

Amino-PRX (5G) / ASO (0 nM, 50 nM, 100 nM, 200 nM, 300 nM, 400 nM)
Lipofectamine™2000 / ASO (0 nM, 50 nM, 100 nM, 200 nM, 300 nM, 400 nM)

After incubation under conditions at 37°C and 5% $CO_2$ for 4 hours in the presence of the sample, the cells were washed twice with serum-free culture medium. Next, 200 μL of 10% FBS-containing culture medium was added, and the mixture was then incubated for 20 hours under conditions at 37°C and 5% $CO_2$. After washing with HBSS twice, 100 μL of HBSS and 10 μL of WST-1 (Dojindo, Kumamoto, Japan) were mixed. Subsequently, 110 μL of the HBSS/WST-1 mixture was added to each well, and the resulting mixture was then incubated at 37°C for 1 hour. Absorbance was measured with a Micro plate reader (measurement wavelength: 450 nm; reference wavelength: 655 nm).

The results are shown in FIG. 26. Amino-PRX (5G)/ASO showed significantly lower cytotoxicity than Lipofectamine™ 2000/ASO. This has suggested that Amino-PRX (5G) is safer than Lipofectamine™ 2000, which is a commercially available reagent for nucleic acid transfection, at the time of ASO delivery.

(Example 12) mRNA Knockdown Effect of Amino-PRX (5G)/Gapmer-Type ASO

In order to evaluate the nucleic acid delivery potential of Amino-PRX (5G) by using an antisense oligonucleotide (gapmer-type ASO) as a model nucleic acid, the following test was performed.

HeLa/GFP cells were seeded on a 24-well plate (3.75 × $10^4$ cells/well) and incubated at 37°C for 24 hours. Next, 300 μL of serum-free culture medium containing one of the following samples was added to the cells after washed twice with serum-free culture medium. As gapmer-type ASO for GFP knockdown, GFP ASO gapmer (<u>GAA</u>CTTCAGGGTC<u>AGC</u> (SEQ ID NO: 9); phosphorothioate modification (S-modification) was added between all the nucleotides; LNAs are underlined) was used.

(Sample)

HBSS (control)
Gapmer-type ASO alone (100 nM)
Amino-PRX (5G) NP2/gapmer-type ASO (100 nM)
Amino-PRX (5G) NP5/gapmer-type ASO (100 nM)
Amino-PRX (5G) NP10/gapmer-type ASO (100 nM)
Note that the NP of NP2, NP5, or NP10 indicates the ratio between the amino group (plus charge) of Amino-PRX (5G) and the phosphate group (minus charge) of gapmer-type ASO.

After incubation at 37°C for 4 hours in the presence of the sample, the cells were washed twice with serum-free culture medium. After 500 μL of DMEM containing 10% FBS was added, the cells were incubated at 37°C for 44 hours. Flow cytometry was used to evaluate the gene knockdown effect.

The results are shown in FIG. 27. When the gapmer-type ASO was introduced into cells by using Amino-PRX (5G), the GFP knockdown effect was enhanced. This has suggested that Amino-PRX (5G) may also be useful in gapmer-type ASO delivery.

(Example 13) Acidic Protein Delivery Potential of Amino-PRX (5G)

The acidic protein delivery potential of Amino-PRX (5G) was evaluated using β-galactosidase (β-gal) as an acidic model protein. Specifically, a complex of Amino-PRX (5G) and β-gal was introduced into a cell, and whether or not endosome escape and intracellular release of β-gal were possible was examined by measuring intracellular β-gal enzyme activity.

The intracellular β-gal enzyme activity was measured using SPiDER-β-Gal (Dojindo, Kumamoto, Japan), which is an intracellular β-gal activity detection kit. SPiDER-β-Gal permeates through the cell membrane, is cleaved by intracellular β-gal, and then binds to intracellular proteins to emit fluorescence, so that only the enzyme activity of β-gal present in the cell can be specifically detected.

HeLa cells were seeded ($1.5 \times 10^4$ cells/well) on a 35-mm glass base dish, and incubated at 37°C for 24 hours. Next, 200 mL of Amino-PRX (5G)/β-gal (β-gal: 25 nM) complex solution was added to the cells after washed twice with serum-free culture medium. After incubation under conditions at 37°C and 5% $CO_2$ for 4 hours, the cells were washed twice with serum-free culture medium. Subsequently, 200 mL of SPiDER-β-Gal was added thereto, and the mixture was incubated at 37°C for 15 minutes. The cells were fixed by treatment with 4% paraformaldehyde for 10 minutes, and the nuclei were stained using Hoechst 33342. Thereafter, fluorescence derived from SPiDER-β-Gal was observed under a fluorescence microscope (excitation wavelength: 480 nm; fluorescence wavelength: 530 nm).

The results are shown in FIG. 28. β-gal introduced into cells by using Amino-PRX (5G) maintained high enzyme activity. This has suggested that Amino-PRX (5G) is a useful carrier capable of securing intracellular uptake of a protein, endosome escape, and release of the protein in the cytoplasm even in protein delivery.

(Example 14) mRNA Delivery Potential of Amino-PRX (5G)

In order to evaluate the mRNA delivery potential of Amino-PRX (5G) by using mCherry mRNA, the following test was performed.

HeLa cells were seeded on a 24 well plate ($3.75 \times 10^4$ cells/well), and incubated at 37°C for 24 hours. Next, 300 μL of serum-free culture medium containing one of the following samples was added to the cells after washed twice with serum-free culture medium.

(Sample)

mCherry mRNA alone (1500 ng)
Lipofectamine™2000 / mCherry mRNA (1500ng)
Amino-PRX (5G) NP0.5 / mCherry mRNA (1500ng)
Amino-PRX (5G) NP0.75 / mCherry mRNA (1500ng)
Amino-PRX (5G) NP1 / mCherry mRNA (1500ng)
Amino-PRX (5G) NP2 / mCherry mRNA (1500ng)

Note that the NP of NP0.5, NP0.75, NP1, or NP2 indicates the ratio between the amino group (plus charge) of Amino-PRX (5G) and the phosphate group (minus charge) of mCherry mRNA.

After incubation at 37°C for 4 hours in the presence of the sample, the cells were washed twice with serum-free culture medium. After 500 μL of DMEM containing 10% FBS was added, the cells were incubated at 37°C for 44 hours. The mean fluorescence intensity (MFI) of mCherry was evaluated by flow cytometry.

The results are shown in FIG. 29. When mCherry mRNA was introduced into cells by using Amino-PRX (5G), an increase in the expression level of mCherry was observed. This has suggested that Amino-PRX (5G) may also be useful as a carrier for mRNA delivery.

(Example 15) To Optimize Degree of Substitution in Amino-PRX (5G) The optimization of the degree of substitution in Amino-PRX (5G) was studied, and the Cas9 RNP delivery potential of less substituted Amino-PRX(5G) constructed was evaluated. Here, the "degree of substitution" is the number of functional groups modified in one macrocyclic molecule in PRX. The number of functional groups refers to the total number of modifications with various functional groups per macrocyclic molecule when a plurality of types of functional groups are bonded to the macrocyclic molecule in PRX. The number of functional groups refers to the total number of functional group modifications per macrocyclic molecule when the single kind of functional groups is bonded to the macrocyclic molecule in PRX. Note that in Amino-PRX (5G), the macrocyclic molecule is cyclodextrin (CyD). The term "less substituted" means that the number of functional group modifications is small. Here, the wording "the number of functional group modifications is small" means that the number of modifications with various functional groups per macrocyclic molecule in PRX is 1 or less for each functional group (when a single type of functional group is bonded to the macrocyclic molecule in PRX, the number of modifications with the functional group is 1 or less). That is, in the case of Amino-PRX (5G), the term "less substituted" (i.e., the number of functional group modifications is small) means that the number of modifications with Cys or DET per CyD molecule in PRX is 1 or less.

As less substituted Amino-PRX (5G), Cys(0.6)-DET(0.7)-PRX was used. The "Cys(0.6)-DET(0.7)-PRX" indicates that 0.6 Cys in average and 0.7 DET in average are bonded to one CyD molecule in PRX.

HeLa/GFP cells were seeded on a 24-well plate ($3.75 \times 10^4$ cells/well) and incubated at 37°C for 24 hours. Next, 500 μL of serum-free culture medium containing one of the following samples was added to the cells after washed twice with serum-free culture medium.

(Sample)

HBSS (control)
Cas9/sgGFP RNP alone (29.2 nM)
CRISPRMAX / Cas9 RNP (sgCont) (29.2 nM)
CRISPRMAX / Cas9 RNP (sgGFP) (29.2 nM)
Less substituted Amino-PRX (5G)/Cas9 RNP (sgcont) (29.2 nM)
Less substituted Amino-PRX (5G)/Cas9 RNP (sgGFP) (29.2 nM)

After incubation at 37°C for 4 hours in the presence of the sample, the cells were washed twice with serum-free culture medium. After 500 μL of DMEM containing 10% FBS was added, the cells were incubated at 37°C for 44 hours. The culture medium was changed every day and the cells were cultured for a total of 120 hours. The GFP knockout rate was then evaluated by flow cytometry.

The results are shown in FIG. 30. Less substituted Amino-PRX (5G)/Cas9 RNP (sgGFP) showed a better genome editing effect than CRISPRMAX/Cas9 RNP (sgGFP). Meanwhile, at the same Cas9 RNP concentration (29.2 nM), highly substituted Amino-PRX (5G)/Cas9 RNP showed a genome editing effect equivalent to that of CRISPRMAX/Cas9 RNP (FIG. 17). Note that highly substituted Amino-PRX (5G) in FIG. 17 is Cys(1.2)-DET(1.3)-PRX. This has suggested that less substituted Amino-PRX (5G) has higher Cas9 RNP releasability than highly substituted Amino-PRX (5G), thereby increasing the genome editing effect. In addition, it has been suggested that the number of modifications with Cys or DET per CyD molecule in PRX is preferably 1 or less and more preferably 0.7 or less.

In addition, when the degree of substitution is excessively low, the solubility of the carrier in water is lowered. This weakens the interaction between the carrier and the drug. In view of this, the degree of substitution should be preferably 0.5 or more and more preferably 1 or more. For example, in the case of Amino-PRX (5G), it is considered that the total number of modifications with Cys or DET per CyD molecule in PRX is preferably 0.5 or more and more preferably 1 or more.

## Claims

1. A polyrotaxane comprising: a plurality of macrocyclic molecules; an axial molecule penetrating a ring of each macrocyclic molecule; and caps bonded to ends of the axial molecule, wherein
an amine-containing modifying moiety having a monovalent proton at neutral pH or a divalent proton at acidic pH is bonded to at least one of the macrocyclic molecules.

2. The polyrotaxane according to claim 1, wherein the modifying moiety has a secondary amine and an amino group.

3. The polyrotaxane according to claim 2, wherein the modifying moiety is diethylenetriamine.

4. A polyrotaxane comprising: a plurality of macrocyclic molecules; an axial molecule penetrating a ring of each macrocyclic molecule; and caps bonded to ends of the axial molecule, wherein
an amino group is bonded, via an intracellularly degradable bond, to at least one of the macrocyclic molecules.

5. The polyrotaxane according to any one of claims 1 to 3, wherein separately from the modifying moiety, an amino group is bonded, via an intracellularly degradable bond, to at least one of the macrocyclic molecules.

6. The polyrotaxane according to claim 4 or 5, wherein the intracellularly degradable bond is a bond selected from the group consisting of carbamate, ketal, amide, ester, and disulfide bonds.

7. The polyrotaxane according to claim 6, wherein the intracellularly degradable bond is a disulfide bond.

8. The polyrotaxane according to claim 7, wherein cystamine is bonded to at least one of the macrocyclic molecules.

9. The polyrotaxane according to any one of claims 1 to 8, wherein each cap is bound, via an intracellularly degradable bond, to the axial molecule.

10. The polyrotaxane according to claim 9, wherein the intracellularly degradable bond is a bond selected from the group consisting of carbamate, ketal, amide, ester, and disulfide bonds.

11. The polyrotaxane according to claim 10, wherein the intracellularly degradable bond is a carbamate bond.

12. The polyrotaxane according to any one of claims 1 to 11, wherein each macrocyclic molecule is $\alpha$-cyclodextrin.

13. The polyrotaxane according to any one of claims 1 to 12, wherein the axial molecule is PEG.

14. A polyrotaxane composition comprising the polyrotaxane according to claim 1 and the polyrotaxane according to claim 4.

15. A polyion complex comprising a biological material and the polyrotaxane according to any one of claims 1 to 13.

16. The polyion complex according to claim 15, wherein the biological material is a nucleic acid molecule or a complex of a Cas9 protein and a guide RNA (Cas9 RNP).

17. The polyion complex according to claim 15 or 16, wherein an imprinting rate is from 20% to 100%.

18. A method of producing the polyion complex according to any one of claims 15 to 17, the method comprising:
mixing the biological material with the polyrotaxane according to any one of claims 1 to 13 to form a polyion complex.

19. A method of delivering a biological material into a cell, the method comprising
bringing the polyion complex according to any one of claims 15 to 17 into contact with a cell to incorporate the polyion complex into the cell.

20. A method of delivering a biological material into a cell, the method comprising:

    producing a polyion complex by the method according to claim 18; and
    bringing the produced polyion complex into contact with a cell to incorporate the polyion complex into the cell.

**21.** A method for genome editing, comprising:
bringing a polyion complex of a Cas9 protein/guide RNA complex (Cas9 RNP) and the polyrotaxane according to any one of claims 1 to 13 into contact with a cell to incorporate the polyion complex into the cell.

**22.** A method for genome editing, comprising:

mixing a Cas9 protein/guide RNA complex (Cas9 RNP) and the polyrotaxane according to any one of claims 1 to 13 to form a polyion complex, and
bringing the polyion complex into contact with a cell to incorporate the polyion complex into the cell.

**23.** An agent for delivering a biological material into a cell, comprising the polyrotaxane according to any one of claims 1 to 13.

**24.** A pharmaceutical composition comprising the polyrotaxane according to any one of claims 1 to 13 or the polyion complex according to any one of claims 15 to 17.

FIG. 1A

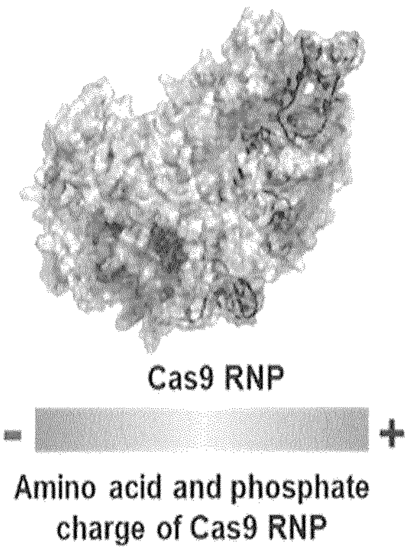


FIG. 1B

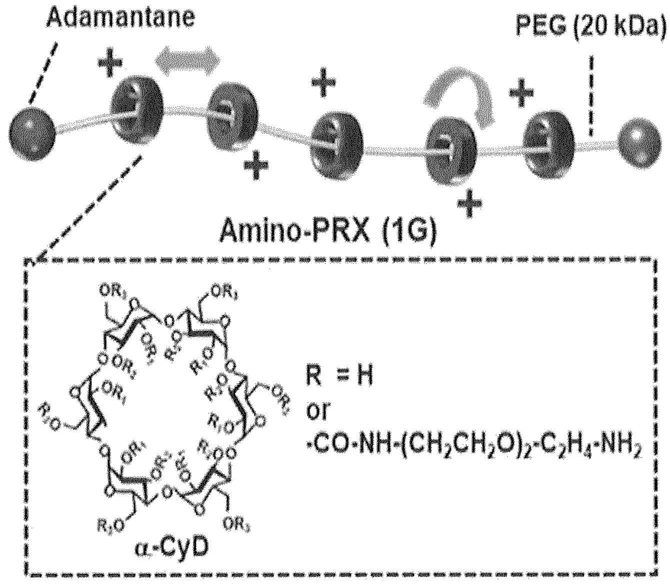


FIG. 1C

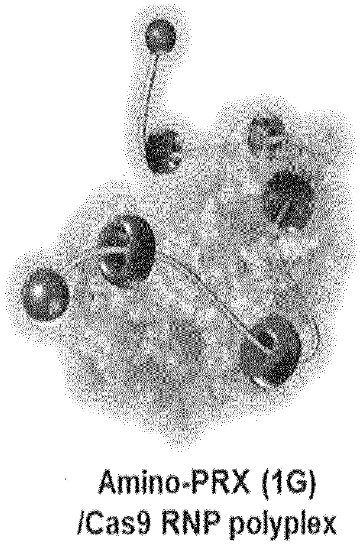

FIG. 2

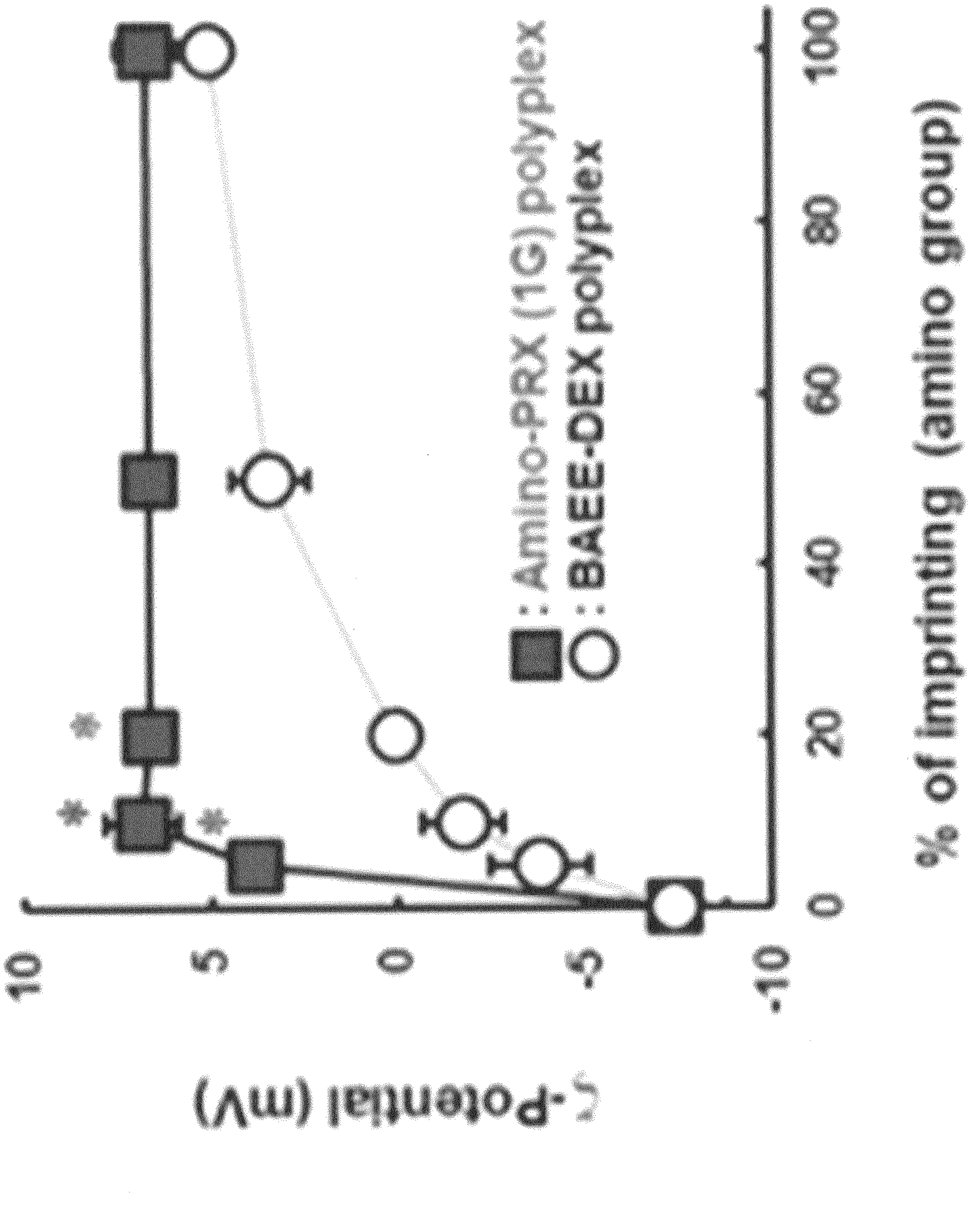
ζ-Potential (mV)
10
5
0
-5
-10
0
20
40
60
80
100
% of imprinting (amino group)
■ : Amino-PRX (1G) polyplex
○ : BAEE-DEX polyplex
*
*
*

FIG. 3

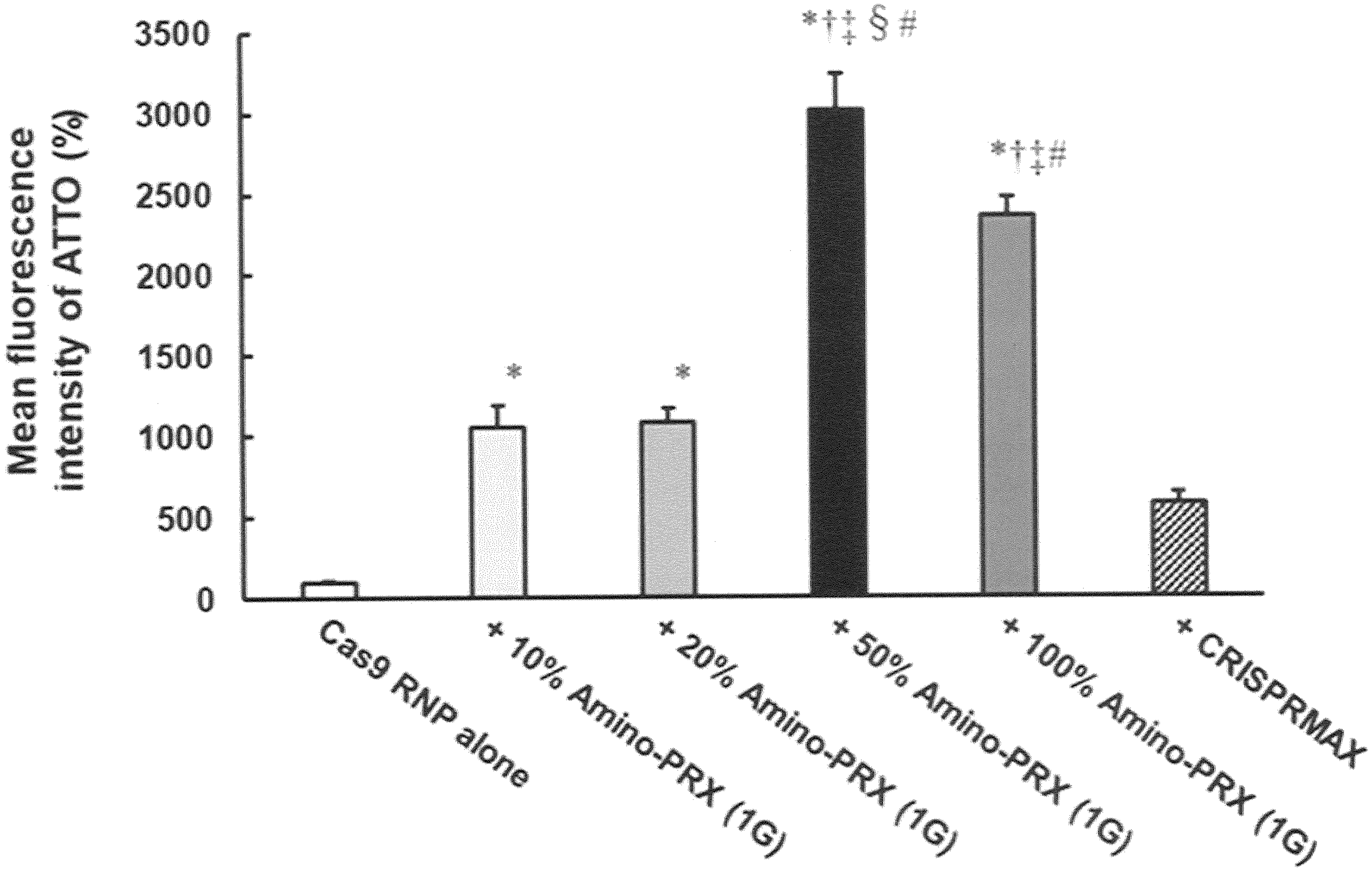


* $p < 0.05$, compared with Cas9 RNP alone.
† $p < 0.05$, compared with + 10% Amino-PRX (1G).
‡ $p < 0.05$, compared with + 20%10% Amino-PRX (1G).
§ $p < 0.05$, compared with + 100% 10% Amino-PRX (1G).
# $p < 0.05$, compared with CRISPRMAX.

FIG. 4A

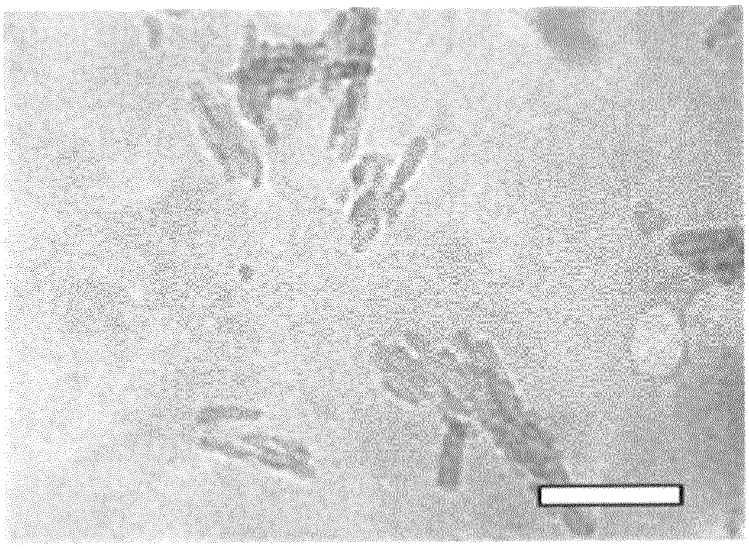

FIG. 4B

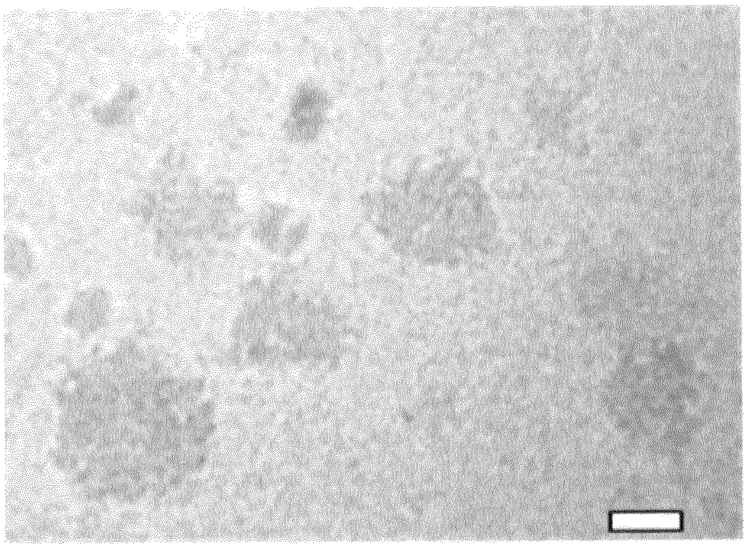

FIG. 4C

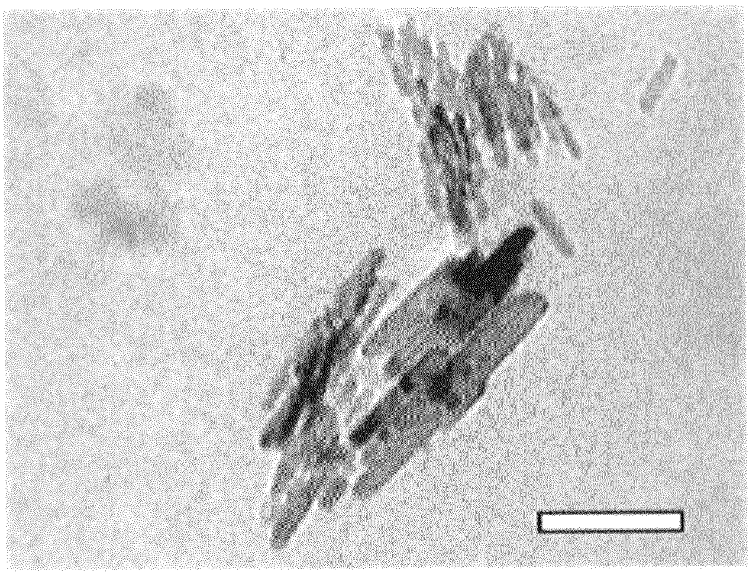

FIG. 5A

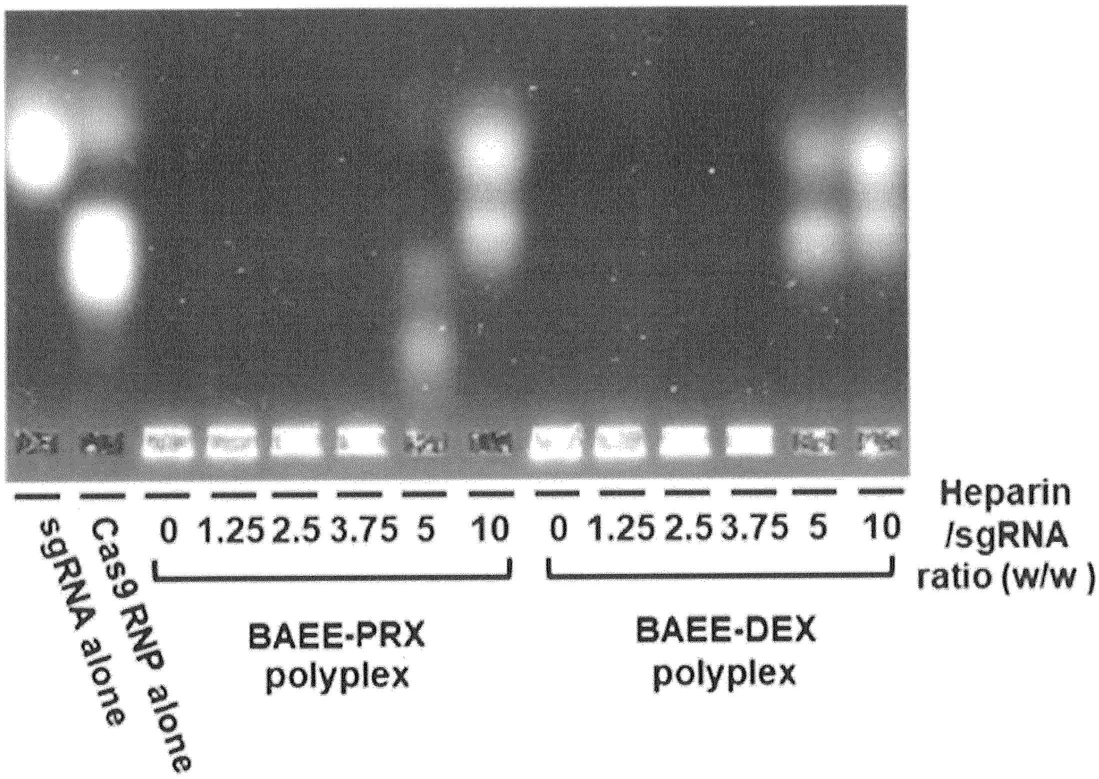
sgRNA alone
Cas9 RNP alone
0
1.25
2.5
3.75
5
10
0
1.25
2.5
3.75
5
10
Heparin /sgRNA ratio (w/w )
BAEE-PRX polyplex
BAEE-DEX polyplex

FIG. 5B

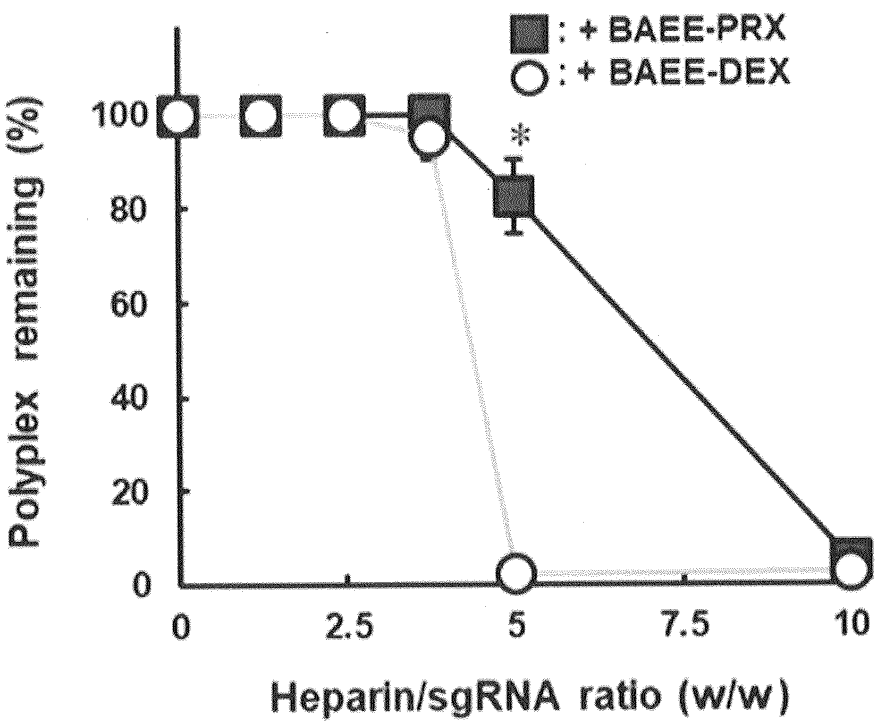
: + BAEE-PRX
: + BAEE-DEX
Polyplex remaining (%)
100
80
60
40
20
0
*
0
2.5
5
7.5
10
Heparin/sgRNA ratio (w/w)

FIG. 6A

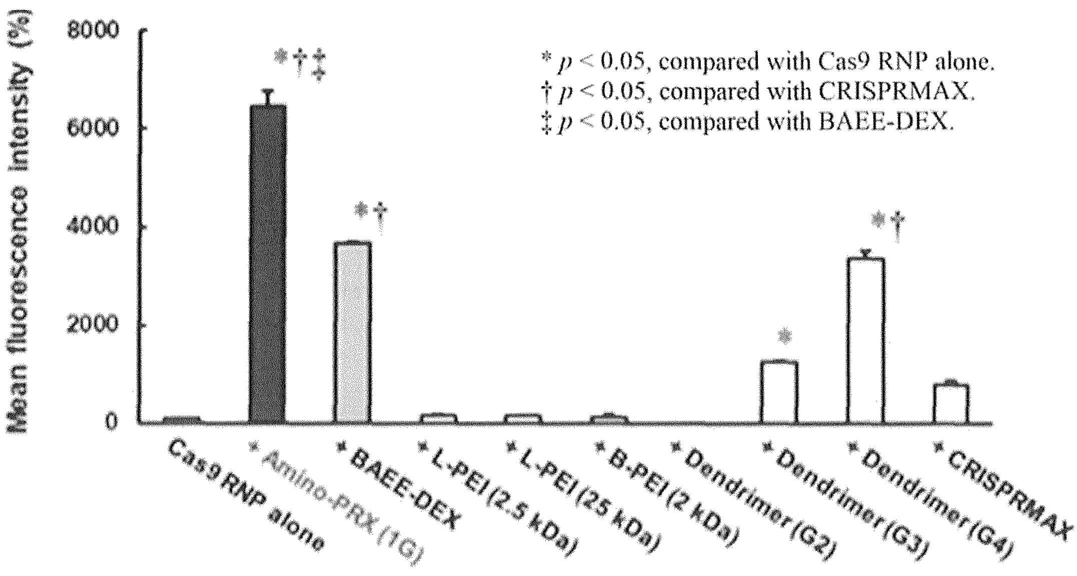


FIG. 6B

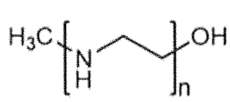
L-PEI

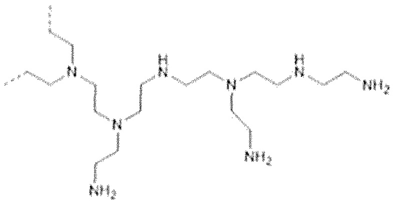
B-PEI

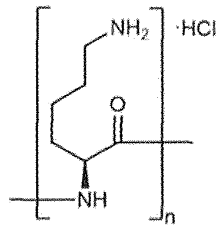
PLL

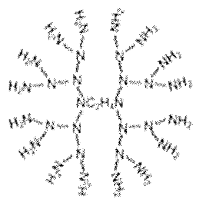

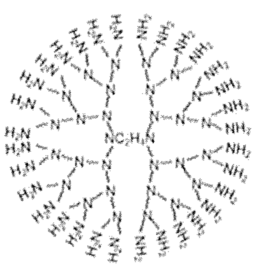

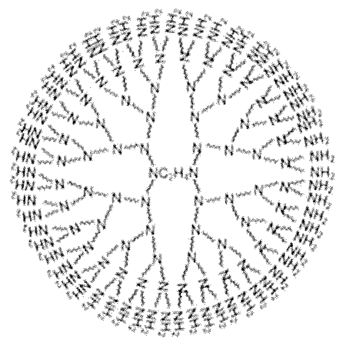

= $-C_2H_4CONHC_2H_4-$

Dendrimer (G2) Dendrimer (G3) Dendrimer (G4)

FIG. 6C

| Polymer | Number of functional amino groups | Molecular weight (kDa) |
|---|---|---|
| L-PEI (2.5 kDa) | 58 | 2.5 |
| L-PEI (25 kDa) | 580 | 25 |
| B-PEI | 8 | 2 |
| PLL (50 kDa) | 239 | 50 |
| PLL (120 kDa) | 576 | 120 |
| Dendrimer (G2) | 16 | 3.3 |
| Dendrimer (G3) | 32 | 6.9 |
| Dendrimer (G4) | 64 | 14.2 |

FIG. 7

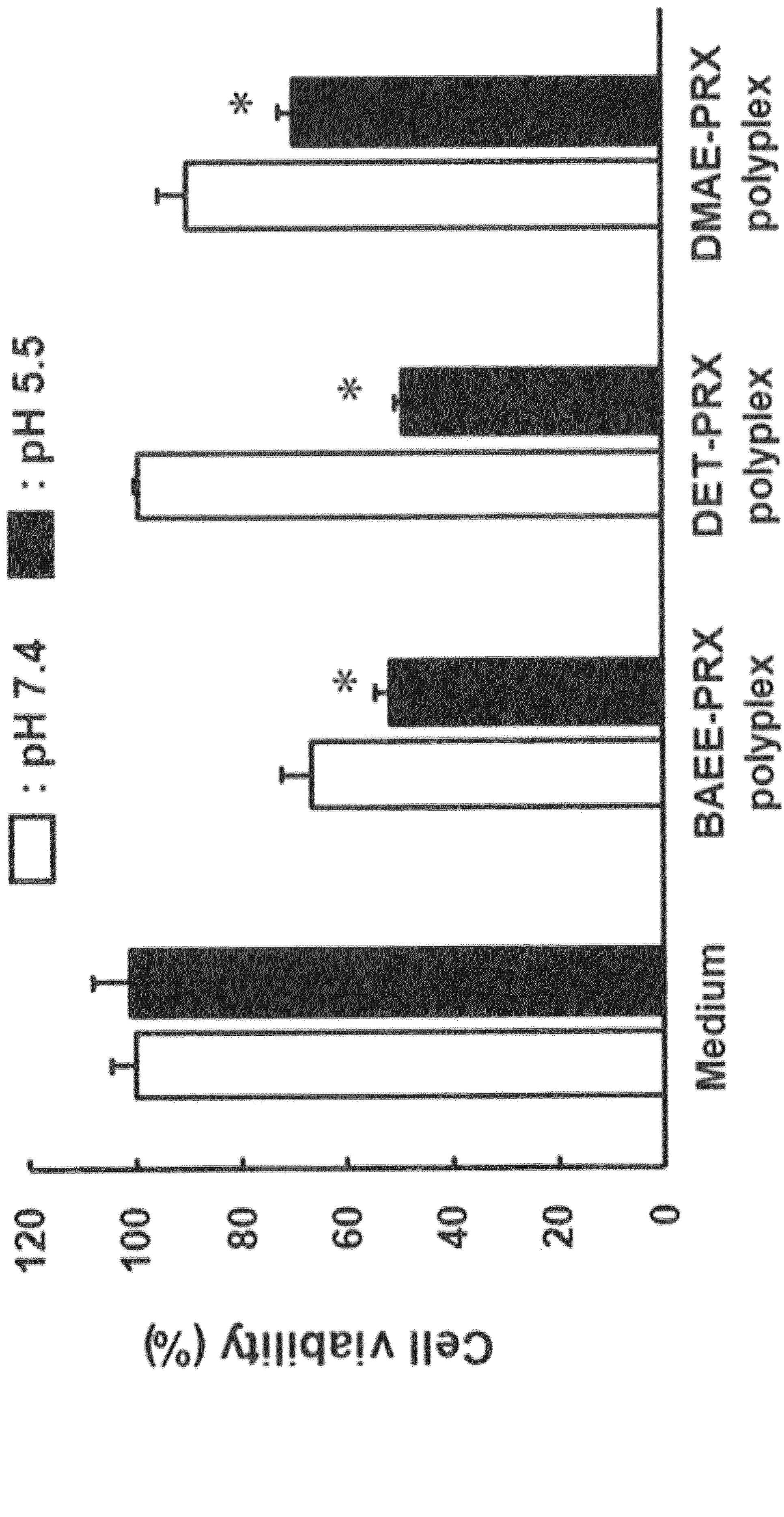

: pH 7.4
: pH 5.5
Cell viability (%)
0
20
40
60
80
100
120
Medium
BAEE-PRX polyplex
DET-PRX polyplex
DMAE-PRX polyplex
*
*
*

FIG. 8

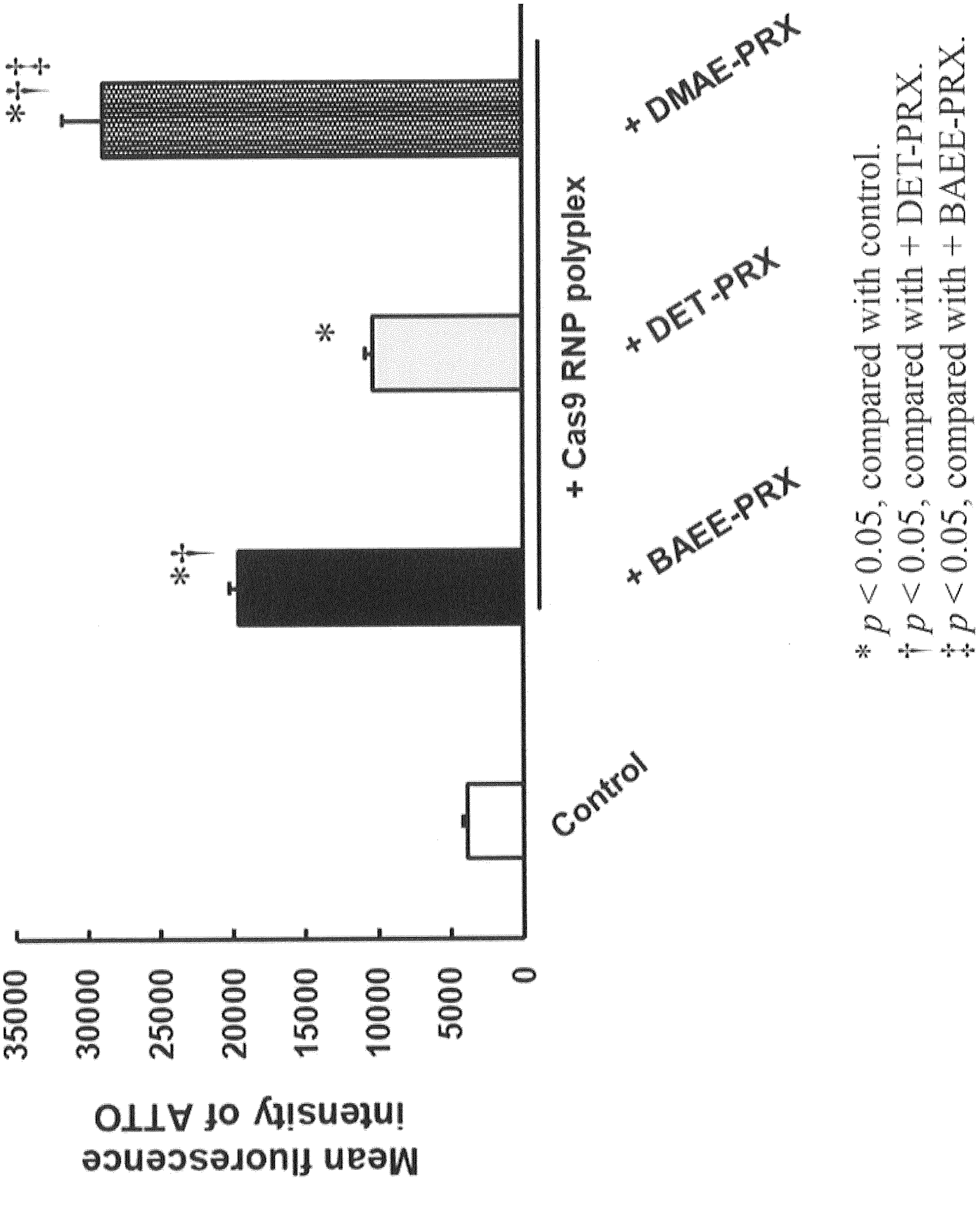
Mean fluorescence intensity of ATTO
35000
30000
25000
20000
15000
10000
5000
0
Control
+ BAEE-PRX
+ DET-PRX
+ DMAE-PRX
+ Cas9 RNP polyplex
*†
*
*†‡
* p < 0.05, compared with control.
† p < 0.05, compared with + DET-PRX.
‡ p < 0.05, compared with + BAEE-PRX.

FIG. 9A

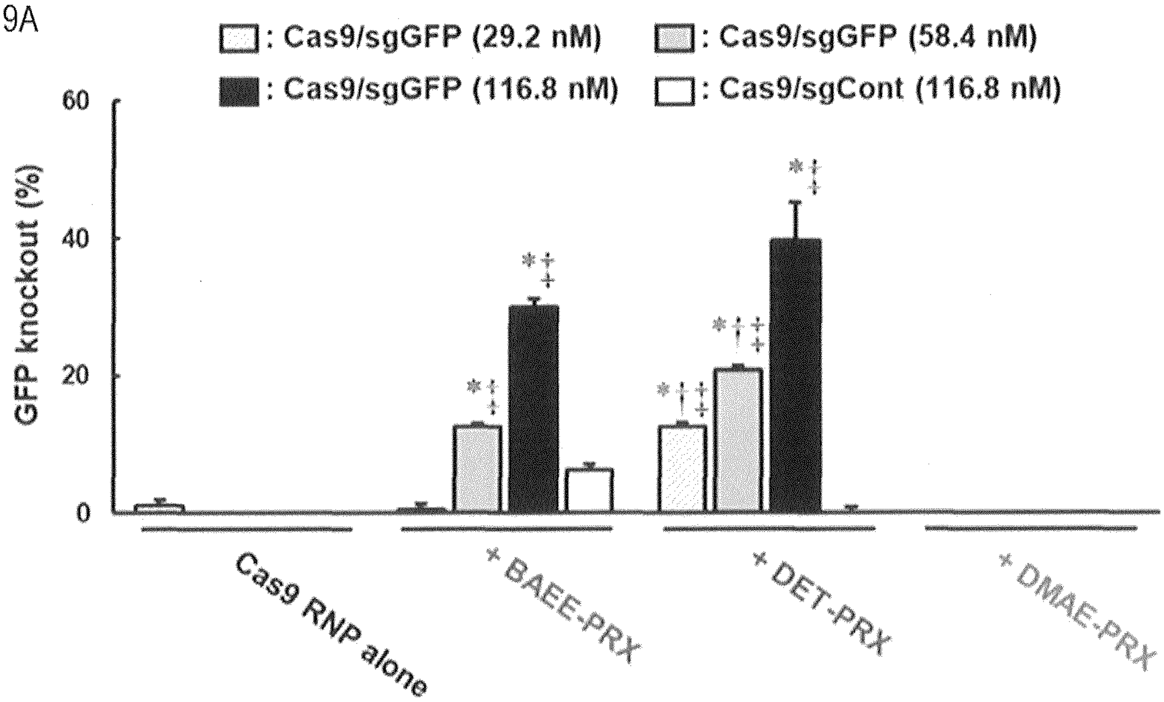


* $p < 0.05$, compared with Cas9 RNP alone.
† $p < 0.05$, compared with BAEE-PRX polyplex.
‡ $p < 0.05$, compared with DMAE-PRX polyplex.

FIG. 9B

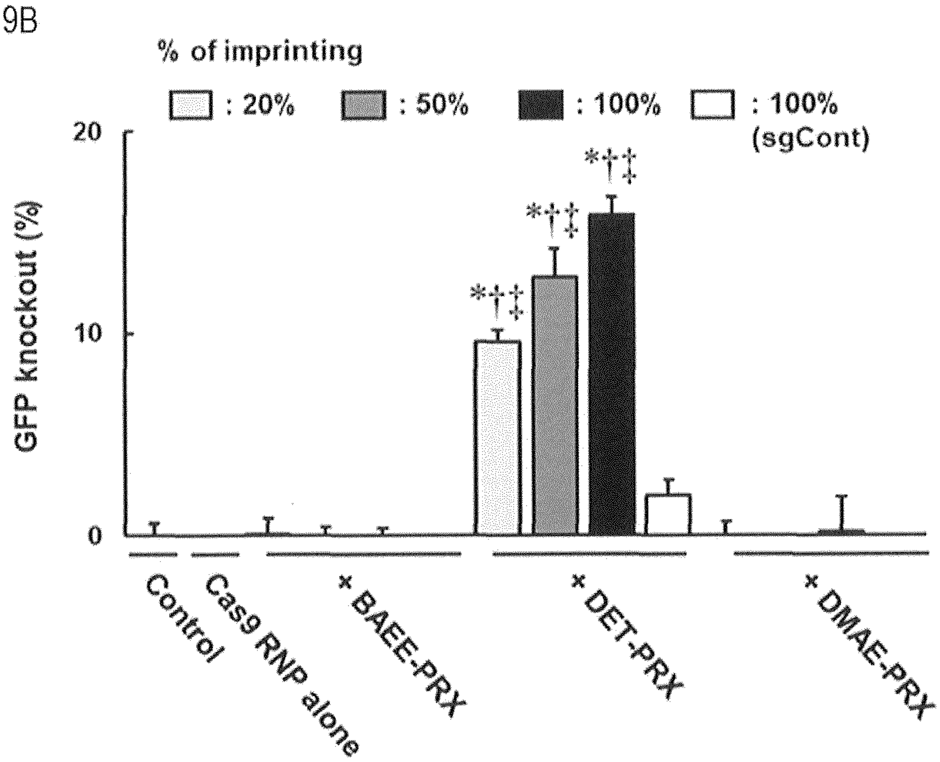


* $p < 0.05$, compared with Cas9 RNP alone.
† $p < 0.05$, compared with +BAEE-PRX.
‡ $p < 0.05$, compared with + DMAE-PRX.

FIG. 10

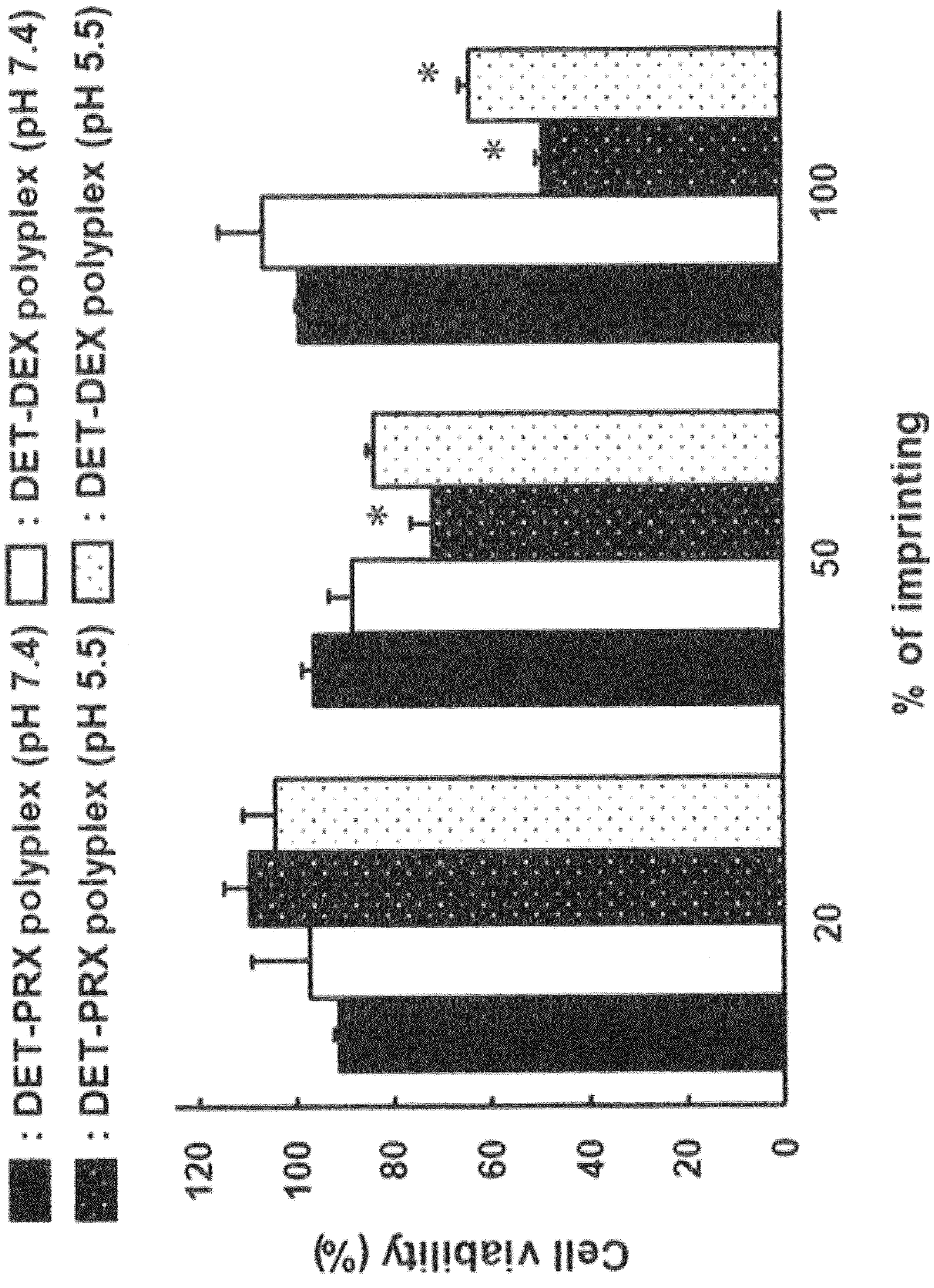
: DET-PRX polyplex (pH 7.4)
: DET-DEX polyplex (pH 7.4)
: DET-PRX polyplex (pH 5.5)
: DET-DEX polyplex (pH 5.5)
Cell viability (%)
120
100
80
60
40
20
0
*
*
*
20
50
100
% of imprinting

FIG. 11

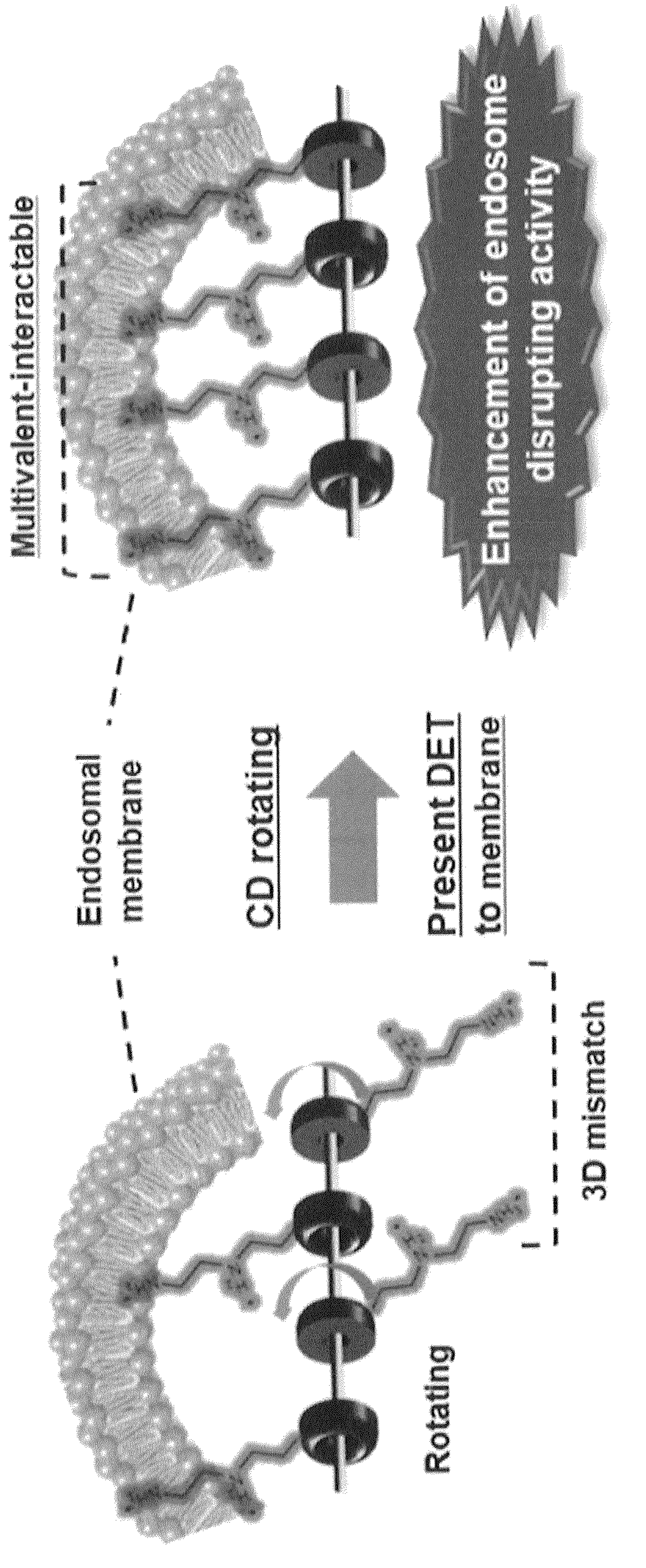
Rotating
3D mismatch
Endosomal
membrane
CD rotating
Present DET
to membrane
Multivalent-interactable
Enhancement of endosome
disrupting activity

FIG. 12A

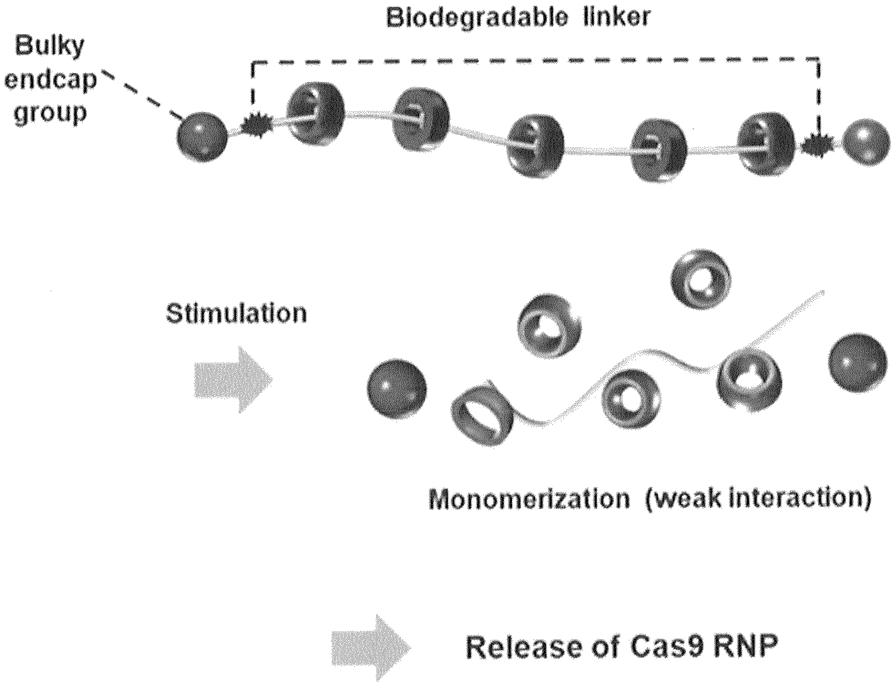


FIG. 12B

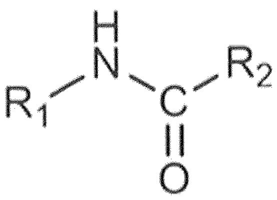

Amide bond
(non-cleavable control)

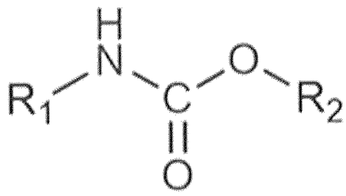

Carbamate bond
(CES-cleavable, cytosol)

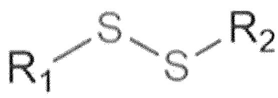

Disulfide bond
(GSH-cleavable, cytosol)

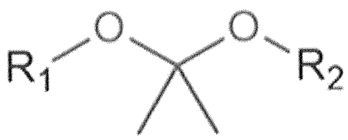

Ketal bond
(Acid-cleavable, endosome)

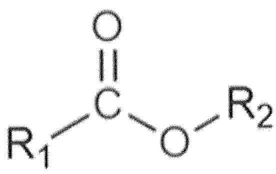

Ester bond
(Acid-cleavable, endosome)

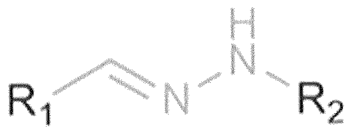

Hydrazone bond
(Acid-cleavable, endosome

FIG. 13

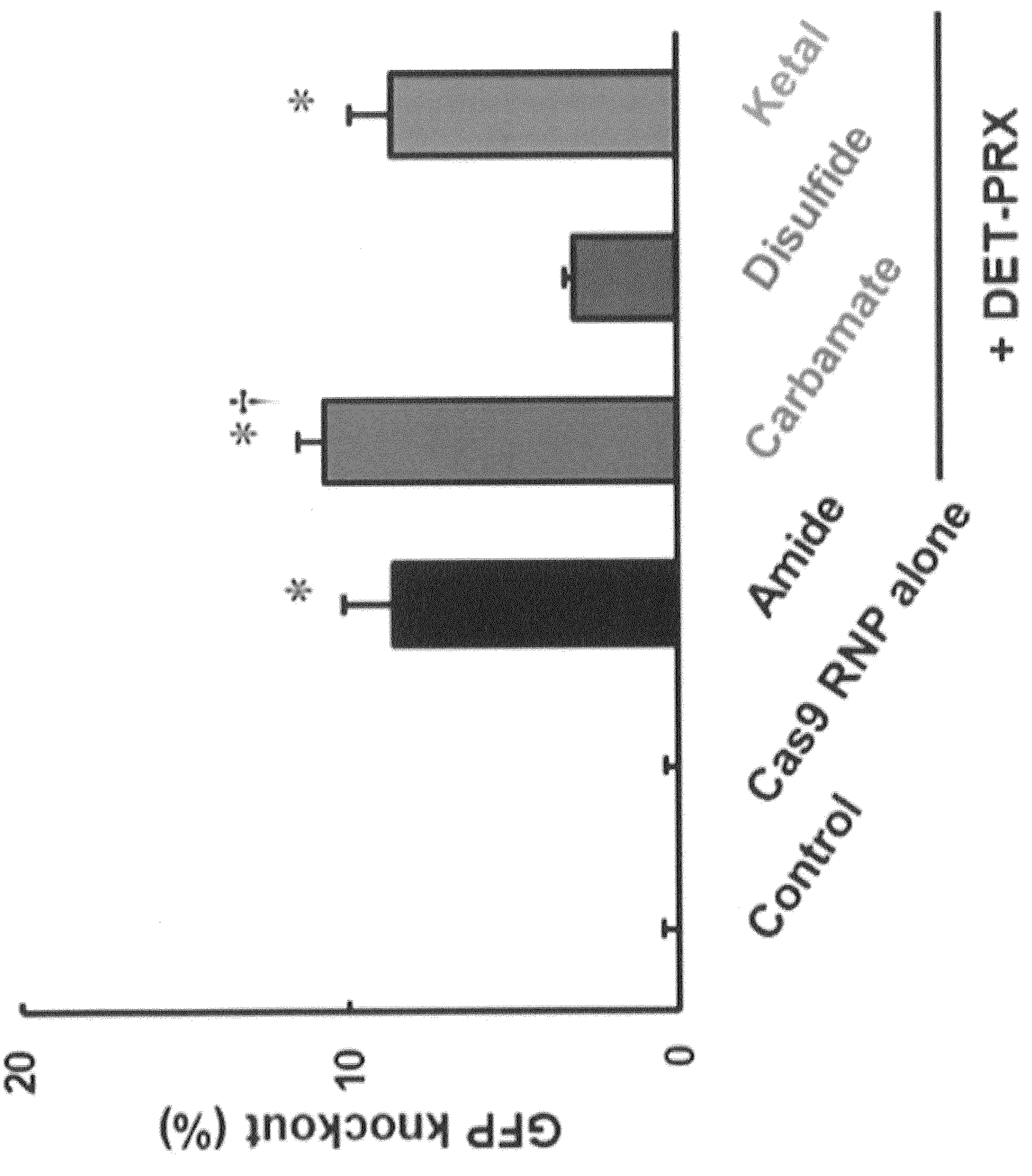


* $p < 0.05$, compared with Cas9 RNP alone.

† $p < 0.05$, compared with disulfide-DET-PRX polyplex.

FIG. 14A

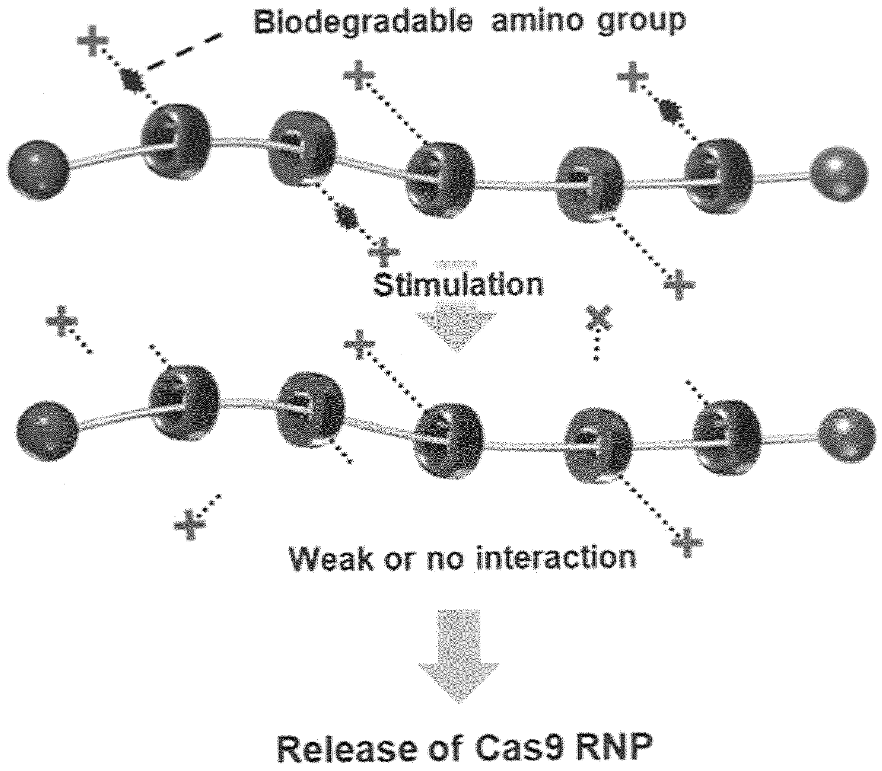


FIG. 14B

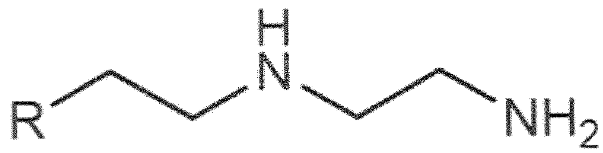

DET (endosome disruptable)

+

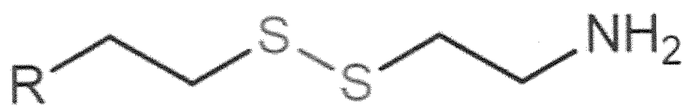

Cystamine (Cys, GSH-cleavable)

FIG. 15A

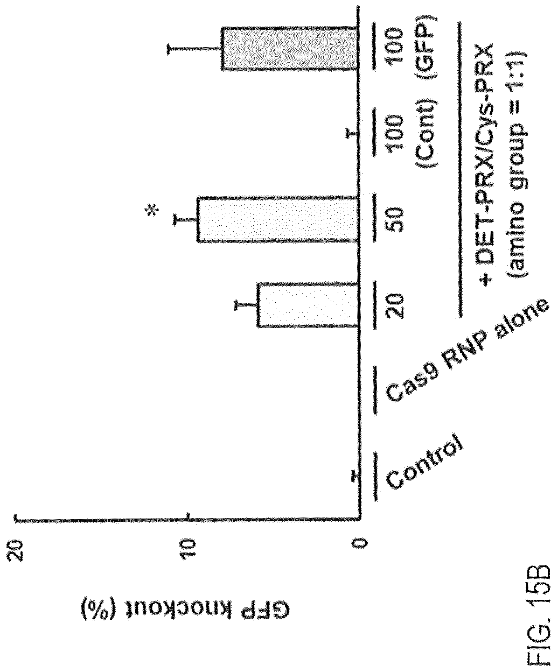


FIG. 15B

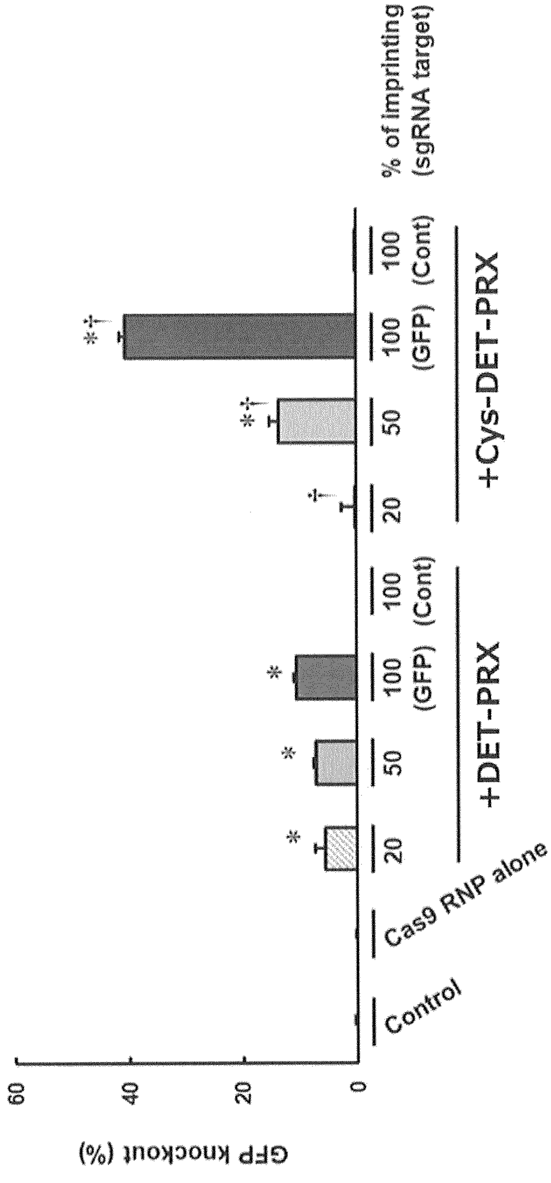


*$p < 0.05$, compared with Cas9 RNP alone.
†$p < 0.05$, compared with Amino-PRX (2G) polyplex.

FIG. 16A

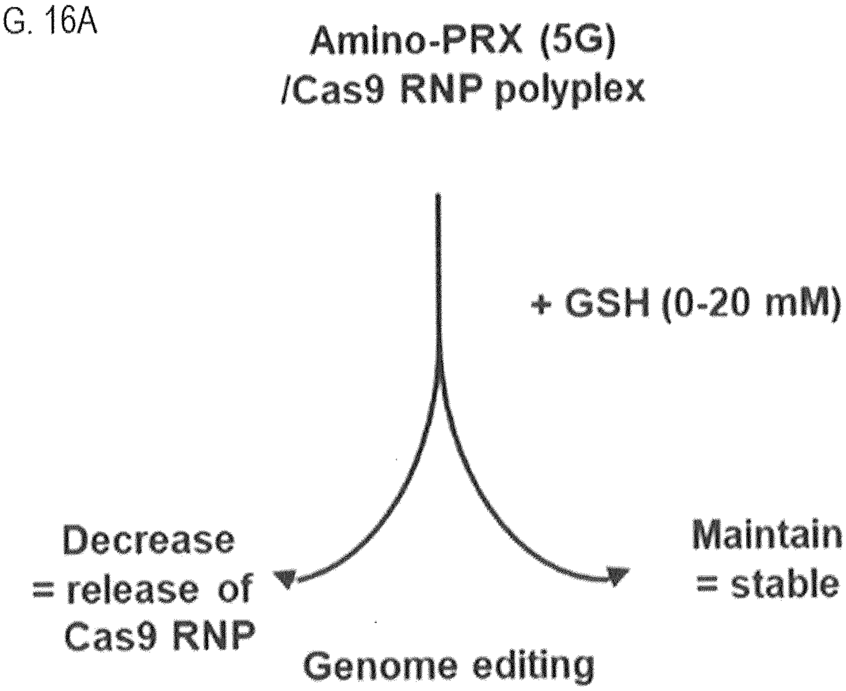


FIG. 16B

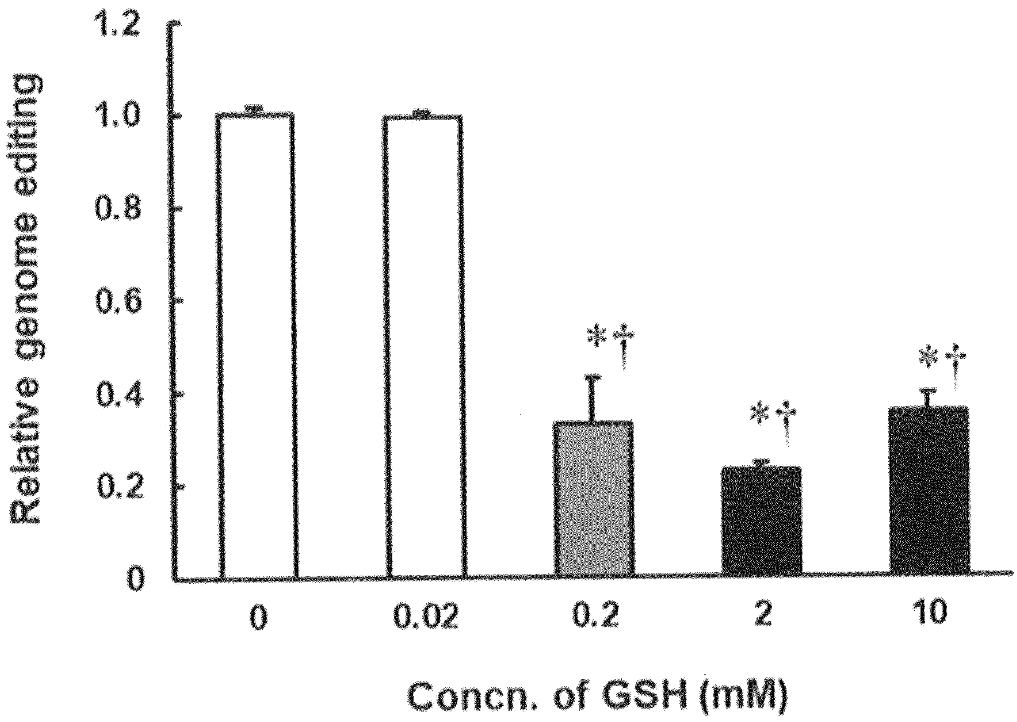


* $p < 0.05$, compared with 0 mM.
† $p < 0.05$, compared with 0.02 mM.

FIG. 17

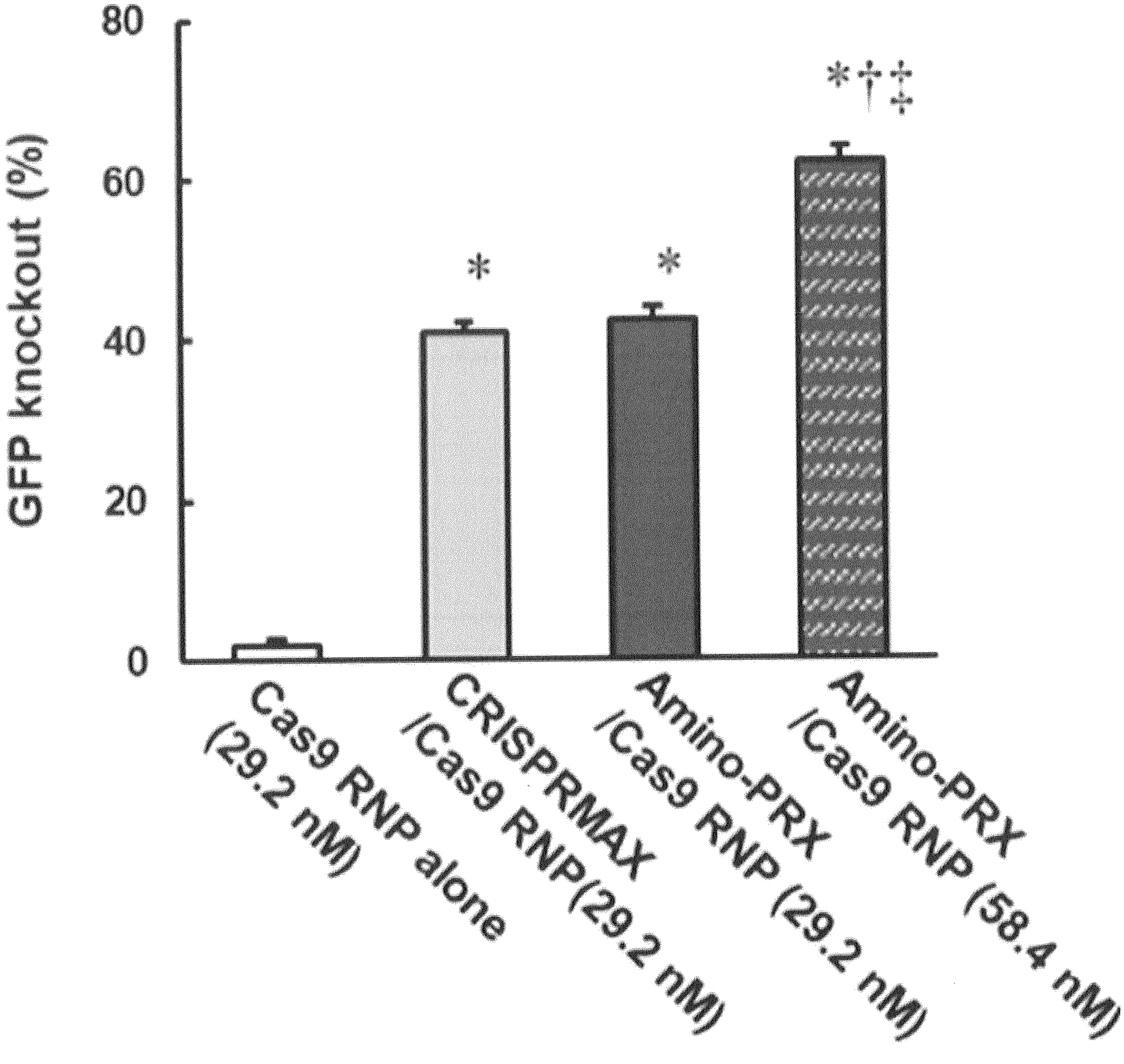


* $p < 0.05$, compared with Cas9 RNP alone.
† $p < 0.05$, compared with CRISPRMAX/Cas9 RNP (29.2 nM).
‡ $p < 0.05$, compared with Amino-PRX/Cas9 RNP (29.2 nM).

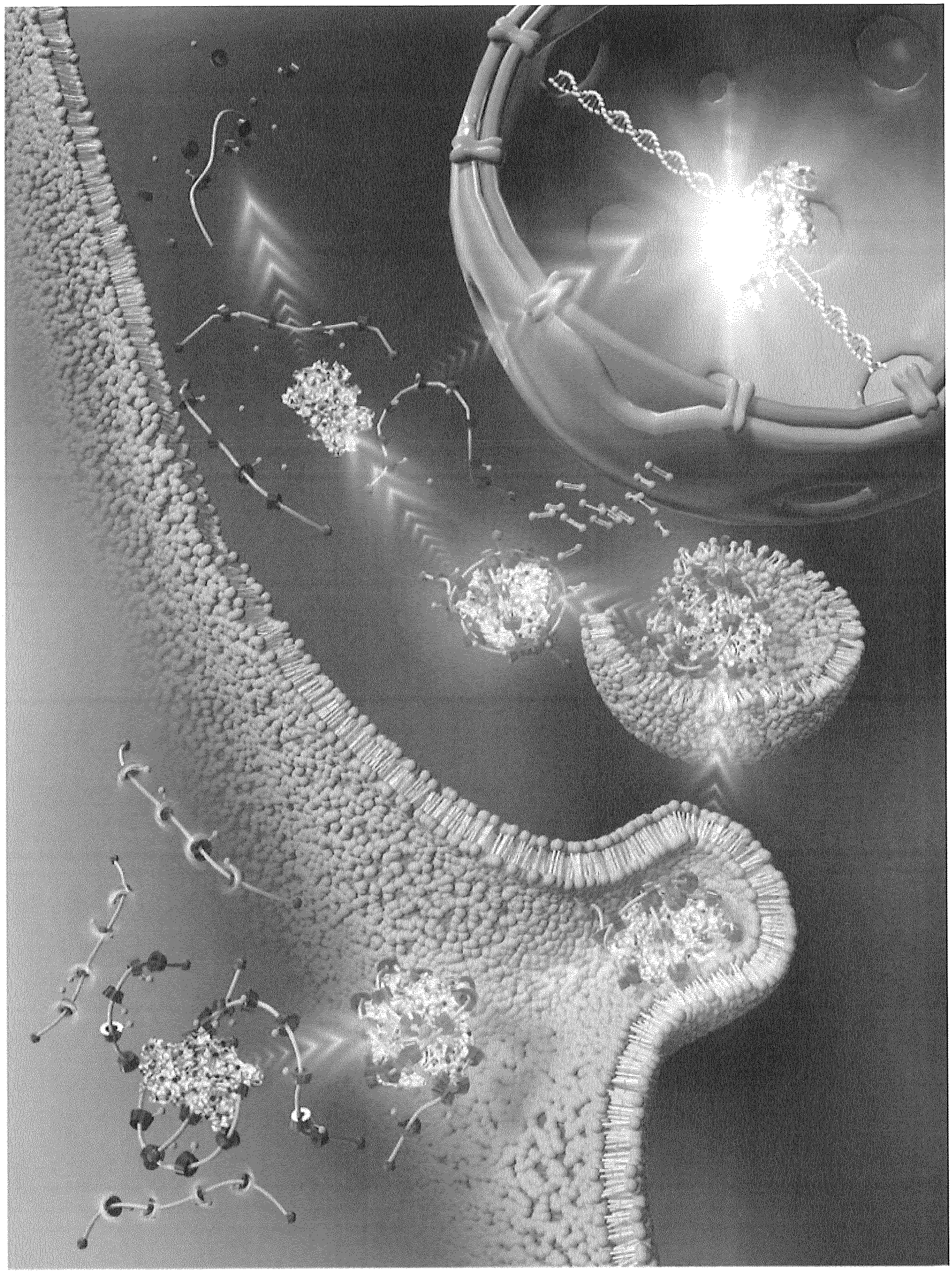

FIG. 18

FIG. 19A

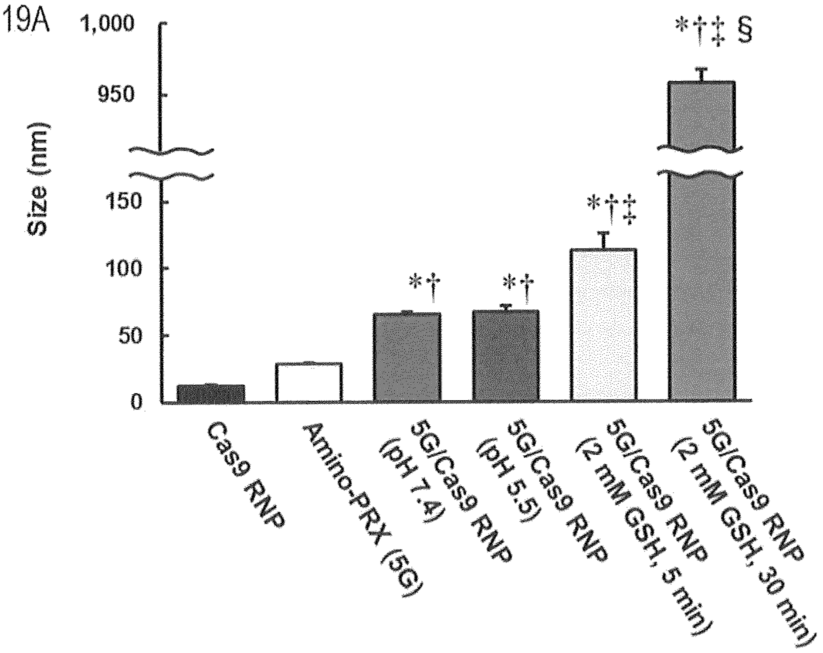


FIG. 19B

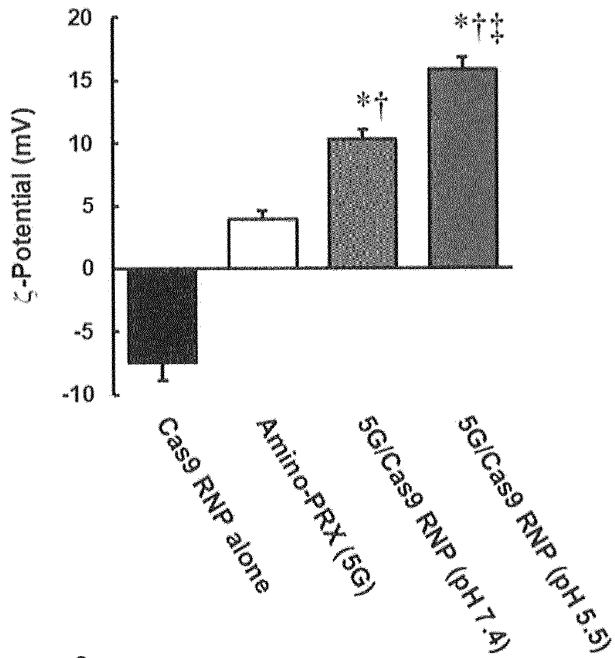


FIG. 19C

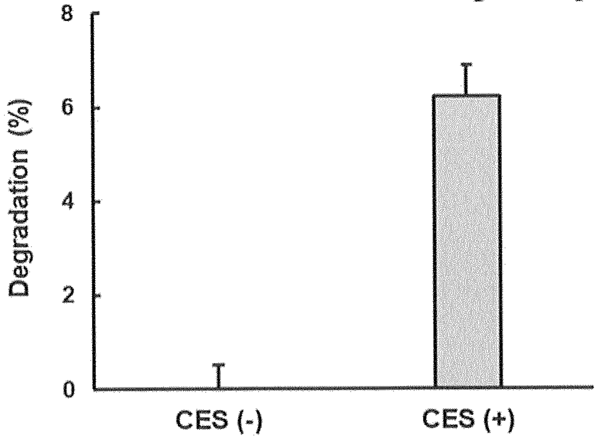

FIG. 20

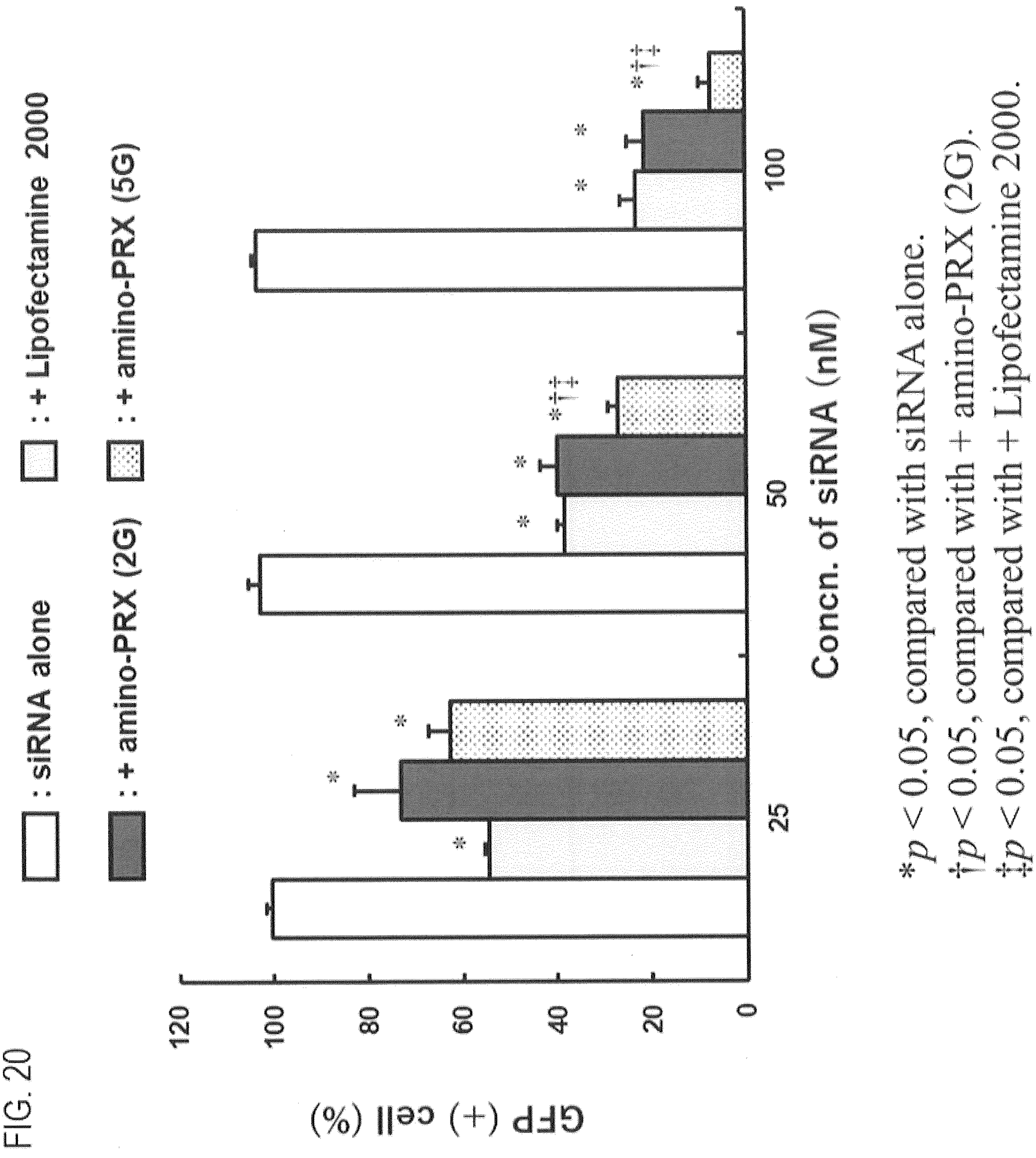

: siRNA alone
: + Lipofectamine 2000
: + amino-PRX (2G)
: + amino-PRX (5G)
GFP (+) cell (%)
0
20
40
60
80
100
120
25
50
100
Concn. of siRNA (nM)
*
*†‡
*p < 0.05, compared with siRNA alone.
†p < 0.05, compared with + amino-PRX (2G).
‡p < 0.05, compared with + Lipofectamine 2000.

FIG. 21

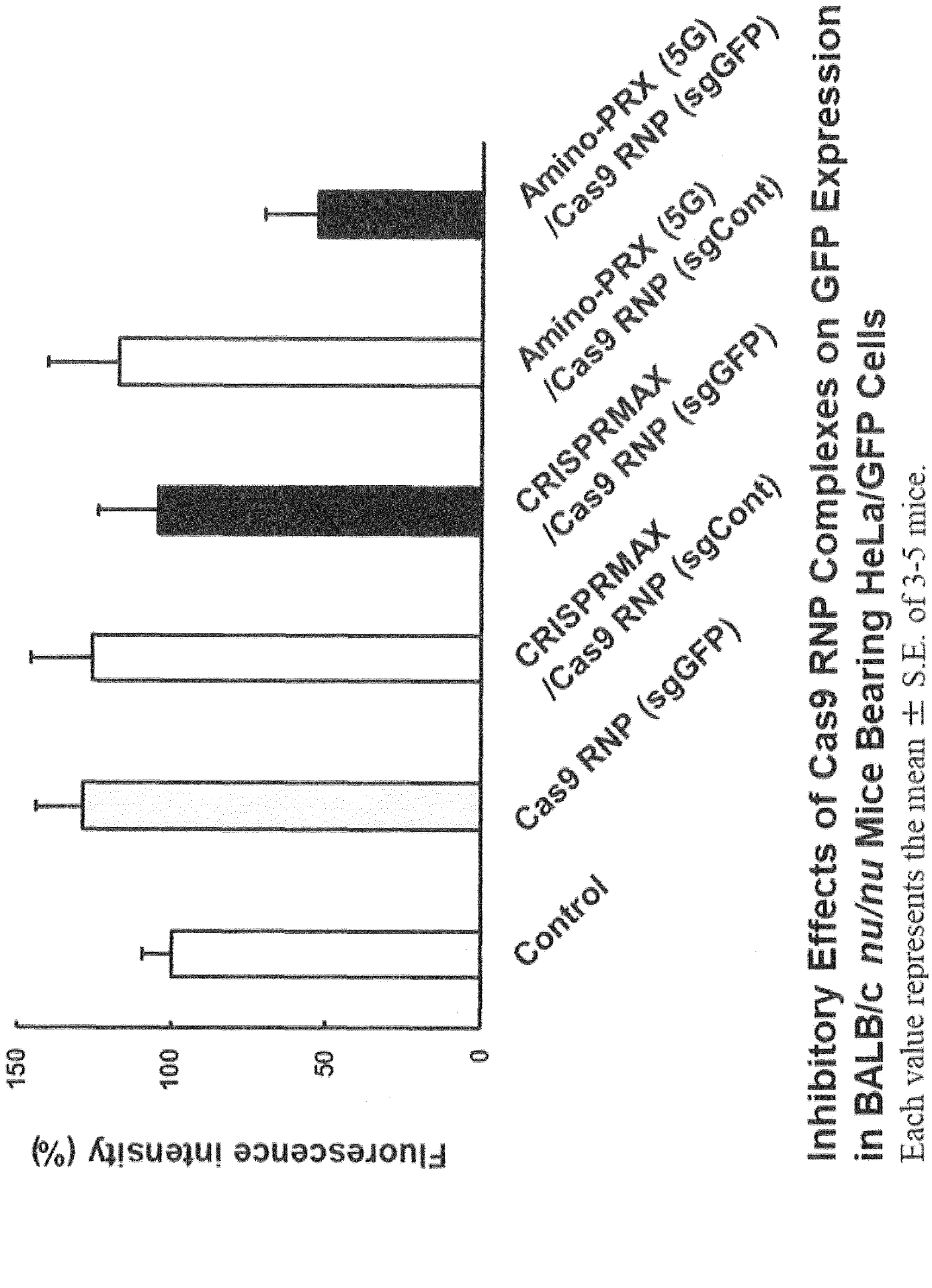
Fluorescence intensity (%)
150
100
50
0
Control
Cas9 RNP (sgGFP)
CRISPRMAX /Cas9 RNP (sgCont)
CRISPRMAX /Cas9 RNP (sgGFP)
Amino-PRX (5G) /Cas9 RNP (sgCont)
Amino-PRX (5G) /Cas9 RNP (sgGFP)
Inhibitory Effects of Cas9 RNP Complexes on GFP Expression in BALB/c nu/nu Mice Bearing HeLa/GFP Cells
Each value represents the mean ± S.E. of 3-5 mice.

FIG. 22

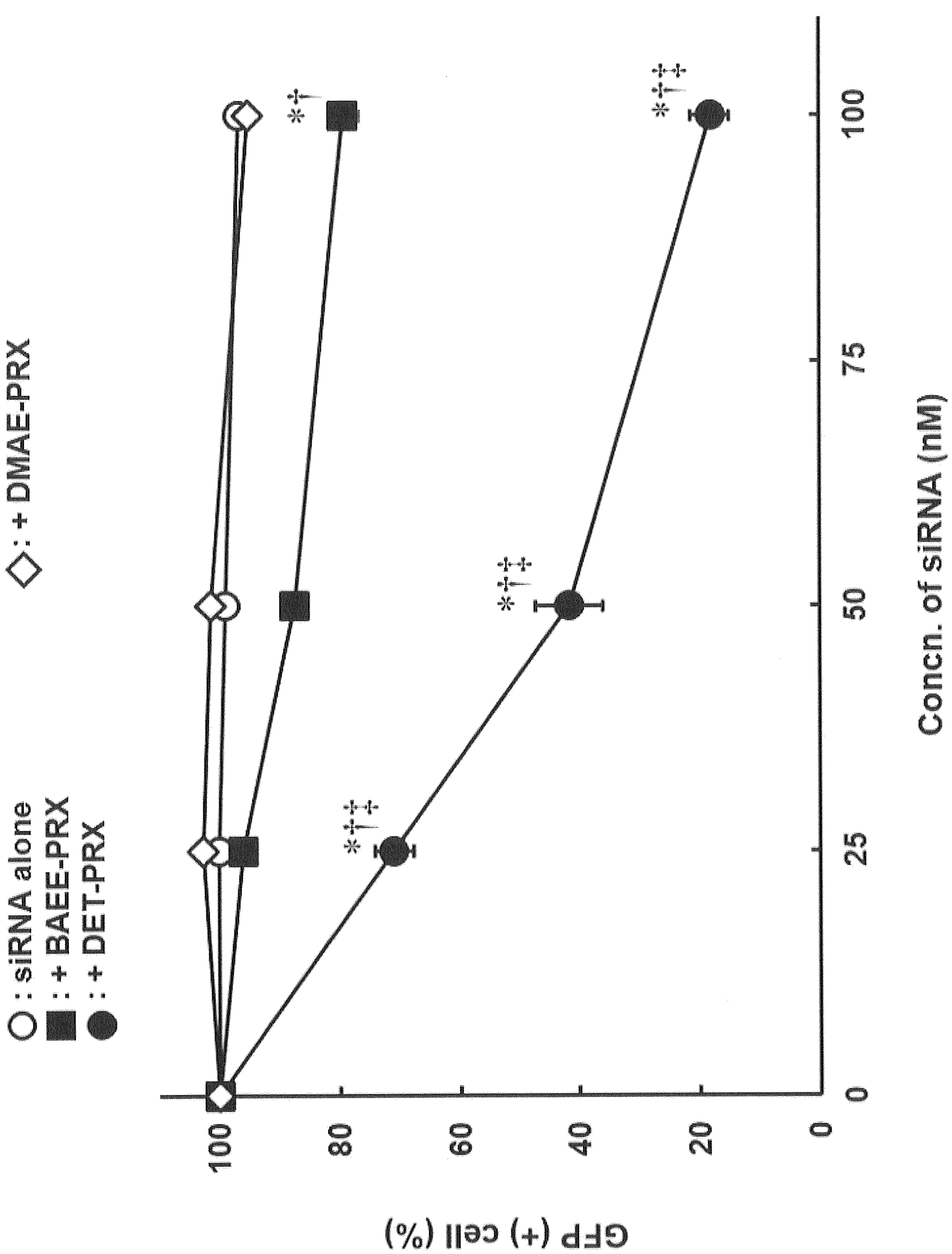

○ : siRNA alone
■ : + BAEE-PRX
● : + DET-PRX
◇ : + DMAE-PRX
GFP (+) cell (%)
100
80
60
40
20
0
0
25
50
75
100
Concn. of siRNA (nM)

FIG. 23

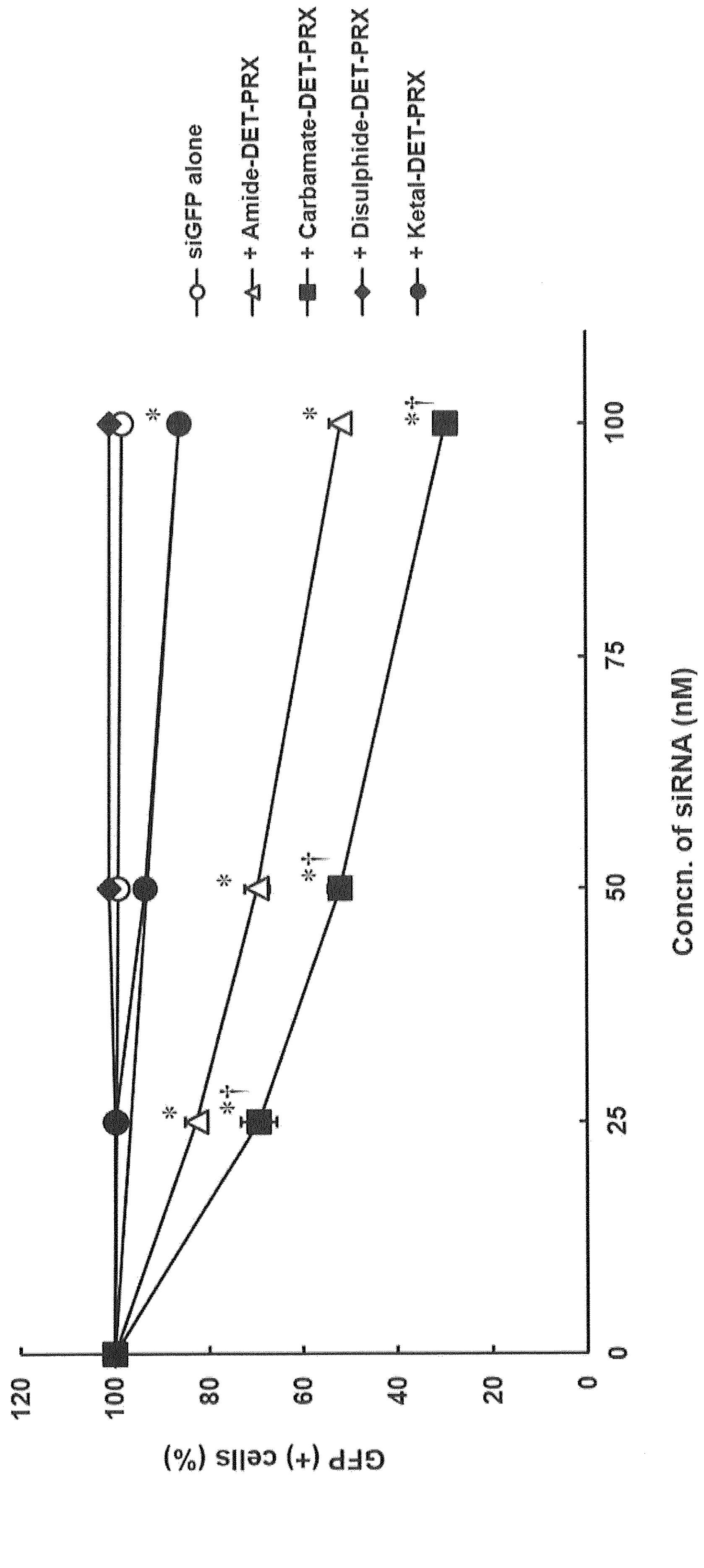
siGFP alone
+ Amide-DET-PRX
+ Carbamate-DET-PRX
+ Disulphide-DET-PRX
+ Ketal-DET-PRX
GFP (+) cells (%)
0
20
40
60
80
100
120
0
25
50
75
100
Concn. of siRNA (nM)
*
*†

FIG. 24

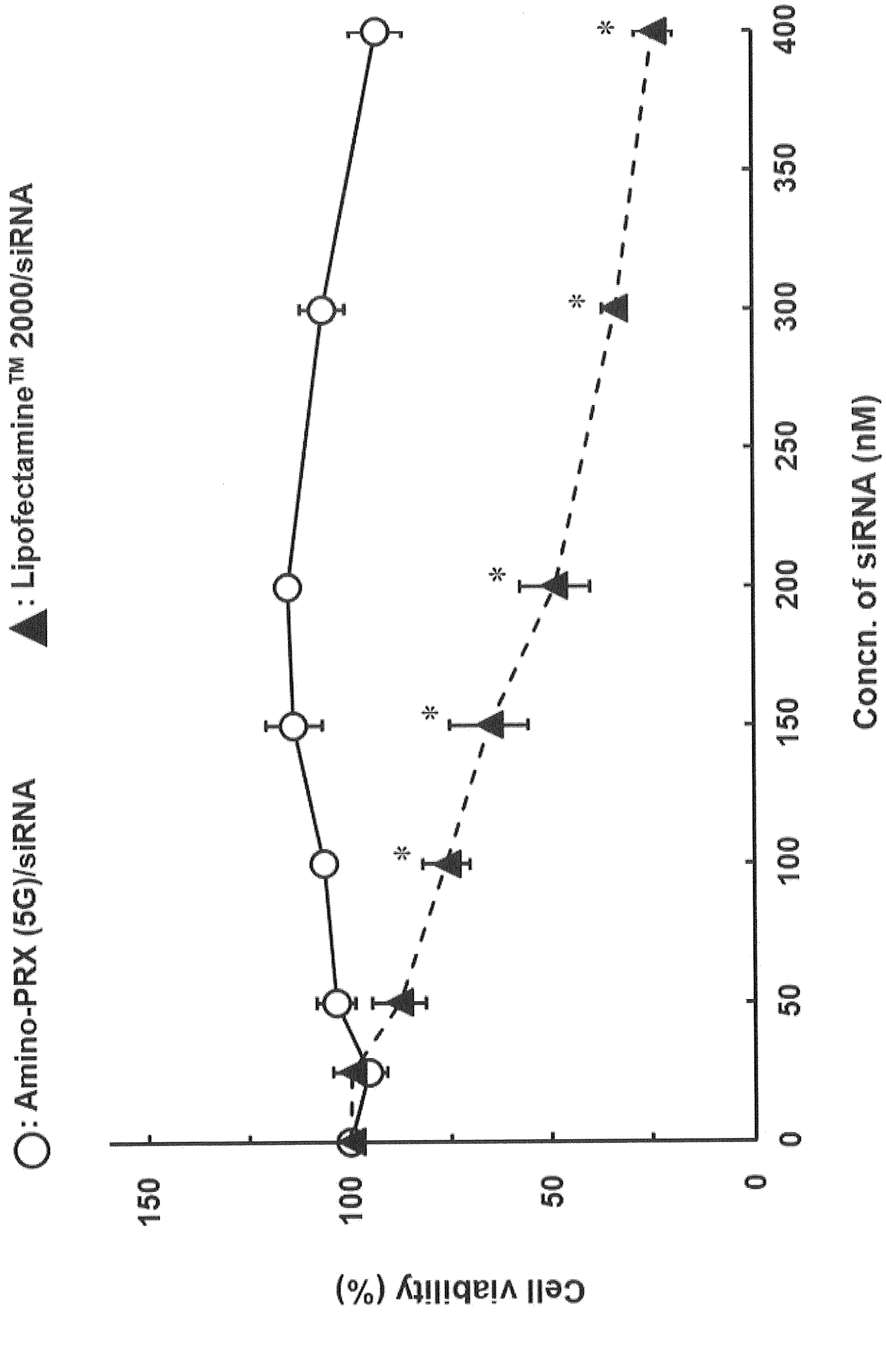
○: Amino-PRX (5G)/siRNA
▲: Lipofectamine™ 2000/siRNA
Cell viability (%)
0
50
100
150
0
50
100
150
200
250
300
350
400
Concn. of siRNA (nM)
*

FIG. 25

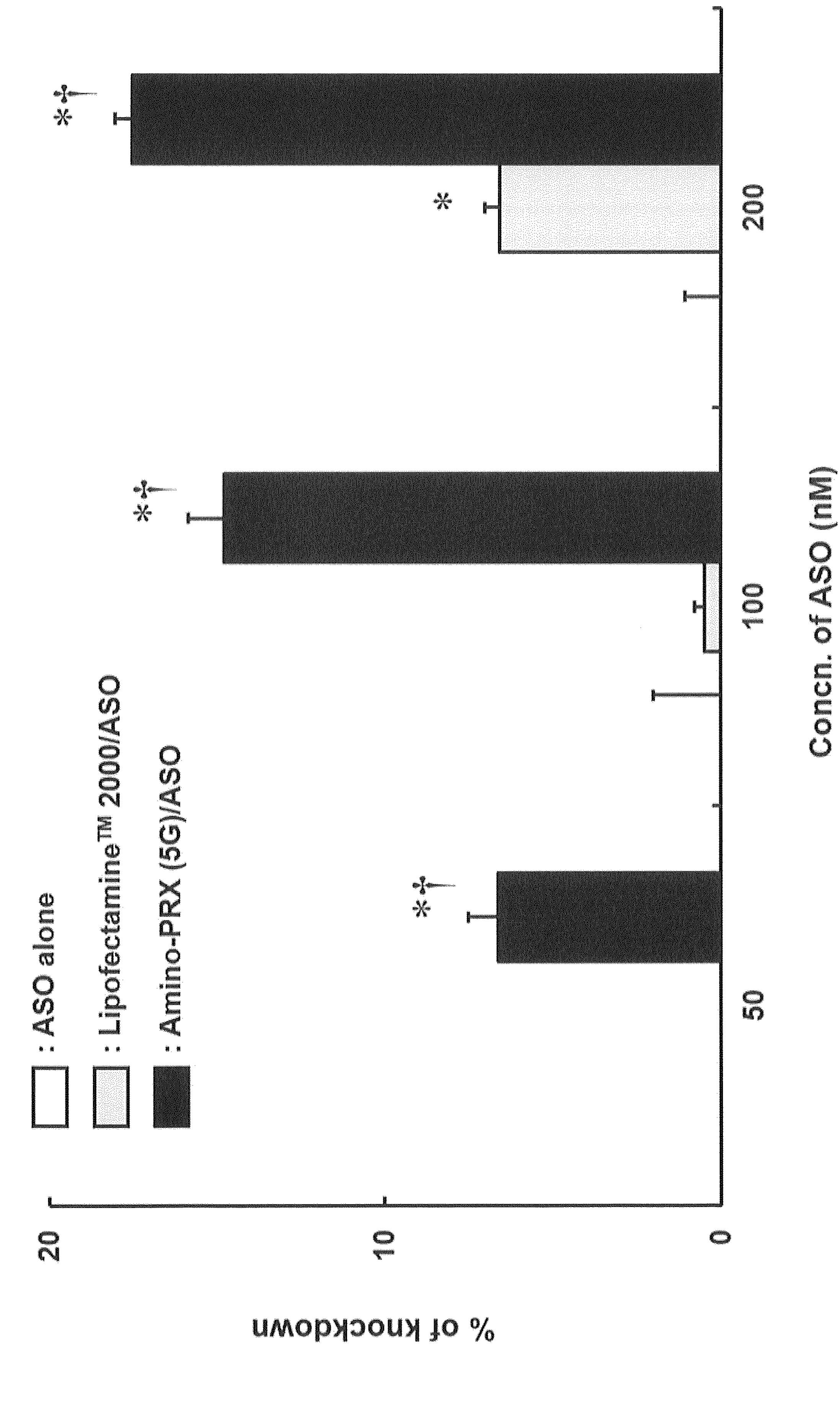
: ASO alone
: Lipofectamine™ 2000/ASO
: Amino-PRX (5G)/ASO
% of knockdown
20
10
0
50
100
200
Concn. of ASO (nM)
*†
*†
*
*†

FIG. 26

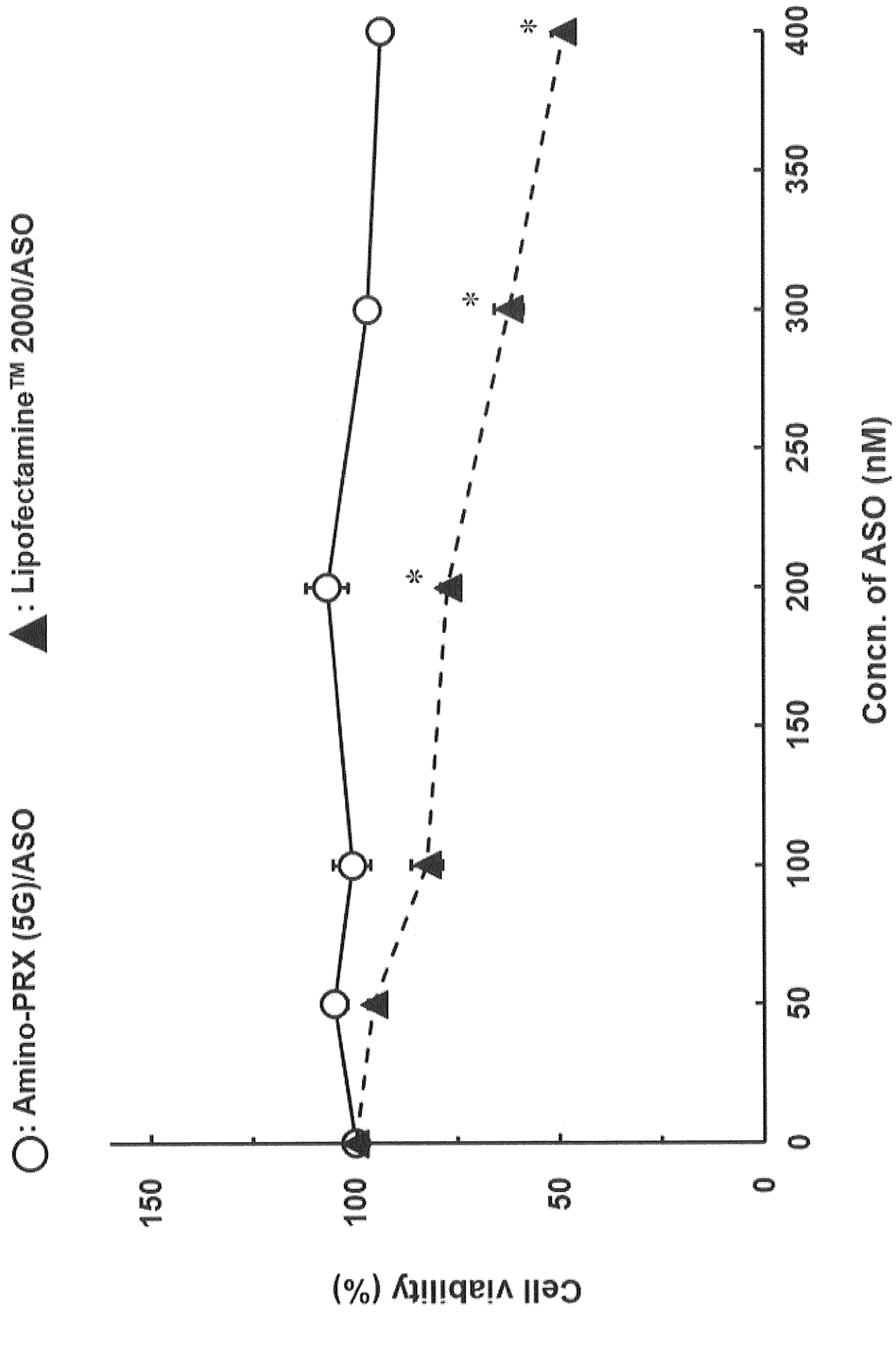
○ : Amino-PRX (5G)/ASO
▲ : Lipofectamine™ 2000/ASO
Cell viability (%)
150
100
50
0
0
50
100
150
200
250
300
350
400
Concn. of ASO (nM)
*
*
*

FIG. 27

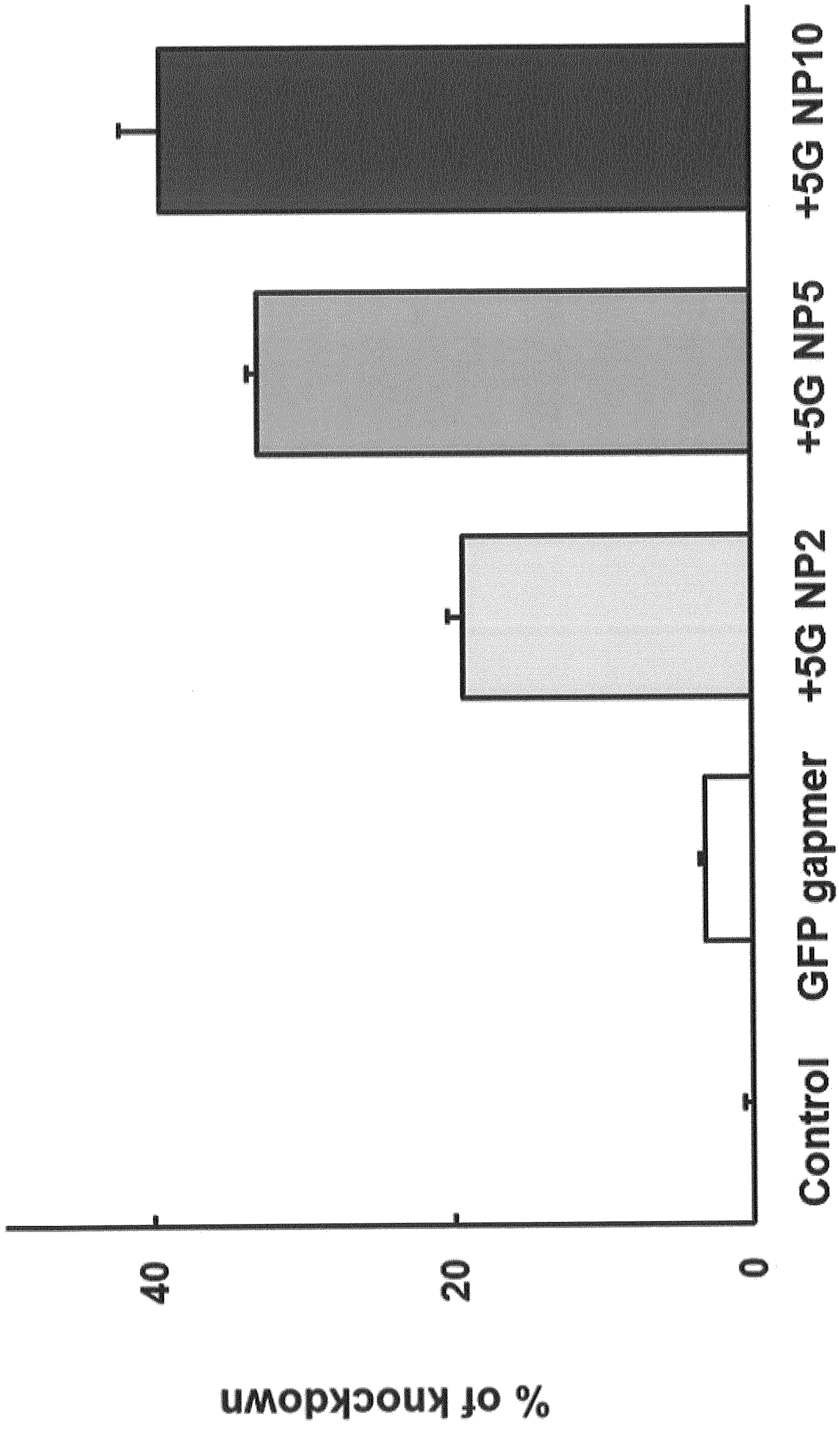

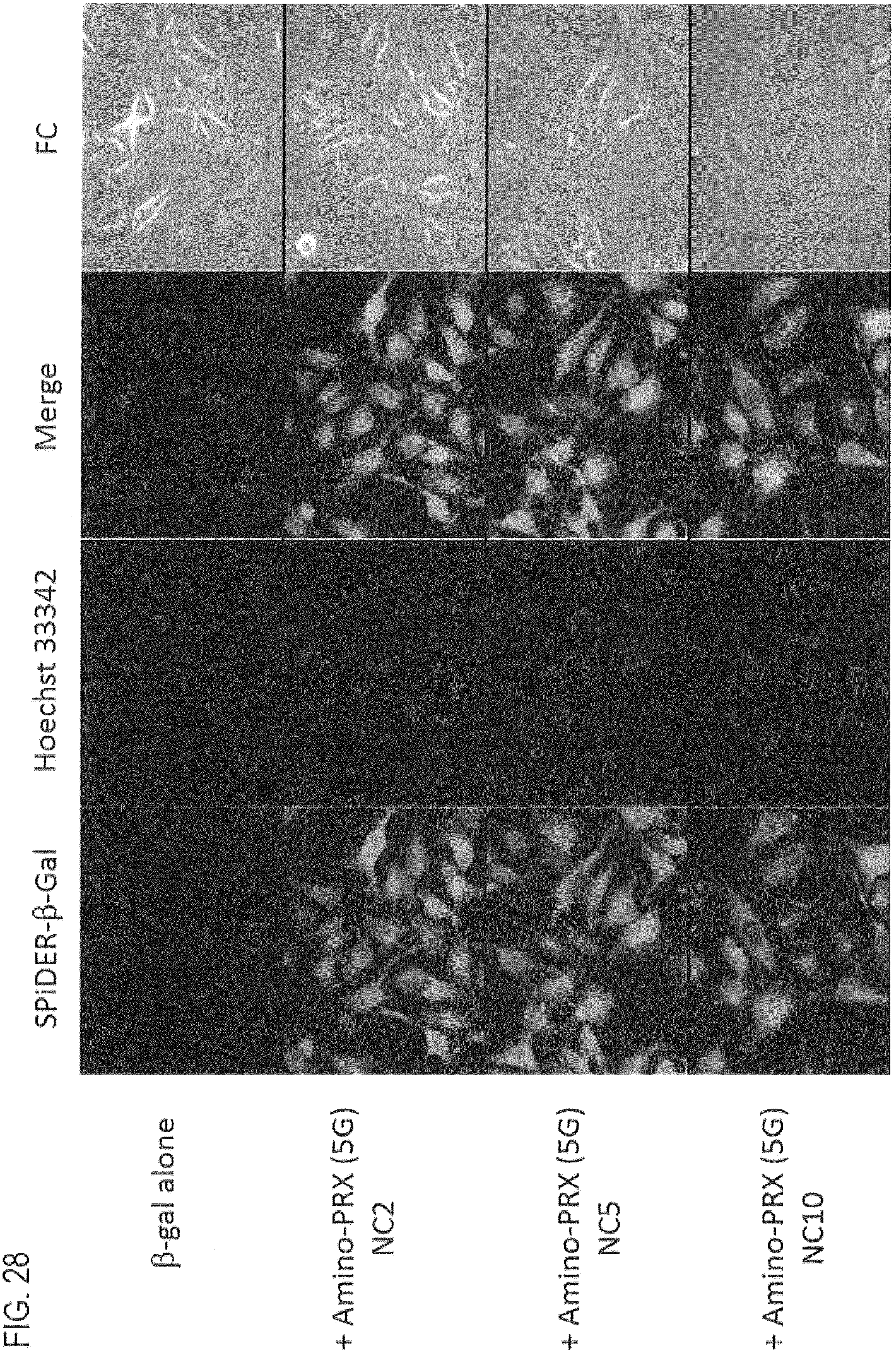
FC
Merge
Hoechst 33342
SPiDER-β-Gal
β-gal alone
+ Amino-PRX (5G) NC2
+ Amino-PRX (5G) NC5
+ Amino-PRX (5G) NC10

FIG. 28

FIG. 29

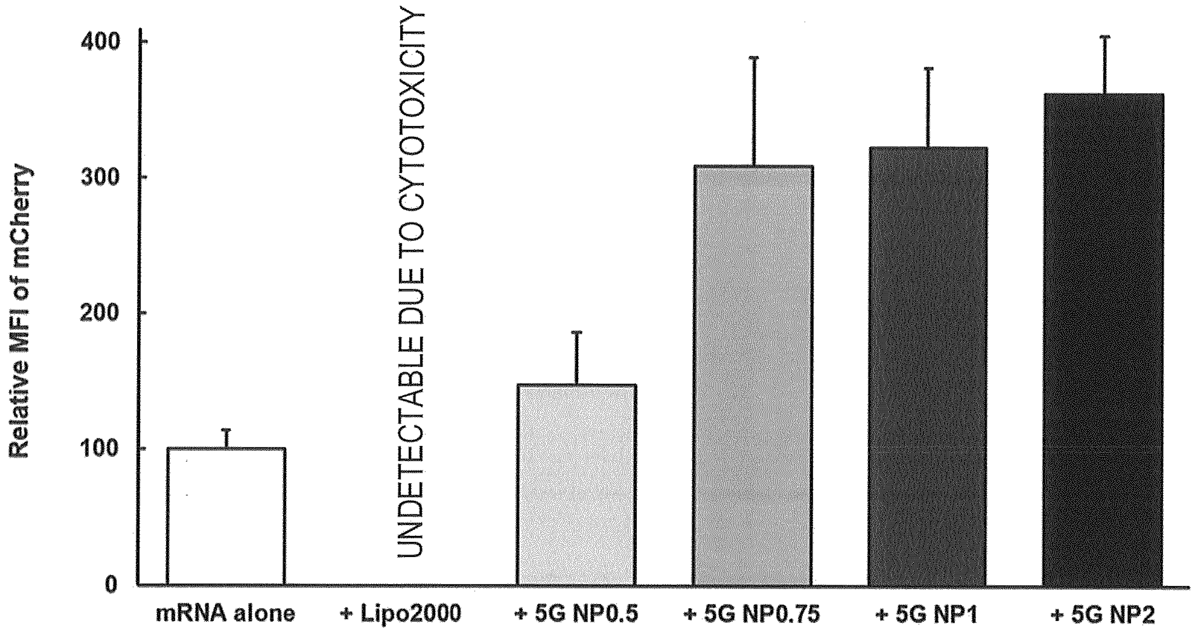
Relative MFI of mCherry
400
300
200
100
0
UNDETECTABLE DUE TO CYTOTOXICITY
mRNA alone
+ Lipo2000
+ 5G NP0.5
+ 5G NP0.75
+ 5G NP1
+ 5G NP2

FIG. 30

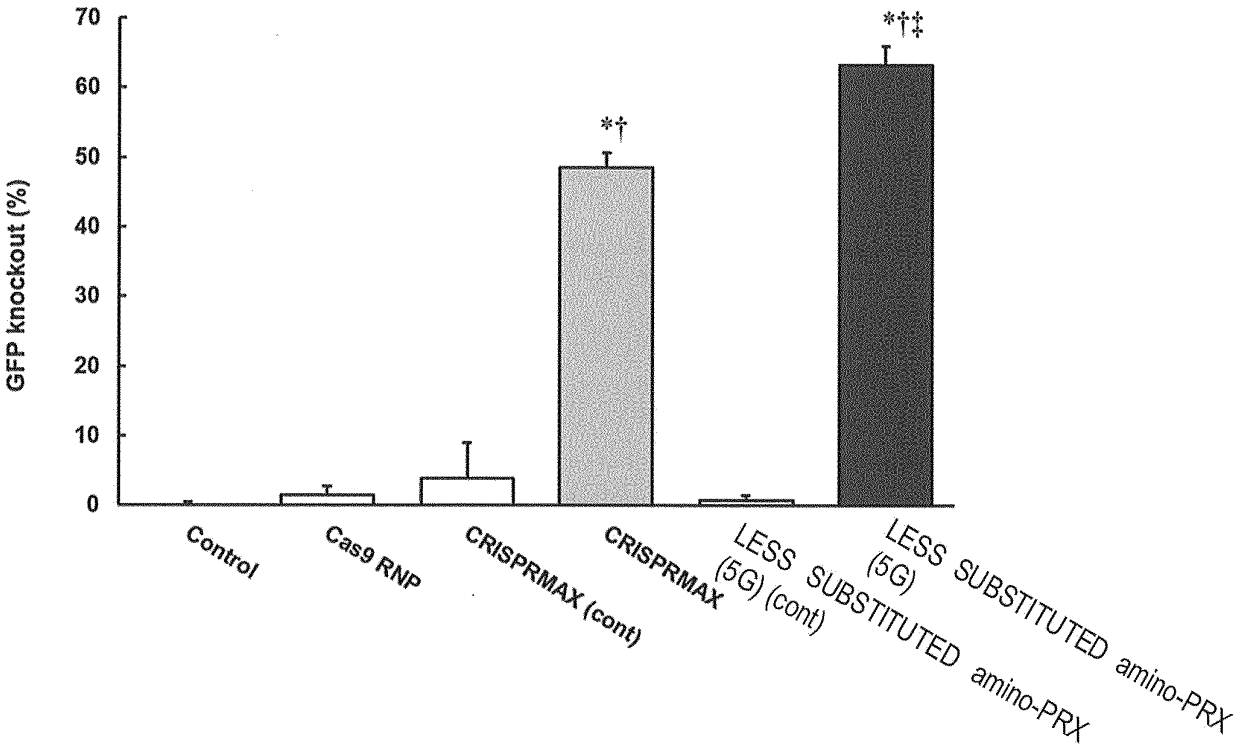
GFP knockout (%)
70
60
50
40
30
20
10
0
*†
*†‡
Control
Cas9 RNP
CRISPRMAX (cont)
CRISPRMAX
LESS SUBSTITUTED amino-PRX (5G) (cont)
LESS SUBSTITUTED amino-PRX (5G)

## INTERNATIONAL SEARCH REPORT

International application No.
**PCT/JP2022/002968**

### A. CLASSIFICATION OF SUBJECT MATTER

***C08B 37/16***(2006.01)i; ***A61K 9/16***(2006.01)i; ***A61K 38/46***(2006.01)i; ***A61K 47/34***(2017.01)i; ***A61K 47/40***(2006.01)i; ***A61K 48/00***(2006.01)i; ***A61P 43/00***(2006.01)i; ***C12N 15/09***(2006.01)i; ***C12Q 1/68***(2018.01)i
FI: C08B37/16; C12N15/09 110; A61K38/46; A61K48/00; A61P43/00 105; A61K47/40; A61K47/34; C12Q1/68 ZNA; A61K9/16

According to International Patent Classification (IPC) or to both national classification and IPC

### B. FIELDS SEARCHED

Minimum documentation searched (classification system followed by classification symbols)

C08B37/16; A61K9/16; A61K38/46; A61K47/34; A61K47/40; A61K48/00; A61P43/00; C12N15/09; C12Q1/68

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br>Y | CN 105906816 A (KUNMING UNIVERSITY OF SCIENCE AND TECHNOLOGY) 31 August 2016 (2016-08-31)<br>in particular, formula II | 1-5, 9-11, 13-20, 23,24<br>1-24 |
| X<br>Y | Materials Science & Engineering, C: Materials for Biological Applications, 2017, 71, 1028-1036, DOI 10.1016/j.msec.2016.11.055<br>in particular, scheme 1 | 1-5, 9-11, 13-20, 23, 24<br>1-24 |
| X<br>Y | Carbohydrate Research, 2015, 412, 7-14, DOI 10.1016/j.carres.2015.04.021<br>in particular, scheme 3 | 1-5, 9-11, 13-20, 23, 24<br>1-24 |
| Y | Biomaterials, 2013, 34(10), 2480-2491, DOI 10.1016/j.biomaterials.2012.12.006<br>fig. 1 | 1-24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2022** | **29 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

| International application No. |
|---|
| **PCT/JP2022/002968** |

**C. DOCUMENTS CONSIDERED TO BE RELEVANT**

| Category* | Citation of document, with indication, where appropriate, of the relevant passages | Relevant to claim No. |
|---|---|---|
| Y | Small, 2016, 12(45), 6223-6232, DOI 10.1002/smll.201601966<br>in particular, supporting information, scheme S1 | 1-24 |
| Y | Colloids and Surfaces, B: Biointerfaces, 2013, 109, 167-175, DOI 10.1016/j.colsurfb.2013.03.048<br>fig. 2 | 1-24 |
| Y | WO 2015/025815 A1 (NATIONAL UNIVERSITY CORPORATION TOKYO MEDICAL ANDDENTAL UNIVERSITY) 26 February 2015 (2015-02-26)<br>in particular, examples | 1-24 |
| Y | JP 2018-522566 A (CARIBOU BIOSCIENCES, INC.) 16 August 2018 (2018-08-16)<br>in particular, paragraph [0228] | 1-24 |
| A | Russian Journal of General Chemistry, 2017, 87(9), 2125-2129, DOI 10.1134/S1070363217090377<br>scheme 1 | 1-24 |
| A | ACS Applied Materials & Interfaces, 2019, 11(4), 4425-4438, DOI 10.1021/acsami.8b19950<br>fig. 2 | 1-24 |
| A | JP 2018-024768 A (BRIDGESTONE CORP.) 15 February 2018 (2018-02-15)<br>examples | 1-24 |

Form PCT/ISA/210 (second sheet) (January 2015)

INTERNATIONAL SEARCH REPORT

International application No.
PCT/JP2022/002968

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
**Information on patent family members**

International application No.
**PCT/JP2022/002968**

| Patent document cited in search report | Publication date (day/month/year) | Patent family member(s) | Publication date (day/month/year) |
|---|---|---|---|
| CN 105906816 A | 31 August 2016 | (Family: none) | |
| WO 2015/025815 A1 | 26 February 2015 | US 2016/0199512 A1<br>examples<br>EP 3037097 A1 | |
| JP 2018-522566 A | 16 August 2018 | WO 2017/027423 A1<br>paragraph [0268]<br>EP 3320092 A1<br>EP 3461894 A1<br>CN 107922944 A<br>KR 10-2018-0030084 A<br>US 2017/0037432 A1<br>US 2017/0044508 A1 | |
| JP 2018-024768 A | 15 February 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021010606 A
- WO 2015025815 A


### Non-patent literature cited in the description


- **S. KIM et al.** *Genome Res,* 2014, vol. 24, 112-1019
- **M. WANG et al.** *Proc. Natl. Acad. Sci.,* 2015, vol. 113, 2868-2873
- **GAO, X et al.** *Nature,* 2018, vol. 553, 217-221
- **WEI, T et al.** *Nat Commun,* 2020, vol. 11, 3232
- **LEE, K et al.** *Elife,* 2017, vol. 6
- **WANG, Y. et al.** *ACS Appl Mater Interfaces,* 2018, vol. 10, 31915-31927
- **TAHARABARU, T. et al.** *ACS Appl Mater Interfaces,* 2020, vol. 12, 21386-21397
- **LEE, K. et al.** *Nat Biomed Eng,* 2017, vol. 1, 889-901
- **LEE, B. et al.** *Nat Biomed Eng,* 2018, vol. 2, 497-507
- **CHO, E. Y. et al.** *J Nanobiotechnology,* 2019, vol. 17, 19
- **LIU, C. et al.** *Sci Adv,* 2019, vol. 5, eaaw8922
- **RUI, Y et al.** *Sci Adv,* 2019, vol. 5, eaay3255
- **CHEN, G et al.** *Nat Nanotechnol,* 2019, vol. 14, 974-980
- **AKIRA HARADA et al.** *Nature,* 1992, vol. 356, 325-327
- **AKIRA HARADA et al.** *Chem. Rev.,* 2009, vol. 109, 5974-6023
- **JUN ARAKI et al.** *Macromolecules,* 2005, vol. 38, 7524-7527
- **WENZ, G et al.** *Chemical Reviews,* 2006, vol. 106, 782-817
- **TAMURA, A. et al.** *Chem Commun (Camb),* 2014, vol. 50, 13433-13446
- **HIGASHI, T et al.** *Chem Pharm Bull (Tokyo),* 2019, vol. 67, 289-298
- **YASUDA, Y. et al.** *J Am Chem Soc,* 2019, vol. 141, 9655-9663
- **OOYA, T. et al.** *Journal of the American Chemical Society,* 2006, vol. 128, 3852-3853
- **EMAMI, M. R et al.** *Advanced Therapeutics,* 2019, vol. 2, 1900061
- **TAMURA, A et al.** *Biomaterials,* 2013, vol. 34, 2480-2491
- **TAMURA, A et al.** *Sci Rep,* 2013, vol. 3, 2252
- **TAMURA, A. et al.** *Macromol Biosci,* 2015, vol. 15, 1134-1145
- **ATSUSHI TAMURA et al.** *Biomaterials,* 2013, vol. 34, 2480-2491
- **ATSUSHI TAMURA et al.** *Sci Rep,* 2013, vol. 3, 2252
- **YING JI et al.** *Biomaterials,* 2019, vol. 192, 416-428
- **MICHAEL R. ENAMI et al.** *Adv. Therap.,* 2019, vol. 2, 1900061
- **TAO WAN et al.** *Journal of Controlled Release,* 2020, vol. 322, 236-247
- *Journal of Controlled Release,* 2001, vol. 76, 11-25
- *Biomacromolecules,* 2003, vol. 4, 1426-1432
- *Langmuir,* 2011, vol. 27 (2), 612-617
- *Angew. Chem. Int. Ed,* 2013, vol. 52, 7300-7305
- *J. Mater. Chem. B,* 2013, vol. 1, 3535-3544
- *Biomaterials,* 2013, vol. 34, 2480-2491
- *Scientific Reports,* 2013, vol. 3, 2252
- **CHOW, R.D et al.** *Nat Biomed Eng,* 2020
- **ARAKI, J. et al.** *Macromolecules,* 2005, vol. 38, 7524-7527
- **LIU, F et al.** *Nat Cell Biol.,* 2017, vol. 19, 1358-1370
- **MIYATA, K et al.** *Journal of the American Chemical Society,* 2008, vol. 130, 16287-16294